(19) Europäisches Patentamt
European Patent Office
Office européen des brevets

(11) **EP 3 865 544 A1**

(12) **EUROPEAN PATENT APPLICATION**

(43) Date of publication:
**18.08.2021 Bulletin 2021/33**

(21) Application number: **20156964.7**

(22) Date of filing: **12.02.2020**

(51) Int Cl.:
***C09B 11/28*** *(2006.01)* ***C09B 57/00*** *(2006.01)*
***G01N 33/53*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Max-Planck-Gesellschaft zur Förderung der Wissenschaften e.V.**
**80539 München (DE)**

(72) Inventors:
- **BUCEVICIUS, Jonas**
  **37083 Göttingen (DE)**
- **GERASIMATIE, Ruta**
  **37085 Göttingen (DE)**
- **KOSTIUK, Goergij**
  **37085 Göttingen (DE)**
- **LUKINAVICIUS, Grazvydas**
  **37085 Göttingen (DE)**

(74) Representative: **v. Bezold & Partner Patentanwälte - PartG mbB**
**Akademiestraße 7**
**80799 München (DE)**

# (54) FLUORESCENT RHODAMINE DYES WITH ENHANCED CELL PERMEABILITY

(57) The invention relates to novel fluorescent rhodamine dyes with enhanced cell permeability which are rhodamine 4'-isomers having the following general structural formula A:

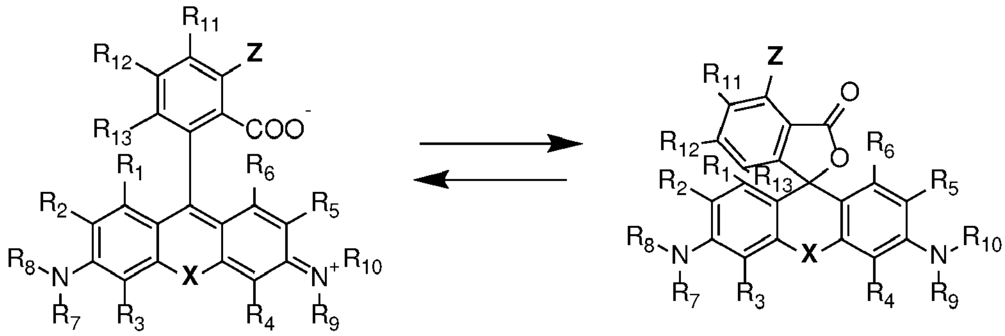

Zwitterion form Spirolactone form

**Aa** **Ab**

wherein Z is selected from O(alkyl), O(aryl), S(alkyl), S(aryl), S(O)(alkyl), S(O)(aryl), $S(O)_2$(alkyl), $S(O)_2$(aryl), $S(O)_2$(-O-alkyl), $S(O)_2$(-O-aryl), $S(O)_2NH$(alkyl), $S(O)_2NH$(aryl), $S(O)_2N(alkyl)_2$, $S(O)_2N(aryl)_2$, $S(O)_2N$(alkyl)(aryl), NH(alkyl), $N(alkyl)_2$, N(alkyl)(aryl), NH(aryl), $N(aryl)_2$, C(O)O(alkyl), C(O)O(aryl), C(O)(alkyl), C(O)(aryl), P(O)OH(-NH-alkyl), P(O)OH(-O-alkyl), P(O)OH(-NH-aryl), P(O)OH(-O-aryl), $P(O)(-O-alkyl)_2$, $P(O)(-NH-alkyl)_2$, $P(O)OH(-N(alkyl)_2)$, $P(O)OH(-N(aryl)_2)$, $P(O)(-N(aryl)_2)_2$, $P(O)(-N(alkyl)_2)(-N(alkyl)_2)$, $P(O)(-O-aryl)_2$, $P(O)(-NH-aryl)_2$, P(O)(-O-alkyl)(-O-aryl), P(O)(-NH-alkyl)(-O-aryl), P(O)(-O-alkyl)(-NH-aryl), P(O)(-NH-alkyl)(-NH-aryl), in particularly -C(O)OH, -C(O)NH(alkyl), C(O)NH(aryl), $CON(alkyl)_2$, or any group which induces a neighboring group effect via steric, ionic or bonding interactions with the adjacent carboxyl group resulting in a shift of the equilibrium between zwitterionic form and spirolactone form towards the spirolactone form.

The invention further relates to 4'-isomer derivatives and probes comprising such 4'-isomers coupled to at least one reactive group or ligand which is capable to interact with or bind to other molecules, wherein said reactive group or ligand

EP 3 865 544 A1

may be coupled to the rhodamine 4'-isomer fluorophore either directly or via a linker.

Another aspect of the invention relates to the use of these compounds and conjugates as labels in microscopic, spectroscopic and other imaging techniques and/or as cell permeable substances penetrating through membranes of living and fixed cells in vivo or in vitro.

## Description

Background of the invention

Modern super-resolution fluorescence microscopy techniques permit observation of biological processes in living organisms down to molecular level (Sahl, S. J.; Hell, S. W.; Jakobs, S., Nat. Rev. Mol. Cell. Biol. 2017, 18 (11), 685-701; Hell, S. W, Angew Chem Int Ed Engl 2015, 54 (28), 8054-66). Success of imaging experiments depends on the availability of biocompatible fluorescent probes for the specific labelling of cellular structures. Usually, these probes are constructed by coupling a fluorescent dye to a small molecule ligand via a linker to target a protein of interest. A linker and a fluorophore make significant contributions to the final properties of such probes often resulting in compromised cell permeability and off-targeting (Wang, L.; Frei, M. S.; Salim, A.; Johnsson, K., J Am Chem Soc 2019, 141 (7), 2770-2781). The ideal biocompatible fluorophore fulfil at least the following five criteria: First, the fluorescent dye should be highly photostable avoiding rapid bleaching in the course of an experiment. Second, it should have high extinction coefficient and quantum yield that results in a good brightness. Third, a derivatization of the fluorescent dye molecule with (i) ligands that specifically bind to other (bio)molecules in vitro or in vivo or, (ii) reactive groups such as activated esters (iii) molecules that can control the fluorescence properties of the fluorophore should be possible. Fourth, it should not interact with biomolecules present in the cell and show minimal background. Fifth, the fluorophore should be membrane permeable. Numerous fluorophores exist that fulfil the first three criteria, the tetramethylrhodamine (TMR) being an example of fluorophore whose derivatives show relatively low permeability and high off-targeting. Only few fluorophores available that also fulfil the fifth criterion. Recently several groups introduced fluorophores that show minimal background binding or are fluorogenic and are able to mask this unspecific binding. These fluorophores are various derivatives of rhodamines. The most prominent examples are carbopyronines (CP) (Butkevich A.N.; Mitronova G.Y.; Sidenstein. S.C.; Klocke J.L.; Kamin D.; Meineke D.N.H.; D'Este E.; Kraemer P.T.; Danzl J.G.; Belov V.N.; Hell S.W., Angew. Chem. Int. Ed. 2016, 55, 3290 -3294) and silicon-rhodamines (SiR) (Figure 1) (Lukinavičius G; Umezawa K; Olivier N; Honigmann A; Yang G; Plass T; Mueller V; Reymond L; Corrêa IR Jr; Luo ZG; Schultz C; Lemke EA; Heppenstall P; Eggeling C; Manley S; Johnsson K, Nat Chem, 2013, 5(2), 132-139).

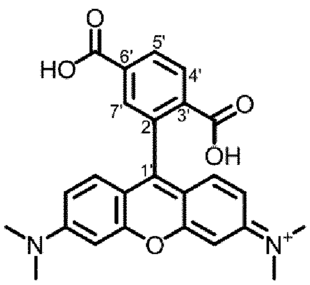
tetramethylrhodamine (TMR)

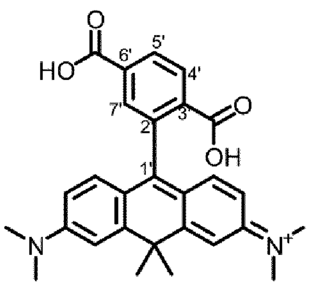
carbopyronine (CP)

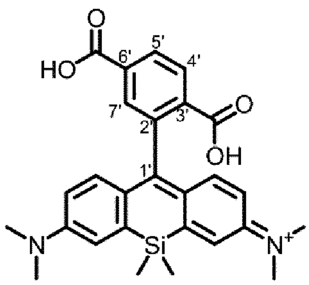
silicon-rhodamine (SiR)

**Scheme 1.** Structures of commonly used rhodamines

A lot of interest is dedicated to the rhodamine class fluorophores, which are able to cycle between non-fluorescent spirolactone and a fluorescent zwitterion forms (Scheme 2). This property is exploited to generate fluorogenic probes, which are sensitive to number of environmental factors: pH, ion concentration, enzyme, tension force, local microenvironment polarity or light (Uno, S. N.; Tiwari, D. K.; Kamiya, M.; Arai, Y.; Nagai, T.; Urano, Y., Microscopy (Oxf) 2015, 64 (4), 263-77). Because spirolactone is a more hydrophobic molecule than zwitterion, the change of the equilibrium between these two forms contributes to the permeability of the rhodamine-based fluorescent probes (Lukinavičius, G.; Reymond, L.; Umezawa, K.; Sallin, O.; D'Este, E.; Gottfert, F.; Ta, H.; Hell, S. W.; Urano, Y.; Johnsson, K., J. Am. Chem. Soc. 2016, 138 (30), 9365-8.

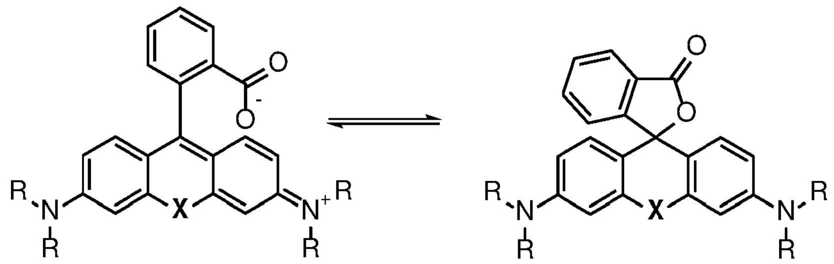

**Scheme 2.** Equilibrium between fluorescent (open) and non-fluorescent spirolactone forms (closed) in rhodamines

It is assumed that regular rhodamines and carbon-substituted analogs (carbopyronines) have this equilibrium shifted towards zwitterion form, resulting in relatively poor permeability. Several studies attempted to induce spirolactone form preference by introduction of the electron-withdrawing groups in the xanthene core (Zheng, Q.; Ayala, A. X.; Chung, I.; Weigel, A. V.; Ranjan, A.; Falco, N.; Grimm, J. B.; Tkachuk, A. N.; Wu, C.; Lippincott-Schwartz, J.; Singer, R. H.; Lavis, L. D., ACS Cent Sci 2019, 5 (9), 1602-1613) or in the benzoic acid substituent (Wang, L.; Tran, M.; D'Este, E.; Roberti, J.; Koch, B.; Xue, L.; Johnsson, K., bioRxiv 2019, 690867). However, all these approaches result in a bulkier core structure and alter physicochemical properties of the dyes.

The marking of proteins and nucleic acids with fluorescent dyes in living cells represents a widely used technique in life sciences and medical research. Multiple strategies have been developed to reach this goal. The most frequently used methods are: the tetracysteine tag that binds to biarsenical fluorophores, the SNAP/CLIP-tag that irreversibly reacts with benzylguanine (BG) or benzylcytosine (BC) derivatives (Keppler, A.; Gendreizig, S.; Gronemeyer, T.; Pick, H.; Vogel, H.; Johnsson, K., Nat Biotechnol 2003, 21 (1), 86-9), the Halo-tag that reacts with primary chlorides and dihydrofolate reductase that binds to trimethoprim derivatives (Hinner, M. J.; Johnsson, K., Curr Opin Biotechnol 2010, 21 (6), 766-76). Alternatively, a specific fluorescence labelling of a protein of interest can be achieved through the incorporation of an unnatural, fluorescent amino acid (Liu, C. C.; Schultz, P. G., Annu Rev Biochem 2010, 79, 413-44). Finally, proteins, DNA or RNA can be labelled using specifically binding ligands. The final probe is composed of the targeting moiety, linker and fluorescent dye. All these components contribute to the final properties of the probe. One on the most important property is cell membrane permeability, which allows efficient delivery of fluorophore to the target. This problem is often overcome by delivering fluorophore into the cell through invasive methods such as microinjection (Keppler, A.; Arrivoli, C.; Sironi, L.; Ellenberg, J., Biotechniques 2006, 41 (2), 167-70, 172, 174-5), bead-loading (Maurel, D.; Banala, S.; Laroche, T.; Johnsson, K., ACS Chem Biol 2010, 5 (5), 507-16) or electroporation (Jones, S. A.; Shim, S. H.; He, J.; Zhuang, X., Nat Methods 2011, 8 (6), 499-508).

In view of the drawbacks of fluorescent dyes of the prior art and the alternative strategies to obviate the limitations of their delivery to living cells and tissues, the main object of the present invention is to provide improved fluorescent dyes, in particular fluorescent dyes with enhanced cell permeability while still exhibiting desirable optical characteristics such as high photostability, high extinction coefficients and high quantum yields.

This objective has been achieved by providing novel compounds and fluorescent dyes according to claims 1-9, conjugates comprising said compounds and dyes according to claim 10, as well as the applications of the claimed compounds according to claims 11 to 20.

Description of the invention

The novel fluorescent dyes of the invention are rhodamine 4'-isomers that have the following general structural formula A

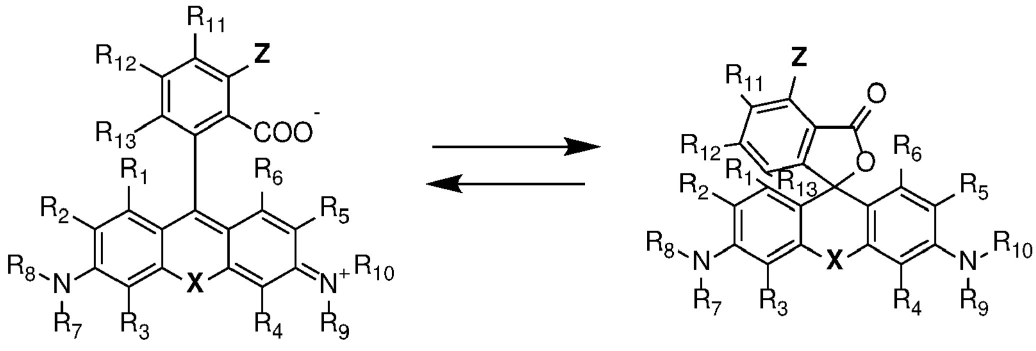

Zwitterion form

Spirolactone form

**Aa**

**Ab**

wherein

$R_1$, $R_2$, $R_3$, $R_4$ $R_5$ $R_6$ $R_7$ $R_8$ $R_9$ $R_{10}$ $R_{11}$ $R_{12}$ $R_{13}$ are independently selected from H, halogen, D, CN, OH, O(alkyl), O(aryl), SH, S(alkyl), S(aryl), S(O)(alkyl), S(O)(aryl), $S(O)_2$(alkyl), $S(O)_2$(aryl), $N_3$, $NH_2$, NH(alkyl), N(alkyl)$_2$, NH(aryl), NH(aryl)$_2$, $NO_2$, CHO, C(O)(alkyl), C(O)(aryl), COOH, COO(alkyl) COO(aryl), C(O)NH(alkyl), C(O)NH(aryl), C(O)N(alkyl)$_2$, C(O)N(aryl)$_2$, P(O)OH(alkyl), P(O)OH(aryl), P(O)(-O-alkyl)$_2$, P(O)(-O-aryl)$_2$, $PO_3H_2$, $SO_3H$, alkyl, bulky alkyl, substituted alkyl, alkenyl, substituted alkenyl, substituted bulky alkenyl;

$R_2$ and $R_8$ or $R_7$ and $R_8$ taken together may form a cyclic structure;

$R_5$ and $R_9$ or $R_9$ and $R_{10}$ taken together may form a cyclic structure;

$R_3$ and $R_7$ or $R_4$ and $R_9$ taken together may form a cyclic structure;

$R_1$ and $R_2$ or $R_5$ and $R_6$ taken together may form a cyclic structure;

X is selected from $CR_{14}R_{15}$, a heteroatom, in particular O, S, $NR_{14}$, $SO_2$, P(O)OH, P(O)$OR_{14}$, $SiR_{14}R_{15}$, $GeR_{14}R_{15}$, where $R_{14}$ and $R_{15}$ are alkyl or aryl;

Z is selected from O(alkyl), O(aryl), S(alkyl), S(aryl), S(O)(alkyl), S(O)(aryl), $S(O)_2$(alkyl), $S(O)_2$(aryl), $S(O)_2$(-O-alkyl), $S(O)_2$(-O-aryl), $S(O)_2$NH(alkyl), $S(O)_2$NH(aryl), $S(O)_2$N(alkyl)$_2$, $S(O)_2$N(aryl)$_2$, $S(O)_2$N(alkyl)(aryl), NH(alkyl), N(alkyl)$_2$, N(alkyl)(aryl), NH(aryl), N(aryl)$_2$, C(O)O(alkyl), C(O)O(aryl), C(O)(alkyl), C(O)(aryl), P(O)OH(-NH-alkyl), P(O)OH(-O-alkyl), P(O)OH(-NH-aryl), P(O)OH(-O-aryl), P(O)(-O-alkyl)$_2$, P(O)(-NH-alkyl)$_2$, P(O)OH(-N(alkyl)$_2$), P(O)OH(-N(aryl)$_2$), P(O)(-N(aryl)$_2$)$_2$, P(O)(-N(alkyl)$_2$)(-N(alkyl)$_2$), P(O)(-O-aryl)$_2$, P(O)(-NH-aryl)$_2$, P(O)(-O-alkyl)(-O-aryl), P(O)(-NH-alkyl)(-O-aryl), P(O)(-O-alkyl)(-NH-aryl), P(O)(-NH-alkyl)(-NH-aryl), in particularly -C(O)OH, -C(O)NH(alkyl), C(O)NH(aryl), CON(alkyl)$_2$, or any group which induces a neighboring group effect via steric, ionic or bonding interactions with the adjacent carboxyl group involved in spirolactone formation resulting in a shift of the equilibrium between zwitterionic form and spirolactone form towards the spirolactone form as indicated by an increased $D_{50}$ value of the 4'-isomer compared to the $D_{50}$ value of a reference isomer, in particular a 5'- or 6'-isomer, and preferably an absolute $D_{50}$ value of the 4'-isomer which is a numeric value of at least 13 or 14, more preferred at least 15, 20, or 25, wherein said $D_{50}$ value represents the dielectric constant at which the absorbance of a dye sample in a mixture of 1,4-dioxane and water is halved.

The $D_{50}$ values indicated herein are determined by measuring the respective absorbance of a sample (dye isomer or derivative or conjugate thereof), in varied water-1,4-dioxane mixtures with known dielectric constants according to an established method (e.g. Åkerlöf G, Short AO. J Am Chem Soc 1936, 58(7): 1241-1243). $D_{50}$ is commonly used as numeric value for the evaluation of the equilibrium changes and represents a dielectric constant at which absorbance is halved (Butkevich A.N.; Mitronova G.Y.; Sidenstein. S.C.; Klocke J.L.; Kamin D.; Meineke D.N.H.; D'Este E.; Kraemer P.T.; Danzl J.G.; Belov V.N.; Hell S.W., Angew. Chem. Int. Ed. 2016, 55, 3290-3294).

The $D_{50}$ values were calculated using the following equation:

$$A = A_0 + (A_{max} - A_0) / \left(1 + \left(\frac{D_{50}}{d}\right)^{Hill}\right) \qquad (1)$$

where $A_o$ - absorbance at $\lambda_{max}$ at $\varepsilon_r = 0$, $A_{max}$ - the highest reached absorbance at $\lambda_{max}$, d - dielectric constant of 1,4-dioxane-water mixture at a given point, *Hill* - Hill slope coefficient determining the steepness of a dose-response curve, $D_{50}$ - corresponds to d value that provokes half of the absorbance amplitude ($A_{max}$-$A_o$).

The above indicated absolute $D_{50}$ value of 15 as obtained by said method corresponds to a mixture of 75% 1,4-dioxane and 25% water.

Preferably $R_1$ to $R_{13}$ are each independently selected from H, alkyl, in particular $C_1$-$C_{20}$ alkyl, $C_1$-$C_{12}$ alkyl, or $C_1$-$C_6$ alkyl.

X is preferably selected from O, $CR_{14}R_{15}$, $SiR_{14}R_{15}$, $GeR_{14}R_{15}$ where $R_{14}$ and $R_{15}$ are alkyl, in particular $C_1$-$C_{20}$ alkyl, $C_1$-$C_{12}$ alkyl, or $C_1$-$C_6$ alkyl, or aryl, in particular phenyl or naphthyl.

Said group Z which induces a neighboring group effect via steric, ionic or bonding interactions according to the invention is neither Cl, $NH_2$ or $NO_2$ and is preferably selected from the following: -C(O)OH, -C(O)NH(alkyl), C(O)NH(aryl), CON(alkyl)$_2$, with alkyl in particular $C_1$-$C_{20}$ alkyl, $C_1$-$C_{12}$ alkyl, or $C_1$-$C_6$ alkyl.

In some specific embodiments, the fluorescent dyes according to the present invention have one of the following structural formulae **B - E**

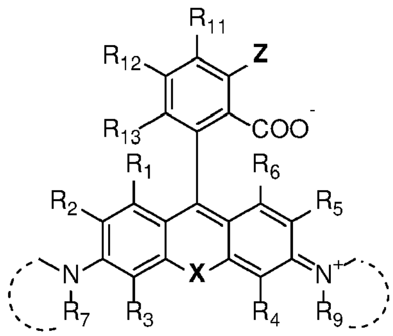

**B**

wherein the cyclic structure formed by $R_7$ and $R_8$ and/or by $R_9$ and $R_{10}$ taken together represents a non-aromatic heterocycle, in particular selected from azetidine, pyrrolidine, piperidine, morpholine, azepane or azecane;

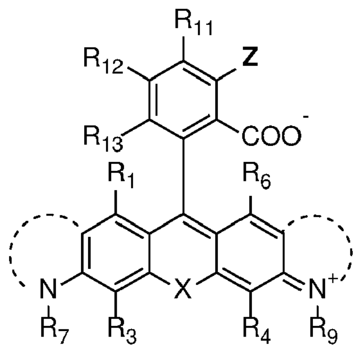

**C**

wherein the cyclic structure formed by $R_2$ and $R_8$ and/or by $R_5$ and $R_{10}$ taken together represents an aromatic or non-aromatic 5 or 6 atom membered heterocyclic structure, in particular selected from azetidine, pyrrolidine, piperidine, azepane or azecane;

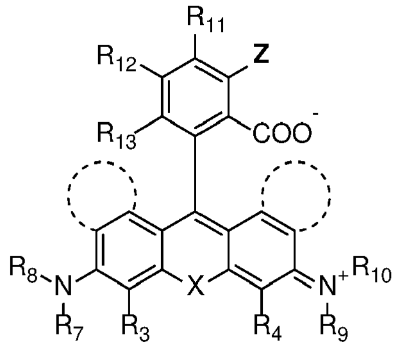

**D**

wherein the cyclic structure formed by $R_1$ and $R_2$ and/or by $R_5$ and $R_6$ taken together represents an aromatic or non-aromatic 5 or 6 atom membered heterocyclic structure, in particular selected from azetidine, pyrrolidine, piperidine, azepane or azecane;

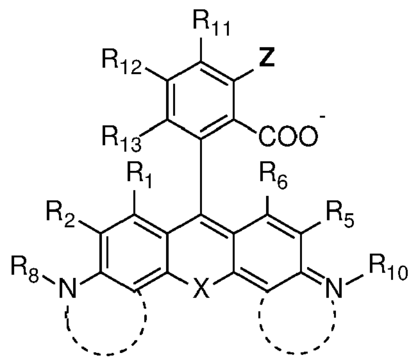

**E**

wherein the cyclic structure formed by $R_3$ and $R_7$ and/or by $R_4$ and $R_9$ taken together represents an aromatic or non-aromatic 5 or 6 atom membered heterocyclic structure, in particular selected from azetidine, pyrrolidine, piperidine, azepane or azecane.

In other specific embodiments, the fluorescent dyes according to the present invention have one of the following structural formulae

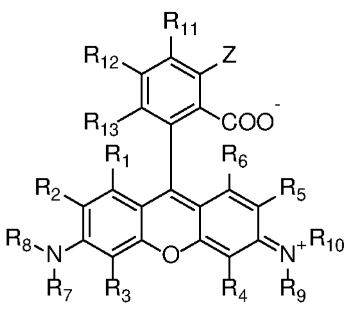

**F**

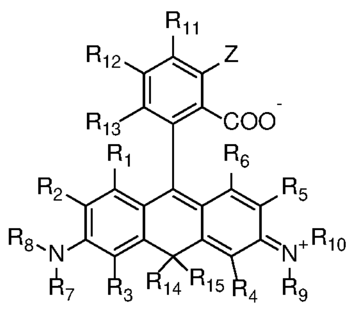

**G**

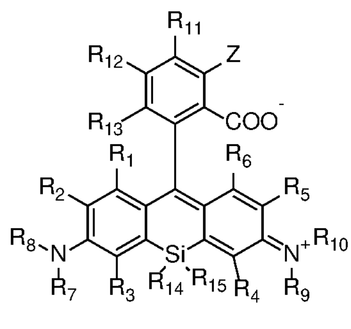

**H**

wherein

$R_1$, $R_2$, $R_3$, $R_4$ $R_5$ $R_6$ $R_7$ $R_8$ $R_9$ $R_{10}$ $R_{11}$ $R_{12}$ $R_{13}$ are independently selected from H, halogen, D, CN, OH, O(alkyl), O(aryl), SH, S(alkyl), S(aryl), S(O)(alkyl), S(O)(aryl), $S(O)_2$(alkyl), $S(O)_2$(aryl), $N_3$, $NH_2$, NH(alkyl), N(alkyl)$_2$, NH(aryl), NH(aryl)$_2$, $NO_2$, CHO, C(O)(alkyl), C(O)(aryl), COOH, COO(alkyl) COO(aryl), C(O)NH(alkyl), C(O)NH(aryl), C(O)N(alkyl)$_2$, C(O)N(aryl)$_2$, P(O)OH(alkyl), P(O)OH(aryl), P(O)(-O-alkyl)$_2$, P(O)(-O-aryl)$_2$, $PO_3H_2$, $SO_3H$, alkyl,

bulky alkyl, substituted alkyl, alkenyl, substituted alkenyl, substituted bulky alkenyl;
$R_2$ and $R_8$ or $R_7$ and $R_8$ taken together may form a cyclic structure;
$R_5$ and $R_9$ or $R_9$ and $R_{10}$ taken together may form a cyclic structure;
$R_3$ and $R_7$ or $R_4$ and $R_9$ taken together may form a cyclic structure;
$R_1$ and $R_2$ or $R_5$ and $R_6$ taken together may form a cyclic structure;
Z is selected from O(alkyl), O(aryl), S(alkyl), S(aryl), S(O)(alkyl), S(O)(aryl), $S(O)_2$(alkyl), $S(O)_2$(aryl), $S(O)_2$(-O-alkyl), $S(O)_2$(-O-aryl), $S(O)_2$NH(alkyl), $S(O)_2$NH(aryl), $S(O)_2$N(alkyl)$_2$, $S(O)_2$N(aryl)$_2$, $S(O)_2$N(alkyl)(aryl), NH(alkyl), N(alkyl)$_2$, N(alkyl)(aryl), NH(aryl), N(aryl)$_2$, C(O)O(alkyl), C(O)O(aryl), C(O)(alkyl), C(O)(aryl), P(O)OH(-NH-alkyl), P(O)OH(-O-alkyl), P(O)OH(-NH-aryl), P(O)OH(-O-aryl), P(O)(-O-alkyl)$_2$, P(O)(-NH-alkyl)$_2$, P(O)OH(-N(alkyl)$_2$), P(O)OH(-N(aryl)$_2$), P(O)(-N(aryl)$_2$)$_2$, P(O)(-N(alkyl)$_2$)(-N(alkyl)$_2$), P(O)(-O-aryl)$_2$, P(O)(-NH-aryl)$_2$, P(O)(-O-alkyl)(-O-aryl), P(O)(-NH-alkyl)(-O-aryl), P(O)(-O-alkyl)(-NH-aryl), P(O)(-NH-alkyl)(-NH-aryl), in particularly -C(O)OH, -C(O)NH(alkyl), C(O)NH(aryl), CON(alkyl)$_2$ or any group which induces a neighboring group effect via steric, ionic or bonding interactions as defined above.

More specifically, the fluorescent dyes have one of the following structural formulae:

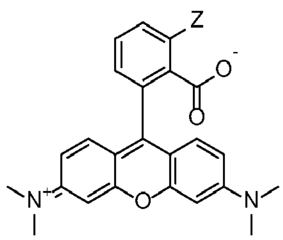
**4-TMR**
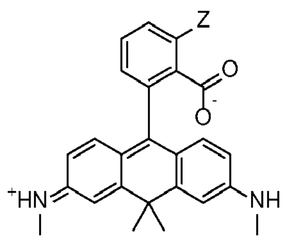
**4-580CP**
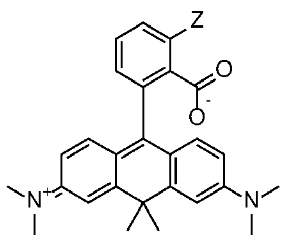
**4-610CP**
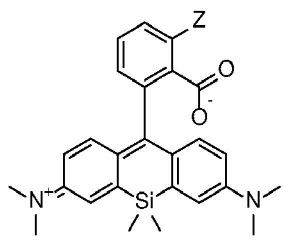
**4-SiR**

wherein
Z is selected from O(alkyl), O(aryl), S(alkyl), S(aryl), S(O)(alkyl), S(O)(aryl), $S(O)_2$(alkyl), $S(O)_2$(aryl), $S(O)_2$(-O-alkyl), $S(O)_2$(-O-aryl), $S(O)_2$NH(alkyl), $S(O)_2$NH(aryl), $S(O)_2$N(alkyl)$_2$, $S(O)_2$N(aryl)$_2$, $S(O)_2$N(alkyl)(aryl), NH(alkyl), N(alkyl)$_2$, N(alkyl)(aryl), NH(aryl), N(aryl)$_2$, C(O)O(alkyl), C(O)O(aryl), C(O)(alkyl), C(O)(aryl), P(O)OH(-NH-alkyl), P(O)OH(-O-alkyl), P(O)OH(-NH-aryl), P(O)OH(-O-aryl), P(O)(-O-alkyl)$_2$, P(O)(-NH-alkyl)$_2$, P(O)OH(-N(alkyl)$_2$), P(O)OH(-N(aryl)$_2$), P(O)(-N(aryl)$_2$)$_2$, P(O)(-N(alkyl)$_2$)(-N(alkyl)$_2$), P(O)(-O-aryl)$_2$, P(O)(-NH-aryl)$_2$, P(O)(-O-alkyl)(-O-aryl), P(O)(-NH-alkyl)(-O-aryl), P(O)(-O-alkyl)(-NH-aryl), P(O)(-NH-alkyl)(-NH-aryl), in particularly -C(O)OH, -C(O)NH(alkyl), C(O)NH(aryl), CON(alkyl)$_2$, or any group which induces a neighboring group effect via steric, ionic or bonding interactions as defined above.

The terms "rhodamine 4'-isomer" or "4'-isomer", are used herein as an abbreviated designation of the claimed rhodamine compounds which are, i.a., characterized by the presence of a substituent group Z at position 4' which is located next to the benzene ring position carrying the COOH group which is involved in spirolactone formation and, thus, immediately adjacent to said COOH group.

The following scheme illustrates the numbering of positions as used in the present application.

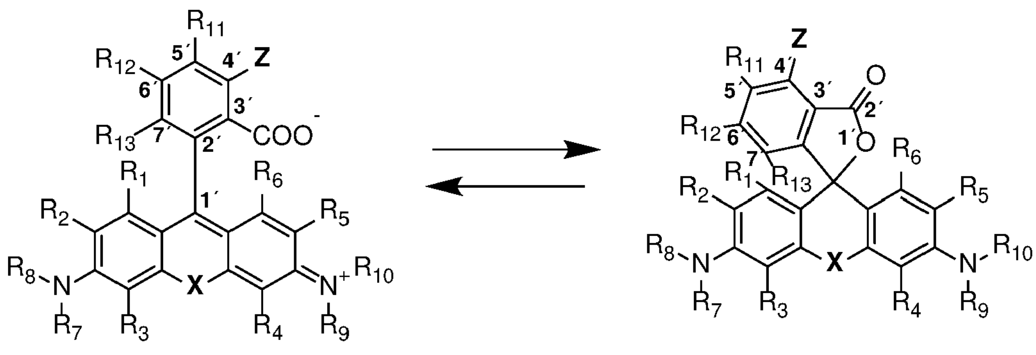
Zwitterion form
Spirolactone form

The term "substituted" as used herein, generally refers to the presence of one or more substituents, in particular substituents selected from the group comprising straight or branched alkyl, in particular $C_1$-$C_4$ alkyl, e.g. methyl, ethyl, propyl, butyl; isoalkyl, e.g. isopropyl, isobutyl (2-methylpropyl); secondary alkyl group, e.g. sec-butyl (but-2-yl); *tert*-alkyl

group, e.g. *tert*-butyl (2-methylpropyl). Additionally, the term "substituted" may refer here to alkyl groups having at least one deuterium-, fluoro-, chloro- or bromo substituent instead of hydrogen atoms, or methoxy, ethoxy, 2-(alkyloxy)ethyloxy groups (alk-$OCH_2CH_2O$), and, in a more general case, oligo(ethylenglycol) residues of the art alk$(OCH_2CH_2)_nOCH_2CH_2$-, where alk = $CH_3$, $C_2H_5$, $C_3H_7$, $C_4H_{10}$, and $n$ = 1-12.

The term "alkyl" refers to any alkyl group selected from the group comprising straight or branched alkyl, more specifically $C_1$-$C_{20}$ alkyl, $C_1$-$C_{12}$ alkyl, or $C_1$-$C_6$ alkyl, e.g. methyl, ethyl, propyl, butyl; isoalkyl, e.g. isopropyl, isobutyl (2-methylpropyl); secondary alkyl group, e.g. sec-butyl (but-2-yl); *tert*-alkyl group, e.g. *tert*-butyl (2-methylpropyl) etc. A bulky alkyl group may comprise, e.g., a large and/or highly branched alkyl group such as a tert.-alkyl group.

The term "alkenyl" refers to any alkenyl group selected from the group comprising straight or branched alkenyl, more specifically $C_1$-$C_{20}$ alkenyl, $C_1$-$C_{12}$ alkenyl, or $C_1$-$C_6$ alkenyl, e.g. an alkenyl group corresponding to one of the exemplary alkyl groups mentioned above. A bulky alkenyl group may comprise, e.g., a large and/or highly branched alkenyl group such as a tert.-alkenyl group.

The terms "aryl" or "aromatic group", as used herein, generally refer to an unsubstituted or substituted mono-, bi- or tricyclic carbocyclic ring system having one, two or three aromatic rings. Representative but not limiting examples are including but not limited to phenyl, naphthyl, anthryl, azulyl, tetrahydronaphthyl, indanyl and indenyl.

The terms "aromatic heterocyclic group" or "heteroaromatic group", as used herein, generally refer to an unsubstituted or substituted cyclic aromatic radical (residue) having from 5 to 10 ring atoms of which at least one ring atom is selected from S, O and N; the radical being joined to the rest of the molecule via any of the ring atoms. Representative, but not limiting examples are furyl, thienyl, pyridinyl, pyrazinyl, pyrimidinyl, pyrrolyl, imidazolyl, thiazolyl, oxazolyl, isooxazolyl, thiadiazolyl, oxadiazolyl, quinolinyl and isoquinolinyl.

The term "activated ester", as used herein, generally refers to a carboxyl group substituted with a group which renders it more reactive with nucleophiles such as, but not limited to, free amino groups of peptides, polyaminoacids, polysaccharides, or analytes under such conditions that no interfering side reactions with other reactive groups of the nucleophile-carrying substance can usefully occur. Examples of such substituent groups that form activated esters directly or by introduction of the linker include N-succinimidyl, sulfo-N-succinimidyl, 1-benzotriazolyl, and the like.

The term "a positional isomer", as used herein, generally refers to regioisomers of the discussed structures where a position of a functional group or other substituent on a parent structure changes, leading to different bonding patterns. The chemical composition and elemental analysis results of positional isomers are identical.

The term "linker", as used herein, generally refers to a straight or branched alkyl chain which may be substituted by one or more functional groups and may include heteroatoms and/or aromatic groups and is connecting a targeting moiety (i.e. a reactive group or ligand) and fluorescent dye. More specifically, the linker may comprise or represent one or more of the following groups: $C_{n+1}$ alkyl linear or branched, e.g. methyl, ethyl, propyl, butyl; isoalkyl, e.g. isopropyl, isobutyl (2-methylpropyl); secondary alkyl group, e.g. sec-butyl (but-2-yl); *tert*-alkyl group, e.g. *tert*-butyl (2-methylpropyl), hydroxylated alkyl $-(CH_2O)_n$-linear or branched, fluorinated alkyl $-(CHF)_{n+1}$- or - $(CF_2)_{n+1}$-, alkenyl $-(CH_2)_k-(CH=CH)_{n+1}-(CH_2)_m$- with single or multiple double bonds, alkinyl $-(CH_2)_k-(C{\equiv}C)_{n+1}-(CH_2)_m$- with single or multiple triple bonds, tris(hydroxymethyl)aminomethane, $O(alkyl)_2$, $O(aryl)_2$, $S(alkyl)_2$, $S(aryl)_2$, $S(O)(alkyl)_2$, $S(O)(aryl)_2$, $S(O)_2(alkyl)_2$, $S(O)_2(aryl)_2$, $N(alkyl)_2$, $NH(aryl)_2$, $C(O)(alkyl)_2$, $C(O)(aryl)_2$, $P(O)(\text{-O-alkyl})_2$, $P(O)(\text{-O-aryl})_2$, $C(O)NH(alkyl)_2$. Here k, n and m = 0 - 20.

The present invention further relates to rhodamine 4'-isomer derivatives or probes comprising a rhodamine 4'-isomer having one of the general formulae A-H as described above,

wherein Z is selected from O(alkyl), O(aryl), S(alkyl), S(aryl), S(O)(alkyl), S(O)(aryl), $S(O)_2$(alkyl), $S(O)_2$(aryl), $S(O)_2$(-O-alkyl), $S(O)_2$(-O-aryl), $S(O)_2$NH(alkyl), $S(O)_2$NH(aryl), $S(O)_2N(alkyl)_2$, $S(O)_2N(aryl)_2$,$S(O)_2$N(alkyl)(aryl), NH(alkyl), $N(alkyl)_2$, N(alkyl)(aryl), NH(aryl), $N(aryl)_2$, C(O)O(alkyl), C(O)O(aryl), C(O)(alkyl), C(O)(aryl), P(O)OH(-NH-alkyl), P(O)OH(-O-alkyl), P(O)OH(-NH-aryl), P(O)OH(-O-aryl), $P(O)(\text{-O-alkyl})_2$, $P(O)(\text{-NH-alkyl})_2$, $P(O)OH(\text{-N(alkyl)}_2)$, $P(O)OH(\text{-N(aryl)}_2)$, $P(O)(\text{-N(aryl)}_2)_2$, $P(O)(\text{-N(alkyl)}_2)(\text{-N(alkyl)}_2)$, $P(O)(\text{-O-aryl})_2$, $P(O)(\text{-NH-aryl})_2$, P(O)(-O-alkyl)(-O-aryl), P(O)(-NH-alkyl)(-O-aryl), P(O)(-O-alkyl)(-NH-aryl), P(O)(-NH-alkyl)(-NH-aryl), in particular -C(O)OH, -C(O)NH(alkyl), C(O)NH(aryl), $CON(alkyl)_2$, or any group which induces a neighboring group effect via steric, ionic or bonding interactions as defined above,

which rhodamine 4'-isomer is coupled to at least one reactive group or ligand which is capable to interact with or bind to other molecules. Said reactive group or ligand may be coupled to the rhodamine 4'-isomer fluorophore either directly or via a linker as defined above.

Typically, the linker comprises or consists of a straight or branched alkyl chain with 1- 21 C atoms which may be substituted by one or more functional groups and may include heteroatoms and/or aromatic groups, and preferably represents a moiety selected from the following group:

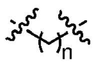
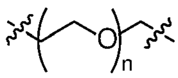
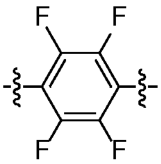
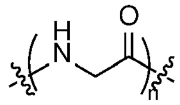
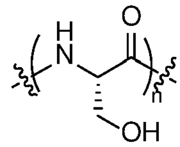

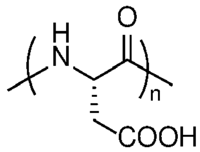
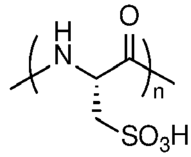
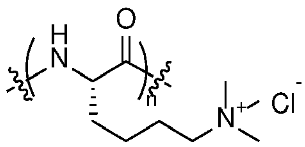
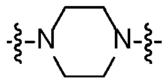

with n = an integer of 1-20.

More specifically, said reactive group may be selected from the group comprising an activated ester, an amine, a thiol, an azide, an ethyne, a maleimide, a tetrazine, N-hydroxysuccinimide or an alcohol group, or wherein the ligand is a ligand which binds specifically to a protein, peptide, nucleotide or nucleic acid, carbohydrate, iodoacetamide or which is capable to effect or participate in chelation of $NH_4^+$ or metal ions, in particular $Li^+$, $Na^+$, $K^+$, $Cs^+$, $Rb^+$, $Cu^+$, $Tl^+$, $Hg^+$, $Ag^+$, $Au^+$, $Ca^{2+}$, $Ba^{2+}$, $Sr^{2+}$, $Be^{2+}$,$Zn^{2+}$, $Ni^{2+}$, $Cu^{2+}$, $Mg^{2+}$, $Co^{2+}$, $Fe^{2+}$, $Mn^{2+}$, $Pt^{2+}$, $Cd^{2+}$, $Hg^{2+}$, $Sn^{2+}$, $Pb^{2+}$,$Au^{3+}$ $Cr^{3+}$, $Co^{3+}$, $Fe^{3+}$, $Al^{3+}$, $Ga^{3+}$, $In^{3+}$,$Sc^{3+}$, $Ti^{3+}$, $Tl^{3+}$, $V^{3+}$, $Y^{3+}$, $La^{3+}$, $Pt^{4+}$, $Pb^{4+}$, $Ce^{4+}$, $Ge^{4+}$, $Th^{4+}$, $Zr^{4+}$, $U^{4+}$.

In a specific embodiment of the rhodamine 4'-isomer derivative according to the present invention, the reactive group or ligand is coupled directly or via a linker to group Z.

In this case, a linker may be also provided by Z, if said group Z as defined above already comprises a linking group, in particular as defined above.

In a preferred embodiment thereof, the reactive group or ligand is coupled by an amide bond formed between a carboxyl functional group or amine functional group provided by Z and an amine functional group or carboxyl functional group provided by the linker or ligand.

However, in other specific embodiments, the reactive group or ligand may be also coupled to other coupling sites on the same ring or other rings of the rhodamine structure, in particular at positions 6' or 5', preferably at position 6'.

In some other specific embodiments, the ligand of the rhodamine 4'-isomer derivative which is capable to interact with or bind to other molecules is benzylguanine and the protein is SNAP-tag, or the ligand is benzylcytosine and the proteine is CLIP-tag, or the ligand is a primary alkyl chloride and the protein is Halo-tag, or the ligand is trimethoprim and the protein is dihydrofolate reductase.

The rhodamine 4'-isomer derivative or probe of the invention has an increased $D_{50}$ value compared to the $D_{50}$ value of a reference isomer derivative, in particular a 5'- or 6' isomer derivative, which is typically higher than that of the corresponding 5'- or 6'-isomer derivative by a difference of at least 10.

Preferably, the absolute $D_{50}$ value of the 4'-isomer derivative or probe is a numeric value of at least 15, preferably at least 20 or 25, and generally is in the range from 15 to 85, typically in the range from 20 to 75 or from 25 to 70.

More specifically, if X = O in the above general formulae A-H, preferably the absolute $D_{50}$ value of the 4'-isomer derivative or probe is in the range from 20 to 30 or 20 to 35, if X = $CR_{14}R_{15}$, preferably the absolute $D_{50}$ value of the 4'-isomer derivative or probe is in the range from 55 to 70, if X = $SiR_{14}R_{15}$ or $GeR_{14}R_{15}$, preferably the absolute $D_{50}$ value of the 4'-isomer derivative or probe is in the range from 70 to 80 or 70 to 85.

A further aspect of the present invention relates to conjugates comprising a rhodamine 4'-isomer or rhodamine 4'-isomer derivative as described above which is coupled to or associated with a molecule selected from the group comprising a peptide, protein, in particular an enzyme or antibody, tubulin or actin, a nucleotide, nucleic acid sequence, including DNA and RNA, a lipid, carbohydrate, an organic or inorganic polyphosphate, a pharmaceutical drug, a metabolite, a reactive oxygen species (ROS), $NH_4^+$, a metal ion, in particular Li+, $Na^+$, $K^+$, $Cs^+$, $Rb^+$, $Cu^+$, $Tl^+$, $Hg^+$, $Ag^+$, $Au^+$, $Ca^{2+}$, $Ba^{2+}$, $Sr^{2+}$, $Be^{2+}$, $Zn^{2+}$, $Ni^{2+}$, $Cu^{2+}$, $Mg^{2+}$, $Co^{2+}$, $Fe^{2+}$, $Mn^{2+}$, $Pt^{2+}$, $Cd^{2+}$, $Hg^{2+}$, $Sn^{2+}$, $Pb^{2+}$, $Au^{3+}$ $Cr^{3+}$, $Co^{3+}$, $Fe^{3+}$, $Al^{3+}$, $Ga^{3+}$, $In^{3+}$,$Sc^{3+}$, $Ti^{3+}$, $Tl^{3+}$,$V^{3+}$,$Y^{3+}$, $La^{3+}$, $Pt^{4+}$, $Pb^{4+}$, $Ce^{4+}$, $Ge^{4+}$, $Th^{4+}$, $Zr^{4+}$, $U^{4+}$, or complex thereof.

The conjugation may comprise formation of at least one covalent chemical bond or at least one molecular complex with a chemical entity or substance, such as amine, carboxylic acid, aldehyde, alcohol, aromatic compound, heterocycle, dye, amino acid, amino acid residue coupled to any chemical entity or molecule, in particular as listed above.

The present invention provides a new class of rhodamine positional isomers, i.e. rhodamine 4'-isomers as defined above, and derivatives thereof that (i); possess high photostability (ii); have high extinction coefficients and high quantum yields (iii); can be derivatized with different molecules (iv); show little or no background binding to cellular biomolecules (v) are extremely membrane-permeable due to a neighboring group effect illustrated in the general structure of the new 4'-isomer dyes shown below

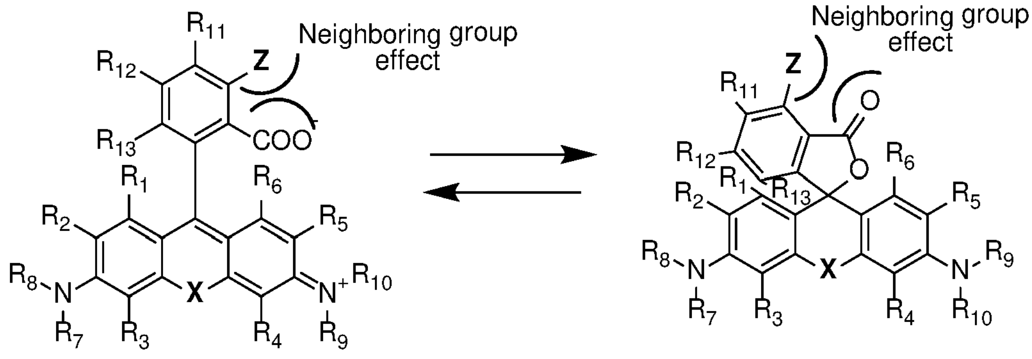


Zwitterion form        Spirolactone form

**Scheme 3**. Neighbouring group effect

As mentioned, the essential feature of these novel fluorescent dyes is the presence of neighbouring groups Z in the benzene ring of the fluorophore. This provokes a specific effect, herein denoted as neighbouring group effect (NGE), which is a superposition of inductive, hydrogen bonding and steric effects (with the latter effects typically more relevant and a merely inductive effect typically will not be sufficient). For example, a significant NGE effect will be typically induced by groups Z which are bulky (steric interactions) and/or which are able to form hydrogen bonding with the carboxyl group responsible for spirolactonization. The exemplary groups Z listed above exhibit one or more of these properties.

This neighbouring group effect is only present in the 4'-isomers of rhodamines and influences the carboxyl group present in the benzene ring, changes spirolactonization rate and shifts equilibrium between spirolactone and zwitterion (see Fig. 1a). Said NGE effect can be detected by NMR or HPLC methods (Fig. 1): Rhodamine 4'-isomers containing NGE show significant NMR chemical shift of the interacting atoms which are not bound via covalent chemical bond and increased HPLC retention times. Most importantly the effect of NGE can be observed by measuring a change of $D_{50}$ value compared to $D_{50}$ value of other positional isomers and similarly with increased $IC_{50}$ values of conjugates. The NGE is especially profound if a linker or a ligand comprising an amine functional group is attached to a carboxylic group Z via formation of an amide bond resulting in conversion of the carboxylic group to carboxamide.

Generally, the NGE of 4'-isomer derivatives is higher than that of corresponding 4'-isomers as such because of the steric and electronic effects induced by substitutents.

Surprisingly, this neigbouring group effect results in dramatically increased cell membrane permeability while keeping all photophysical properties of the compounds almost unchanged.

## General synthesis of the novel fluorescent dyes, probes and conjugates of the invention

### A. Synthesis of rhodamine 4'-isomer dyes

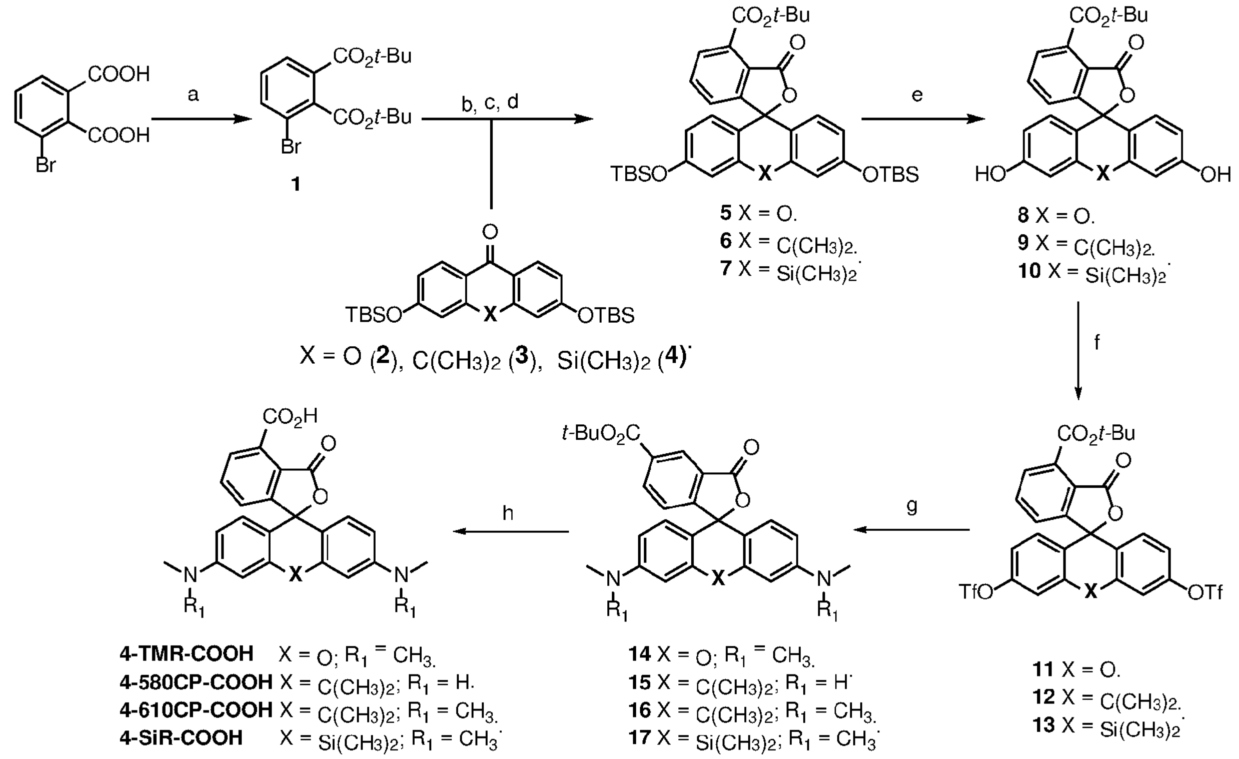


1

X = O (**2**), $C(CH_3)_2$ (**3**), $Si(CH_3)_2$ (**4**).

**5** X = O.
**6** X = $C(CH_3)_2$.
**7** X = $Si(CH_3)_2$.

**8** X = O.
**9** X = $C(CH_3)_2$.
**10** X = $Si(CH_3)_2$.

**4-TMR-COOH** X = O; $R_1$ = $CH_3$.
**4-580CP-COOH** X = $C(CH_3)_2$; $R_1$ = H.
**4-610CP-COOH** X = $C(CH_3)_2$; $R_1$ = $CH_3$.
**4-SiR-COOH** X = $Si(CH_3)_2$; $R_1$ = $CH_3$.

**14** X = O; $R_1$ = $CH_3$.
**15** X = $C(CH_3)_2$; $R_1$ = H.
**16** X = $C(CH_3)_2$; $R_1$ = $CH_3$.
**17** X = $Si(CH_3)_2$; $R_1$ = $CH_3$.

**11** X = O.
**12** X = $C(CH_3)_2$.
**13** X = $Si(CH_3)_2$.

**Scheme 4.** Synthesis of exemplary rhodamine 4'-isomer dyes

Reaction and condition: (a) Isobutylene, cat. $H_2SO_4$, DCM; (b) n-BuLi, ~-116°C, pentane:THF (1:2) (c) Corresponding ketone **2**,**3** or **4**, ~-116°C to r.t. (d) $H_2O$, AcOH, rt. (e) TBAF, THF. 0°C to rt. (f) $Tf_2O$, Pyridine, DCM 0°C to rt. (g) $HNMe_2$ or BocNHMe, $Pd_2(dba)_3$, Xantphos, $CsCO_3$. 1,4-dioxane, 100°C, 4-18h. (h) TFA, DCM, rt. 1-3h.

The general strategy for the synthesis of the novel rhodamine 4 'isomers is outlined above. More details with respect to the synthesis of exemplary compounds are provided in Synthesis Example A below.

**B. Synthesis of rhodamine 4'-isomer fluorescent probes**

Generally, a fluorescent probe is comprised of the fluorophore molecule connected to the ligand, which interacts with the target of interest. The fluorescent probe absorbs light of a specific wavelength and emits light of a different, typically longer, wavelength and it acts as a marker of target of interest for analysis with fluorescent microscopy or spectroscopy. Fluorophore defines the operational wavelengths and ligand defines the target of interest. Fluorophore and the ligand is connected by covalent bond directly or through the linker. Optimal length and hydrophilicity of the linker is usually specific for each target of interest.

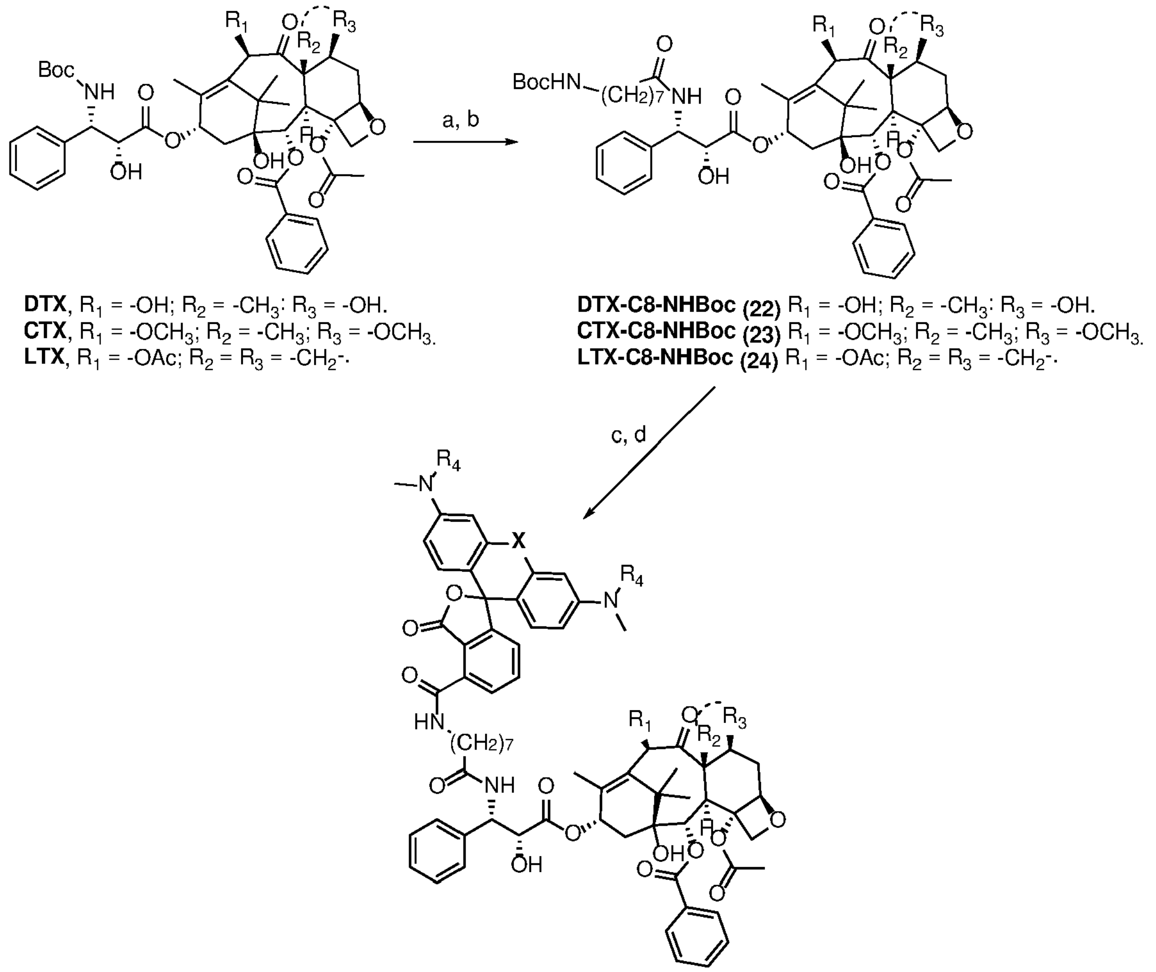


**DTX**, $R_1$ = -OH; $R_2$ = $-CH_3$: $R_3$ = -OH.
**CTX**, $R_1$ = $-OCH_3$; $R_2$ = $-CH_3$; $R_3$ = $-OCH_3$.
**LTX**, $R_1$ = -OAc; $R_2$ = $R_3$ = $-CH_2$-.

**DTX-C8-NHBoc** (22) $R_1$ = -OH; $R_2$ = $-CH_3$: $R_3$ = -OH.
**CTX-C8-NHBoc** (23) $R_1$ = $-OCH_3$; $R_2$ = $-CH_3$; $R_3$ = $-OCH_3$.
**LTX-C8-NHBoc** (24) $R_1$ = -OAc; $R_2$ = $R_3$ = $-CH_2$-.

**4-TMR-DTX** (25) $R_1$ = -OH; $R_2$ = $-CH_3$; $R_3$ = -OH; $R_4$ = $-CH_3$; X = O;
**4-TMR-CTX** (26) $R_1$ = $-OCH_3$; $R_2$ = $-CH_3$; $R_3$ = $-OCH_3$; $R_4$ = $-CH_3$; X = O;
**4-TMR-LTX** (27) $R_1$ = -OAc; $R_2$ = $R_3$ = $-CH_2$-; $R_4$ = $-CH_3$; X = O;
**4-580CP-LTX** (28) $R_1$ = -OAc; $R_2$ = $R_3$ = $-CH_2$-; $R_4$ = -H; X = $C(CH_3)_2$;
**4-610CP-DTX** (29) $R_1$ = -OH; $R_2$ = $-CH_3$; $R_3$ = -OH; $R_4$ = $-CH_3$; X = $C(CH_3)_2$;
**4-610CP-CTX** (30) $R_1$ = $-OCH_3$; $R_2$ = $-CH_3$; $R_3$ = $-OCH_3$; $R_4$ = $-CH_3$; X = $C(CH_3)_2$;
**4-610CP-LTX** (31) $R_1$ = -OAc; $R_2$ = $R_3$ = $-CH_2$-; $R_4$ = $-CH_3$; X = $C(CH_3)_2$;
**4-SiR-DTX** (32) $R_1$ = -OH; $R_2$ = $-CH_3$; $R_3$ = -OH; $R_4$ = $-CH_3$; X = $Si(CH_3)_2$;
**4-SiR-CTX** (33) $R_1$ = $-OCH_3$; $R_2$ = $-CH_3$; $R_3$ = $-OCH_3$; $R_4$ = $-CH_3$; X = $Si(CH_3)_2$;
**4-SiR-LTX** (34) $R_1$ = -OAc; $R_2$ = $R_3$ = $-CH_2$-; $R_4$ = $-CH_3$; X = $Si(CH_3)_2$;

**Scheme 5.** Synthesis of exemplary rhodamine 4'-isomer fluorescent probes

The general strategy for the synthesis of rhodamine 4 'isomer based fluorescent probes is outlined above. More details with respect to the synthesis of exemplary probes/conjugates are provided in Synthesis Example B below.

## Applications

In view of the advantageous properties of the novel fluorescent dyes according to the invention as outlined above, a further aspect of the present invention relates to their use as fluorescent dyes, in particular cell permeable fluorescent dyes, in a variety of applications.

Said fluorescent dyes may be a rhodamine 4'-isomer described above as such or a rhodamine 4'-isomer derivative comprising such a rhodamine 4'-isomer coupled to at least one reactive group or ligand which is capable to interact with or bind to other molecules. Said reactive group or ligand may be coupled to the rhodamine 4'-isomer

fluorophore either directly or via a linker as defined above.

Said reactive group may be selected from the group comprising an activated ester, an amine, a thiol, an azide, an ethyne, a maleimide, a tetrazine, N- hydroxysuccinimide or an alcohol group, or wherein the ligand is a ligand which binds specifically to a protein, peptide, nucleotide or nucleic acid, carbohydrate, iodoacetamide or which is capable to effect or participate in chelation of $NH_4^+$ or metal ions, in particular $Li^+$, $Na^+$, $K^+$, $Cs^+$, $Rb^+$, $Cu^+$, $Tl^+$, $Hg^+$, $Ag^+$, $Au^+$, $Ca^{2+}$, $Ba^{2+}$, $Sr^{2+}$, $Be^{2+}$, $Zn^{2+}$, $Ni^{2+}$, $Cu^{2+}$, $Mg^{2+}$, $Co^{2+}$, $Fe^{2+}$, $Mn^{2+}$, $Pt^{2+}$, $Cd^{2+}$, $Hg^{2+}$, $Sn^{2+}$, $Pb^{2+}$, $Au^{3+}$ $Cr^{3+}$, $Co^{3+}$, $Fe^{3+}$, $Al^{3+}$, $Ga^{3+}$, $In^{3+}$,$Sc^{3+}$,$Ti^{3+}$, $Tl^{3+}$,$V^{3+}$,$Y^{3+}$, $La^{3+}$, $Pt^{4+}$, $Pb^{4+}$, $Ce^{4+}$, $Ge^{4+}$, $Th^{4+}$, $Zr^{4+}$, $U^{4+}$.

In the following, a number of suitable or preferred ligands for various main targets are listed:

| Target | Ligand |
|---|---|
| Actin | 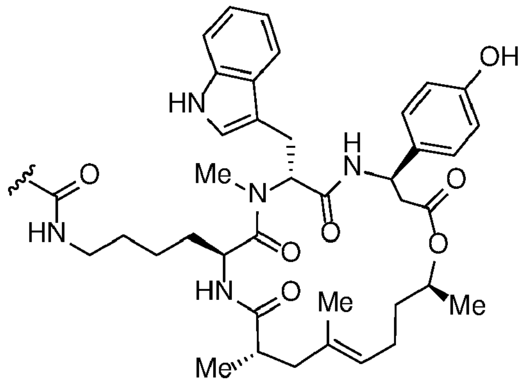<br>**Jasplakinolide derivatives**<br>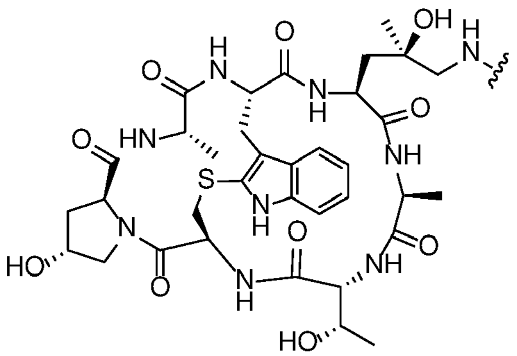<br>**Phalloidin derivatives** |
| Tubulin | 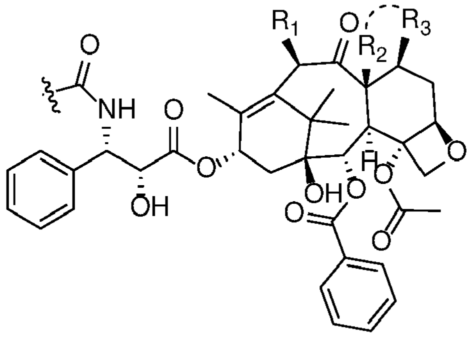<br>Paclitaxel, $R'_1$ = -OAc; $R_2$ = $-CH_3$: $R_3$ = -OH.<br>Docetaxel, $R'_1$ = -OH; $R_2$ = $-CH_3$: $R_3$ = -OH.<br>Cabazitaxel, $R'_1$ = $-OCH_3$; $R_2$ = $-CH_3$; $R_3$ = $-OCH_3$.<br>Larotaxel, $R'_1$ = -OAc; $R_2$ = $R_3$ = $-CH_2-$.<br>**Taxane derivatives** |

(continued)

| Target | Ligand |
|---|---|
| DNA | 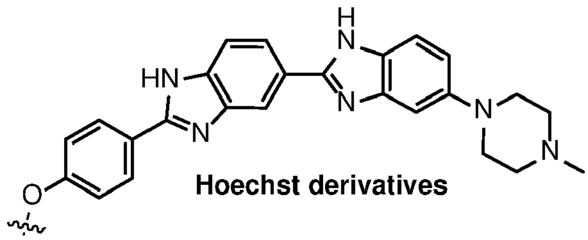<br>**Hoechst derivatives**<br>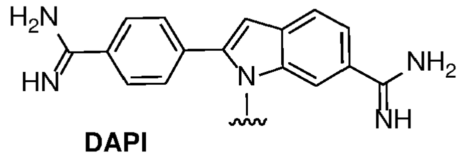<br>**DAPI**<br>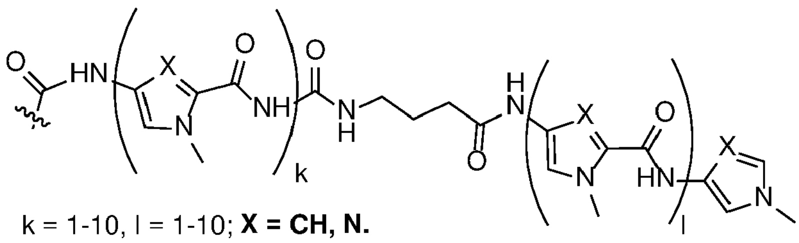<br>k = 1-10, l = 1-10; **X = CH, N.**<br>**Polyamide derivatives**<br>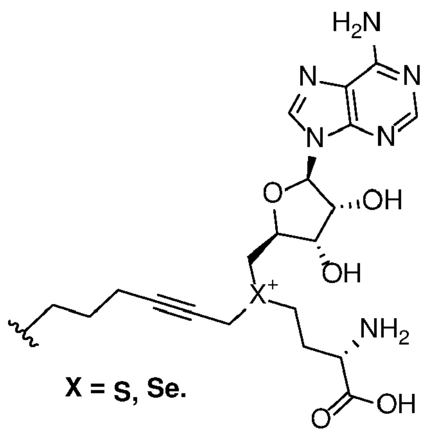<br>**X = S, Se.**<br>**AdoMet cofactor analogs** |

(continued)

| Target | Ligand |
|---|---|
| Pepsin, cathepsins D and E. | 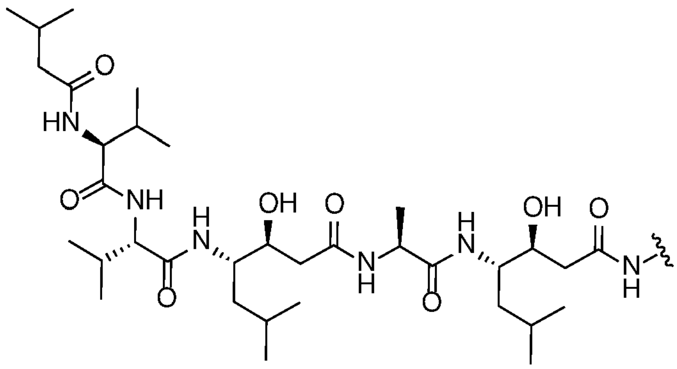 **Pepstatin derivatives** |
| Mitochondria | 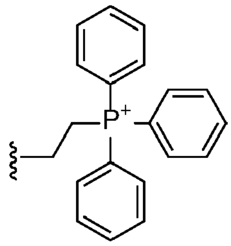 **Triphenylphosphonium derivatives** |
| FKBP12 | 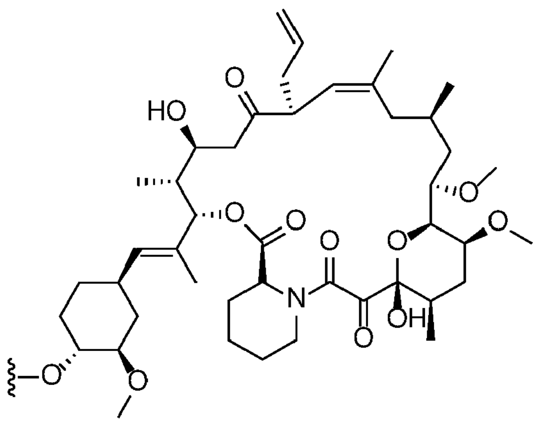 **FK506 derivatives** |
| Kinases | 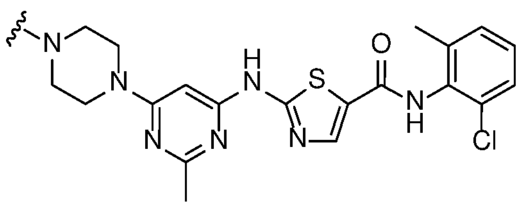 **Dasatinib derivatives** |
| Halo-tag | 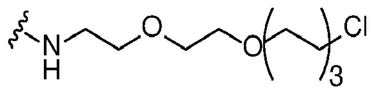 **Haloalkane derivatives** |

(continued)

| Target | Ligand |
|---|---|
| SNAP-tag | 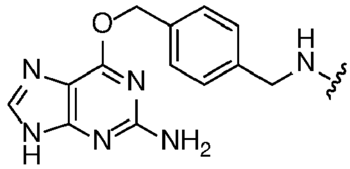<br>**Benzylguanine derivatives**<br>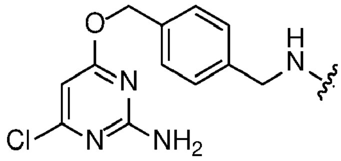<br>**Benzylchloropyrimidine derivatives** |
| CLI P-tag | 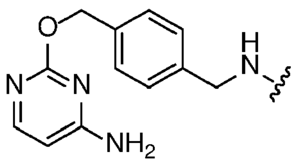<br>**Benzylcytosine derivatives** |
| ACP tag and MCP-tag | 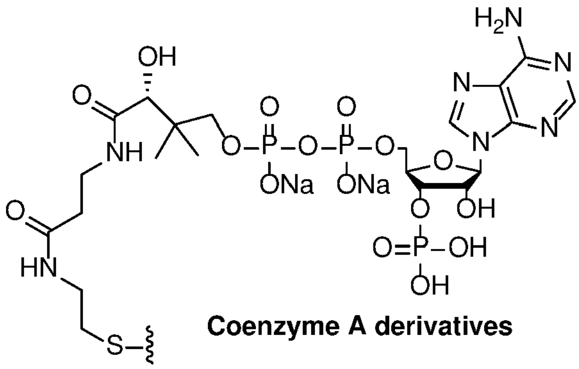<br>**Coenzyme A derivatives** |
| His-tag | 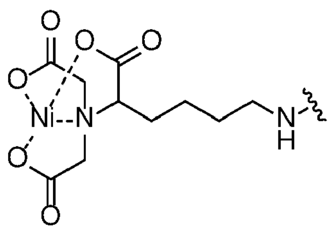<br>**Nitrilotriacetic acid derivatives** |
| Dihydrofolate reductase | 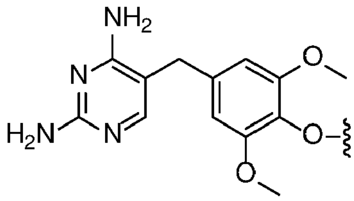<br>**Trimethoprim derivatives** |

(continued)

| Target | Ligand |
|---|---|
| Pooly ADP ribose polymerase (PARP). | 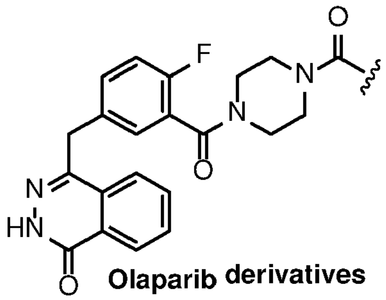 Olaparib derivatives |
| Azide containing DNA, protein, sugar or lipid | 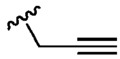 Terminal alkyne derivatives |
| | 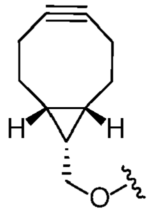 bicyclo[6.1.0]nonyne (BCN) derivatives |
| Terminal alkyne containing DNA, protein, sugar or lipid | 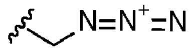 Azide derivatives |
| Trans-cyclooctene (TCO) containing DNA, protein, sugar or lipid | 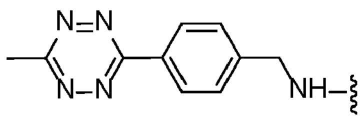 Methyltetrazine derivatives |
| Methyltetrazine containing DNA, protein, sugar or lipid | 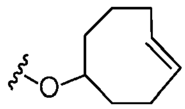 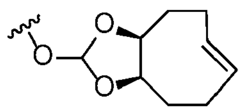 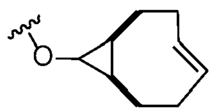 Trans-cyclooctene (TCO) derivatives |

In some specific applications, the ligand of the rhodamine 4'-isomer derivative which is capable to interact with or bind to other molecules is benzylguanine and the protein is SNAP-tag, or the ligand is benzylcytosine and the proteine is CLIP-tag, or the ligand is a primary alkyl chloride and the protein is Halo-tag, or the ligand is trimethoprim and the protein is dihydrofolate reductase.

The fluorescent dyes may used as such or as conjugates with various organic substances, e.g. an amino acid (naturally occurring or not), a peptide, protein, in particular an enzyme or antibody, tubulin or actin, a nucleotide, nucleic acid sequence, including DNA and RNA, a lipid, carbohydrate, an organic or inorganic polyphosphate, a pharmaceutical drug, a toxin, a metabolite, a reactive oxygen species (ROS), $NH_4^+$, a metal ion, in particular, Li+, $Na^+$, $K^+$, $Cs^+$, $Rb^+$, $Cu^+$, $Tl^+$, $Hg^+$, $Ag^+$, $Au^+$, $Ca^{2+}$, $Ba^{2+}$, $Sr^{2+}$, $Be^{2+}$, $Zn^{2+}$, $Ni^{2+}$, $Cu^{2+}$, $Mg^{2+}$, $Co^{2+}$, $Fe^{2+}$, $Mn^{2+}$, $Pt^{2+}$, $Cd^{2+}$, $Hg^{2+}$, $Sn^{2+}$, $Pb^{2+}$, $Au^{3+}$, $Cr^{3+}$, $Co^{3+}$, $Fe^{3+}$, $Al^{3+}$, $Ga^{3+}$, $In^{3+}$, $Sc^{3+}$, $Ti^{3+}$, $Tl^{3+}$, $V^{3+}$, $Y^{3+}$, $La^{3+}$, $Pt^{4+}$, $Pb^{4+}$, $Ce^{4+}$, $Ge^{4+}$, $Th^{4+}$, $Zr^{4+}$, $U^{4+}$, or complex thereof.

The conjugation may comprise formation of at least one covalent chemical bond or at least one molecular complex with a chemical entity or substance, such as amine, carboxylic acid, aldehyde, alcohol, aromatic compound, heterocycle, dye, amino acid, amino acid residue coupled to any chemical entity or molecule, in particular as listed above.

In a preferred embodiment of the present invention, the fluorescent dyes as described above or their conjugates

are used as cell permeable substances penetrating through membranes of living and fixed cells in vivo or in vitro.

In one specific embodiment of the present invention, the fluorescent dyes as described above or their conjugates are used as such or after photoactivation for tracking and monitoring dynamic processes in a sample or in an object.

In a more specific embodiment, a rhodamine 4'-isomer derivative coupled to tubulin, DNA, RNA, lipid, or actin is used for cell cycle monitoring in living cells or living tissues.

In another specific embodiment, rhodamine 4'-isomers derivatives having a high singlet oxygen ($^1O_2$) quantum yield, preferably at least 5 %, are used for chromophore-assisted light inactivation (CALI) and photodynamic therapy (PDT) in living cells and in living organisms such as humans, mammals, birds, fish, hemichordates, molluscs, tunicates, cnidarians, cephalochordates, flatworms, nematodes, annelids, tardigrades, reptiles, arthropods, echinoderms, chaetognathas, rotifers, frogs, plants, sponges or fungi.

The fluorescent dyes or their conjugates may be used as labels in microscopic, spectroscopic and other imaging techniques, in particular fluorescence microscopy and spectroscopy, in microfluidic devices, capillary electrophoresis, fluorescence activated cell sorting, DNA sequencing, sequence-specific genome labeling, analyte tracking techniques in vitro or in vivo.

Said imaging techniques comprise, e.g., stimulated emission depletion microscopy [STED], single molecule spectroscopy, single molecule switching (SMS) "nanoscopy" (diffraction unlimited optical resolution by using switching of the fluorescence of the single molecules, such as single molecule localization microscopy [SMLM], structured illumination microscopy (SIM), light-sheet microscopy, photoactivation localization microscopy [PALM, PALMIRA, fPALM], stochastic optical reconstruction microscopy [STORM]), fluorescence correlation spectroscopy [FCS] or fluorescence anisotropy spectroscopy, fluorescence recovery after photobleaching [FRAP], fluorescence lifetime imaging [FLIM], ground state depletion with individual molecular return [GSDIM], and fluorescence resonant energy transfer [FRET], correlative fluorescence - electron microscopy, correlative fluorescence - cryo-electron microscopy, microscale thermophoresis, fluorescence in situ hybridization (FISH), nuclear magnetic resonance spectroscopy.

Said analyte tracking techniques using a rhodamine 4'-isomer derivative of the present invention may be performed *in vitro* or *in vivo* and may be applied for tracking or monitoring a wide variety of analytes, in particular components of biological cells, such as proteins, lipids, carbohydrates, nucleic acids, sterols, nucleosides, nucleotides, polyphosphates, amino acids, polyamino acids, terpenes, lignines, chitin, chitosan, peptidoglycans, teichoic acids, lipoteichoic acid and vitamins.

In some exemplary and typical embodiments, a method or technique for tracking analytes using a rhodamine 4'-isomer derivative of the present invention comprises at least one of the following sequences of steps:

- interacting with or binding of the rhodamine 4'-isomer derivative to the analyte, which is a metal ion, resulting in metal ion chelation and in a corresponding change in fluorescence properties of the rhodamine 4'-isomer derivative, and using this change in fluorescence properties for metal ion imaging in vitro or in vivo, in particular in living cells and in living organisms such as humans, mammals, birds, fish, hemichordates, molluscs, tunicates, cnidarians, cephalochordates, flatworms, nematodes, annelids, tardigrades, reptiles, arthropods, echinoderms, chaetognathas, rotifers, frogs, plants, sponges or fungi;
- reacting of the rhodamine 4'-isomer derivative with reactive oxygen species (ROS) resulting in a corresponding change in fluorescence properties of the rhodamine 4'-isomer derivative, and using this change in fluorescence properties for ROS imaging in vitro or in vivo, in particular in living cells and in living organisms such as humans, mammals, birds, fish, hemichordates, molluscs, tunicates, cnidarians, cephalochordates, flatworms, nematodes, annelids, tardigrades, reptiles, arthropods, echinoderms, chaetognathas, rotifers, frogs, plants, sponges or fungi;
- selectively interacting or reacting of the rhodamine 4'-isomer derivative with an enzyme, resulting in a corresponding change in fluorescence properties of the rhodamine 4'-isomer derivative, and using this change in fluorescence properties for detection, quantification and imaging of enzymatic activity in vitro and in vivo;
- selectively interacting or reacting of the rhodamine 4'-isomer derivative with a lipid, organic and inorganic polyphosphate, protein, carbohydrate, metabolites, DNA or RNA, resulting in a corresponding change in fluorescence properties, and using this change in fluorescence properties for lipid, polyphosphate, protein, carbohydrate, metabolite, DNA or RNA imaging in vitro or in vivo;
- interacting or reacting of rhodamine 4'-isomers derivatives coupled to any drug or drug candidate with a target molecule or target site in a cell or tissue, resulting in a corresponding change in fluorescence properties, and using this change in fluorescence properties for drug-target interaction monitoring using fluorescence imaging or NMR in vitro or in vivo;
- interacting or reacting of a rhodamine 4'-isomer derivative with oxygen, fluoride or glucose through non-covalent complex or covalent bond formation, resulting in a corresponding change in fluorescence properties of the rhodamine 4'-isomer derivative, and using this change in fluorescence properties for oxygen, fluoride or glucose sensing in vitro or in vivo, in particular in living cells and in living organisms, such as humans, mammals, birds, fish, hemichordates, molluscs, tunicates, cnidarians, cephalochordates, flatworms, nematodes, annelids, tardigrades, reptiles,

arthropods, echinoderms, chaetognathas, rotifers, frogs, plants, sponges or fungi.

Brief description of the Figures

**Fig. 1** illustrates the neighboring group effect in the 4'isomers of rhodamines: **a,** Neighboring group effect shifts spirolactone-zwitterion equilibrium of rhodamines; **b,** Chemical shift differences of amide proton of TMR-LTX regioisomeric probes; **c,** Comparison of retention times of **TMR-LTX** regioisomeric probes in HPLC analysis with a SB-C18 column and isocratic elution conditions.

**Fig. 2** shows **a)** graphs representing the absorbance of **TMR-LTX** positional isomers at $\lambda_{max}$ versus dielectric constant (D) of 1,4-dioxane-water mixtures; **b)** $^{Dye}D_{50}$ values of positional isomers of **TMR-COOH** and $^{probe}D_{50}$ of **TMR-LTX.**

**Fig. 3** illustrates the tubulin labelling of mammalian cells: **a,** structures of the tubulin probes. **b,** Wide-field fluorescence microscopy of living primary fibroblasts stained with 100 nM TMR-LTX isomers for 1h at 37°C; Phase contrast image is shown at the bottom. **c,** Quantification of fluorescence signal in the cytoplasm of living cells stained with tubulin probes; **d,** Cytotoxicity of tubulin fluorescent probes presented as half maximal effective concentration (EC50) after 24h incubation at 37°C in growth media.

**Fig. 4** shows the performance of DNA and actin fluorescent probes based on rhodamines' positional isomers: **a,** Structure of DNA probes showing attachment point positional isomerism; **b,** Wide-field microscopy images of living primary fibroblasts stained with 100 nM 4/5/6-580CP-Hoechst for 1h at 37°C. Phase contrast image is shown at the bottom; **c,** Quantification of DNA probes' fluorescence signal in the nuclei; **d,** Structure of actin probes showing attachment point positional isomerism; **e,** Wide-field microscopy images of the phase contrast (bottom) and fluorescence channel (top). Living primary fibroblasts stained with 100 nM 4/5/6-610CP-JAS for 1h at 37°C; **f,** Quantification of 4/5/6-610CP-JAS fluorescence signal in the cytoplasm of living cells.

**Fig. 5** shows the reactivity of the Halo-tag substrates based on 610CP positional isomers in living U20S cells and in the cell extracts. The cells express genome-encoded vimentin-Halo-tag protein. The samples were fractionated on 4-15% gradient SDS-PAGE and vimentin-Halo-tag was visualized by fluorescence scanning in red channel. To compare the input, the image of the same gel stained with Coomassie blue is shown.

**Fig. 6** shows STED nanoscopy imaging of living cells stained with rhodamine 4'-isomer probes: **a,** Confocal and STED microscopy images of microtubules in living human fibroblasts stained with 100 nM **4-610CP-CTX** for 1h at 37°C; **b,** Confocal and STED microscopy images of microtubules in living human fibroblasts stained with 100 nM **4-SiR-CTX** for 1h at 37°C; **c,** Deconvolved STED image of human female primary fibroblast nucleus stained with 100 nM **4-580CP-Hoechst** showing the inactivated X chromosome (Xi). Inserts - confocal and STED microscopy zoomed-in images of Xi region; **d,** Two-colour STED no-wash image of primary human fibroblasts stained with 100 nM **4-610CP-JAS** and 10 nM **4-TMR-LTX** for 1h at 37°C.

The following Examples are provided to illustrate the present invention in more detail, however, without limiting the invention to the specific conditions and parameters thereof.

General Materials and Methods

All chemical reagents and solvents for synthesis were purchased from commercial suppliers (Sigma-Aldrich, Fluka, Acros) and were used without further purification. The composition of mixed solvents is given by the volume ratio (v/v).

NMR spectra were recorded at 25 °C with an Agilent 400-MR spectrometer at 400.06 MHz ($^1H$) and 100.60 MHz (13C), Bruker Avance III HD 500 spectrometer (av500) at 500.25 MHz ($^1H$) and 125.80 MHz ($^{13}C$), Varian Mercury Plus 300 spectrometer at 300.14 MHz ($^1H$), Varian INOVA 600 (I600) spectrometer at 599.74 MHz ($^1H$) and are reported in ppm. All $^1H$ and $^{13}C$ spectra are referenced to tetramethylsilane ($\delta$ = 0 ppm) using the residual signals of the solvents according to the values reported in literature (Gottlieb H.E.; Kotlyar V.; Nudelman A., J Org Chem 1997, 62, 7512-7515). Multiplicities of signals are described as follows: s = singlet, d = doublet, t = triplet, q = quartet, p = pentet, m = multiplet or overlap of non-equivalent resonances; br = broad signal. Coupling constants (J) are given in Hz.

ESI-MS were recorded on a Varian 500-MS spectrometer (Agilent). ESI-HRMS were recorded on a MICROTOF

spectrometer (Bruker) equipped with ESI ion source (Apollo) and direct injector with LC autosampler Agilent RR 1200. Liquid chromatography: Analytical LC-MS analysis was performed on an Agilent 1260 Infinity II LC/MS system equipped with an autosampler, diode array detector WR, fluorescence detector Spectra and Infinity Lab LC/MSD 6100 series quadruple with API electrospray. Analysis was done by using an Agilent Zorbax SB-C18 RRHT, 2.1 x 50 mm, 1.8 μm threaded column and SUPELCO Titan C18, 2.1 x 75 mm, 1.9 μm column with A: 25 mM $HCOONH_4$ (pH = 3.6) aqueous buffer and B: MeOH

Preparative HPLC was performed on an Interchim puriFlash 4250 2X preparative HPLC/Flash hybrid system (Article No. 1I5140, Interchim) with a 2 mL/ 5 mL injection loop, a 200-600 nm UV-Vis detector and an integrated ELSD detector (Article No. 1A3640, Interchim). Preparative column: Eurospher II 100-5 C18 5 μm, 250×20.0 mm (Article No.: 25PE181E2J, Knauer), typical flow rate: 25 mL/min, unless specified otherwise. Analytical TLC was performed on Merck Millipore ready-to-use plates with silica gel 60 (F254) (Cat. No. 1.05554.0001). Flash chromatography was performed on Biotage Isolera flash purification system using the type of cartridge and solvent gradient indicated.

*Maintenance and preparation of cells*

Human primary dermal fibroblasts and HeLa cells were cultured in high-glucose DMEM (Life Technologies, Cat. No. 31053-028) supplemented with GlutaMAX-1 (Life Technologies, Cat. No. 35050-038) and 10% fetal bovine serum (FBS, Life Technologies, Cat. No. 10270-106) in a humidified 5% $CO_2$ incubator at 37 °C. The cells were split every 3-4 days or at confluence. Cells were seeded in glass bottom 12-well plates (MatTek Corporation, Cat. No. P12G-1.0-14-F).

Cells were stained with the fluorescent probes in DMEM (Thermo Fisher Scientific, Cat. No. 31053-028) supplemented with 10% FBS (Thermo Fisher Scientific, Cat. No. 10082139) at 37 °C and 5% $CO_2$. If needed, the cells were washed 2 times with HBSS (Hanks' balanced salt solution, Lonza, Cat. No. BE10-527F) and imaged in DMEM with 10% FBS. No-wash experiments were performed in DMEM with 10% FBS after probe addition and incubation for the indicated period of time. For Airyscan experiments, cells were seeded in a 10-well plate (Greiner bio-one culture slides, PS, 75/25 mm. (Art. Nr.:543079)) 1 day before staining. The probes were applied to the cells in DMEM medium and after 1h or overnight incubation cells were imaged without washing.

*STED microscope with 775 nm depletion laser*

Comparative confocal and STED images were acquired on Abberior STED 775 QUAD scanning microscope (Abberior Instruments GmbH, Germany) equipped with 561 nm and 640 nm 40 MHz pulsed excitation lasers, a pulsed 775 nm 40 MHz STED laser, and an UPlanSApo 100x/1.40 Oil objective. The following detection windows were used: TMR/580CP channel 615 / 20 nm and 610CP/SiR channel 685 / 70 nm. In this setup, voxel size was 15 - 30 nm in xy plane and 150 nm in z-axis for STED images.

*Measurements of absorbance spectra in 1,4-dioxane-water mixtures*

Measurements of the absorbance changes in 1,4-dioxane-water mixtures were performed by pipetting 2μL stock solutions of dyes or probes in DMSO into a 96 glass bottom well plate (11 wells per sample) made from propylene (Corning 3364). To the wells going from right to left 300 μL of 1,4-dioxane-water mixtures containing 100%, 90%, 80%, 70%, 60%, 50%, 40%, 30%, 20%, 10% or 0% 1,4-dioxane was added (if needed, mixtures with 0.3% SDS are used, with an exception in 100% dioxane due to solubility issues). After incubation for 1 hour at room temperature, absorption of solutions in each well was recorded from 320 nm to 850 nm with wavelength step size of 1 nm on a multiwell plate reader Spark® 20M (Tecan). The background absorption of the glass bottom plate was measured in wells containing only 1,4-dioxane-water mixture with similar amount of DMSO and subtracted from the spectra of the samples. Plots of *$\lambda_{max}$ versus dielectric constant (D) of 1,4-dioxane-water mixtures* (G. Åkerlöf, A.O. Short, The Dielectric Constant of Dioxane-Water Mixtures between 0 and 80°, J. Am. Chem. Soc. 1936 58(7) 1241-1243*).* $D_{50}$ value was obtained by fitting to dose-response equation $EC_{50}$ as implemented in GraphPad 6.0 software:

$$A = A_0 + (A_{max} - A_0)/\left(1 + \left(\frac{D_{50}}{d}\right)^{Hill}\right) \qquad (1)$$

where $A_o$ - absorbance at $\lambda_{max}$ at $\varepsilon_r = 0$, $A_{max}$ - the highest reached absorbance at $\lambda_{max}$. *d* - dielectric constant of 1,4-dioxane-water mixture at a given point, *Hill* - Hill slope coefficient determining the steepness of a dose-response curve, $D_{50}$ - corresponds to d value that provokes half of the absorbance amplitude ($A_{max}$-$A_0$).

*Cell cycle analysis by imaging flow cytometry and $EC_{50}$ determination*

HeLa cells were grown in 6-well plates (~250.000 cells per well) for 24 h in the presence of the fluorescent probe in variable concentrations. The probes were dissolved in DMSO at 500 - 2000-fold stock concentration and added to the media of cultured cells at 500 - 2000-fold dilution accordingly. In parallel, the appropriate DMSO control samples were prepared by adding corresponding amount of DMSO volume to the separate well. Cells were processed according to the NucleoCounter® NC-3000™ two-step cell cycle analysis protocol for cells attached to T-flasks, cell culture plates or micro-carriers. In particular, the 250 μl lysis solution (Solution 10, Chemometec Cat. No. 910-3010) supplemented with 10 μg/ml DAPI (Solution 12, Chemometec Cat. No. 910-3012) was used per well, incubated at 37 °C for 5 min. Then 250 μl of stabilization solution (Solution 11, Chemometec Cat. No. 910-3011) was added. Cells were counted on a NucleoCounter® NC-3000™ in NC-Slide A2™ slides (Chemometec, Cat. No. 942-0001) loaded with ~30 μl of each of the cell suspensions into the chambers of the slide. Each time, ~10.000 cells in total were measured, and the obtained cell cycle histograms were analysed with ChemoMetec NucleoView NC-3000 software, version 2.1.25.8. All experiments were repeated three times and the results are presented as means with standard deviations. The $EC_{50}$ values were determined by plotting the percentage of cells in subG1 phase and fitting the curve in GraphPad Prism 6 to the following function:

$$Y = Y_{min} + (Y_{max} - Y_{min}) / \left(1 + \left(\frac{EC_{50}}{X}\right)^{Hill}\right) \quad (2)$$

where $Y_{min}$ - cells population percentage in subG1 phase then no probe was added, $Y_{max}$ - highest reachable percentage of cells in subG1 phase and shared value for all data sets equal to 69%. $X$ - cells population percentage in subG1 phase then added probe is at X concentration, *Hill* - Hill slope coefficient determining the steepness of a dose-response curve, $EC_{50}$ - the concentration of probe that provoking halfway of subG1 cells in a population between the baseline ($Y_{min}$) and maximum response ($Y_{max}$).

*Processing and visualization of acquired images*

All acquired or reconstructed images were processed and visualized using Fiji (J. Schindelin, I. Arganda-Carreras, E. Frise, V. Kaynig, M. Longair, T. Pietzsch, S. Preibisch, C. Rueden, S. Saalfeld, B. Schmid, J.Y. Tinevez, D.J. White, V. Hartenstein, K. Eliceiri, P. Tomancak, A. Cardona, Fiji: an open-source platform for biological-image analysis, Nat Methods 2012 9(7) 676-82). Line profiles were measured using the "straight line" tool with the line width set to 3 pixels.

For the signal measurements, image files were converted to TIF file using Fiji and analyzed with CellProfiler 3.1.8 (A.E. Carpenter, T.R. Jones, M.R. Lamprecht, C. Clarke, I.H. Kang, O. Friman, D.A. Guertin, J.H. Chang, R.A. Lindquist, J. Moffat, P. Golland, D.M. Sabatini, CellProfiler: image analysis software for identifying and quantifying cell phenotypes, Genome Biol 2006 7(10) R100), where the pipeline identified the nuclear region and measured the mean signal in this region. Background signal was measured in the region which is 3 pixels (450 nm) away from the nulear border and 7 pixels (1050 nm) wide. The background subtracted signal was processed with GraphPad Prism 6.

Actin/tubulin cytosolic signal was estimated using CellProfiler v.3.1.8. Briefly, probe channel was smoothed using median filter, nuclei were identified in DAPI channel and were used as seeds to find cell outlines in a smoothed actin channel. Background was defined as a lower quartile of pixel intensity in the area not covered by cells in the original probe channel. The background was subtracted from the original probe channel, and actin/tubulin staining was measured as mean pixel intensity per object in background-corrected probe channel. Statistical analysis performed by GraphPad Prism 6.

*Labeling of Halo-tagged vimentin in living cells and extracts*

For assessing living cell staining, U20S cells expressing vimentin-Halo-tag were grown on 35 mm diameter plastic plates in DMEM with 10% FBS. 100 nM x-610CP-Halo substrates were added and the cells were incubated at 37°C for 1 h. Then, the cells were washed twice with growth medium and twice with HBSS. 200 μl of CelLytic M solution (Sigma), supplemented with complete EDTA-free protease inhibitor cocktail (Sigma) was added, and the cells were allowed to lyse for 15 min. at room temperature with gentle agitation. The solution was collected and centrifuged for 15 min. at 13000 rpm (4°C). 180 μl of supernatant was mixed with 60 μl 4× SDS-PAGE sample buffer and boiled for 5 min.

To assess reactivity in the cell extracts, the cells were lysed as described above, and the lysates were incubated with 100 nM HaloTag substrates for 30 min. at 37°C before boiling. The samples were fractionated in 4-15% gradient.

SYNTHESIS EXAMPLE A

*Synthesis of rhodamine 4'-isomer dyes*

**A.1 Di-*tert*-butyl 3-bromophthalate (1):**

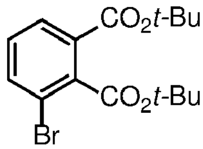

3-Bromophtalic acid (1.0 g, 4.08 mmol) was suspended in DCM (15 mL) in a sealable pressure glass tube. The mixture was cooled in a NaCl/ice bath and ~10 mL of isobutylene gas was condensed in the mixture. Catalytic amount of concentrated sulphuric acid (0.1 mL, 1.87 mmol) was added to the stirred and cooled reaction mixture and the pressure tube was tightly sealed. Reaction mixture was stirred at room temperature for 48 h, during this time the suspension became a clear solution. Then reaction mixture was cooled in ice bath and the tube was carefully opened with release of pressure. The resulting solution was poured to saturated $NaHCO_3$ solution (50 mL), extracted with DCM (2 x 30mL). The organic extracts were combined and washed with water and brine, dried over $Na_2SO_4$. The product was isolated by flash column chromatography (Teledyne Isco RediSep Rf 40g, isocratic hold 5% of EtOAc in Hexane), fractions containing the product were evaporated to give 1.02g (70%) of white solid.
$^1$H NMR (400 MHz, cdcl3) δ 7.79 (dd, *J* = 7.8, 1.1 Hz, 1H), 7.67 (dd, *J* = 8.0, 1.1 Hz, 1H), 7.23 (t, *J* = 7.9 Hz, 1H), 1.61 (s, 9H), 1.56 (s, 9H).
$^{13}$C NMR (101 MHz, cdcl3) δ 165.9, 163.6, 137.9, 136.1, 131.6, 129.5, 128.5, 120.3, 83.0, 82.1, 28.1, 28.0.
ESI-MS, positive mode: m/z = 357.1, 359.1 $[M+H]^+$.
HRMS (ESI) calcd for $C_{16}H_{22}N_2BrO_4$ $[M+H]^+$ 357.0696, found 357.0698.

**A.2 Tert-butyl 3',6'-bis((tert-butyldimethylsilyl)oxy)-3-oxo-3H-spiro[isobenzofuran-1,9'-xanthene]-4-carboxylate (5):**

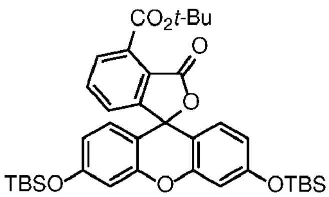

In a 25 mL round-bottom flask, a degassed solution of **1** (740 mg, 2.07 mmol, 2 eq.) in anhydrous THF (6 mL) and pentane (4 mL) was cooled to ~-116 °C (diethyl ether - liquid $N_2$). n-Butyllithium (1.3 mL of 1.6 M solution in hexanes, 2.07 mmol, 2 eq.) was carefully introduced through a needle. Clear solution quickly turned orange and then deep brown; it was stirred at ~-116 °C for 10 min, and the solution of ketone **2** (475 mg, 1.04 mmol, 1 eq.) (Martinez-Peragon, A.; Miguel, D.; Jurado, R.; Justicia, J.; Alvarez-Pez, J. M.; Cuerva, J. M.; Crovetto, L., Chemistry, 2014, 20 (2), 447-55) in THF (3 mL) was injected along the wall of the flask. Stirred at ~-116°C for 10 minutes and then flask was taken out of the cooling bath and left to warm to rt and stirred for further 30 min. The reaction mixture was quenched with water (10 mL), adjusted to pH ~ 5 with acetic acid, extracted with ethyl acetate (3×30 mL), the combined organic layers were washed with brine and dried over $Na_2SO_4$. The products were isolated by flash column chromatography (Büchi Reveleris HP silica 40 g; gradient 0% to 20% ethyl acetate - hexane.) In some cases additional purification was needed (Teledyne Isco RediSep Rf 40g, gradient 20% to 100% of DCM-Hexane). Product yield 433 mg (63%) of off-white solid.
$^1$H NMR (400 MHz, $d_6$-acetone) δ 7.86 (t, J = 7.6 Hz, 1H), 7.82 (dd, J = 7.6, 1.4 Hz, 1H), 7.40 (dd, J = 7.6,1.4 Hz, 1H), 6.81 (d, J = 2.4 Hz, 2H), 6.76 (d, J = 8.6 Hz, 2H), 6.70 (dd, J = 8.6, 2.4 Hz, 2H), 1.66 (s, 9H), 1.00 (s, 18H), 0.27 (s, 12H).
$^{13}$C NMR (101 MHz, $d_6$-acetone) δ 165.9, 165.0, 157.6, 153.9, 152.1, 135.2, 133.1, 129.3, 129.1, 126.0, 123.2, 116.9, 112.4, 107.4, 82.4, 81.3, 27.2, 25.0, 17.9, -5.3.
ESI-MS, positive mode: m/z = 661.3 $[M+H]^+$.

HRMS (ESI) calcd for $C_{37}H_{49}O_7Si_2$ $[M+H]^+$ 661.3011, found 661.3004.

**A.3 Tert-butyl 3,6-bis((tert-butyidimethylsilyl)oxy)-10,10-dimethyl-3'-oxo-3'H,10H-spiro[anthracene-9,1'-isobenzofuran]-4'-carboxylate (6):**

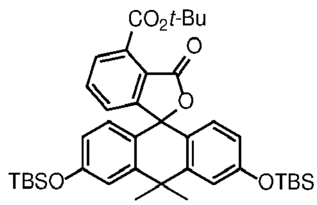

In a 25 mL round-bottom flask, a degassed solution of 1 (740 mg, 2.07 mmol, 2 eq.) in anhydrous THF (6 mL) and pentane (4 mL) was cooled to ~-116 °C (diethyl ether - liquid $N_2$). n-Butyllithium (1.3 mL of 1.6 M solution in hexanes, 2.07 mmol, 2 eq.) was carefully introduced through a needle. Clear solution quickly turned orange and then deep brown; it was stirred at ~-116 °C for 10 min, and the solution of ketone **3** (502 mg, 1.04 mmol, 1 eq.) (Grimm, J. B.; Sung, A. J.; Legant, W. R.; Hulamm, P.; Matlosz, S. M.; Betzig, E.; Lavis, L. D., ACS Chem Biol, 2013, 8(6), 1303-1310) in THF (3 mL) was injected along the wall of the flask. Stirred at ~-116°C for 10 minutes and then flask was taken out of the cooling bath and left to warm to rt and stirred for further 30 min. The reaction mixture was quenched with water (10 mL), adjusted to pH ~ 5 with acetic acid, extracted with ethyl acetate (3×30 mL), the combined organic layers were washed with brine and dried over $Na_2SO_4$. The products were isolated by flash column chromatography (Büchi Reveleris HP silica 40 g; gradient 0% to 20% ethyl acetate - hexane.) In some cases additional purification was needed (Teledyne Isco RediSep Rf 40g, gradient 20% to 100% of DCM-Hexane). Product yield 471 mg (66%) of off-white solid.
$^1H$ NMR (600 MHz, $cdcl_3$) $\delta$ 7.72 (dd, J = 7.6, 0.8 Hz, 1H), 7.58 (t, J = 7.6 Hz, 1H), 7.07 (d, J = 0.8 Hz, 1H), 7.05 (d, J = 2.0 Hz, 2H), 6.66 (d, J = 8.6 Hz, 2H), 6.61 (dd, J = 8.6, 2.0 Hz, 2H), 1.79 (s, 3H), 1.71 (s, 3H), 1.70 (s, 9H), 0.98 (s, 18H), 0.21 (s, 12H).
$^{13}C$ NMR (126 MHz, $cdcl_3$) $\delta$ 167.3, 165.3, 156.4, 156.1, 146.6, 134.1, 133.0, 129.2, 128.9, 125.7, 124.1, 123.2, 118.9, 117.4, 85.0, 83.3, 38.0, 34.9, 33.1, 28.1, 25.7, 18.3, -4.2,
ESI-MS, positive mode: m/z = 687.4 $[M+H]^+$.
HRMS (ESI) calcd for $C_{40}H_{55}O_6Si_2$ $[M+H]^+$ 687.3532, found 687.3523.

**A.4 Tert-butyl 3,7-bis((tert-butyldimethylsilyl)oxy)-5,5-dimethyl-3'-oxo-3'H,5H-spiro[dibenzo[b,e]siline-10,1'-isobenzofuran]-4'-carboxylate (7):**

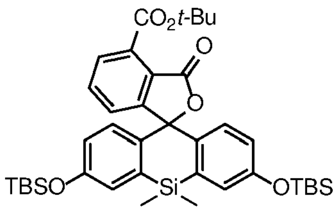

In a 25 mL round-bottom flask, a degassed solution of 1 (740 mg, 2.07 mmol, 2 eq.) in anhydrous THF (6 mL) and pentane (4 mL) was cooled to ~-116 °C (diethyl ether - liquid N2). n-Butyllithium (1.3 mL of 1.6 M solution in hexanes, 2.07 mmol, 2 eq.) was carefully introduced through a needle. Clear solution quickly turned orange and then deep brown; it was stirred at ~-116 °C for 10 min, and the solution of ketone **4** (520 mg, 1.04 mmol, 1 eq.) (Butkevich, A. N.; Belov, V. N.; Kolmakov, K.; Sokolov, V. V.; Shojaei, H.; Sidenstein, S. C.; Kamin, D.; Matthias, J.; Vlijm, R.; Engelhardt, J.; Hell, S. W., Chemistry - A European Journal, 2017, 23(50), 12114-12119) in THF (3 mL) was injected along the wall of the flask. Stirred at ~-116°C for 10 minutes and then flask was taken out of the cooling bath and left to warm to rt and stirred for further 30 min. The reaction mixture was quenched with water (10 mL), adjusted to pH ~ 5 with acetic acid, extracted with ethyl acetate (3×30 mL), the combined organic layers were washed with brine and dried over $Na_2SO_4$. The products were isolated by flash column chromatography (Büchi Reveleris HP silica 40 g; gradient 0% to 20% ethyl acetate - hexane.) In some cases additional purification was needed (Teledyne Isco RediSep Rf 40g, gradient 20% to 100% of

DCM-Hexane). Product yield 446 mg (61%) of off-white solid.
$^1$H NMR (400 MHz, cdcl$_3$) δ 7.69 (dd, J = 7.6, 1.0 Hz, 1H), 7.62 (t, J = 7.6 Hz, 1H), 7.31 (dd, J = 7.6, 1.0 Hz, 1H), 7.09 (d, J = 2.7 Hz, 2H), 6.87 (dd, J = 8.7, 0.4 Hz, 2H), 6.67 (dd, J = 8.7, 2.7 Hz, 2H), 1.65 (s, 9H), 0.96 (s, 18H), 0.60 (s, 3H), 0.56 (s, 3H), 0.17 (s, 12H).
$^{13}$C NMR (101 MHz, cdcl$_3$) δ 167.5, 165.6, 155.2, 155.2, 137.2, 136.9, 134.0, 133.5, 128.9, 128.6, 126.3, 125.0, 123.1, 121.2, 89.1, 83.4, 28.1, 25.7, 18.3, -1.3, -2.9, -4.3, -4.
ESI-MS, positive mode: m/z = 703.3 [M+H]$^+$.
HRMS (ESI) calcd for $C_{39}H_{55}O_6Si_3$ [M+H]$^+$ 703.3301, found 703.3304.

**A.5 Tert-butyl 3',6'-dihydroxy-3-oxo-3H-spiro[isobenzofuran-1,9'-xanthene]-4-carboxylate (8):**

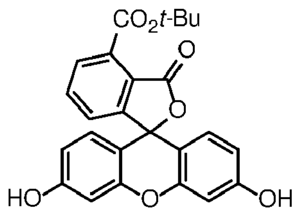

To a cooled (ice-water bath) solution of corresponding compound **5** (420 mg, 0.635 mmol, 1 eq.) in THF (15 mL) tetrabutylammonium fluoride trihydrate (800 mg, 2.54 mmol, 4 eq.) solution in THF (5 mL) was added. The resulting intensively coloured solution was stirred at 0 °C for 1 h. Sat. aq. $NH_4Cl$ (20 mL) was added followed by minimal amount of water necessary to dissolve the solids, the mixture was extracted with ethyl acetate (3×30 mL), the combined organic layers were washed with brine and dried over $Na_2SO_4$. The products were isolated by flash column chromatography (Teledyne Isco RediSep Rf 24 g; gradient 2% to 30% ethyl acetate - $CH_2Cl_2$) and evaporated to obtain viscous oil which solidifies overtime. Product yield 214 mg (78%) of orange solid material.
$^1$H NMR (400 MHz, d$_6$-dmso) δ 10.15 (s, 2H), 7.87 - 7.76 (m, 2H), 7.39 (dd, J = 7.3, 1.4 Hz, 1H), 6.69 (d, J = 2.0 Hz, 2H), 6.63 - 6.47 (m, 4H), 1.60 (s, 9H).
$^{13}$C NMR (101 MHz, d$_6$-dmso) δ 166.1, 164.9, 159.5, 153.4, 151.9, 135.6, 132.2, 129.2, 128.9, 126.3, 122.6, 112.7, 109.3, 102.3, 82.6, 82.2, 27.7.
ESI-MS, positive mode: m/z = 433.1 [M+H]$^+$.
HRMS (ESI) calcd for $C_{25}H_{21}O_7$ [M+H]$^+$ 433.1282, found 433.1279.

**A.6 Tert-butyl 3,6-dihydroxy-10,10-dimethyl-3'-oxo-3'H,10H-spiro[anthracene-9,1'-isobenzofuran]-4'-carboxylate (9):**

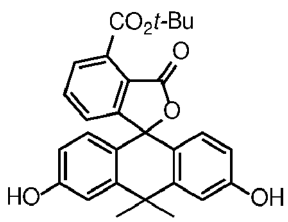

To a cooled (ice-water bath) solution of corresponding compound **6** (445 mg, 0.647 mmol, 1 eq.) in THF (15 mL) tetrabutylammonium fluoride trihydrate (817 mg, 2.59 mmol, 4 eq.) solution in THF (5 mL) was added. The resulting intensively coloured solution was stirred at 0 °C for 1 h. Sat. aq. $NH_4Cl$ (20 mL) was added followed by minimal amount of water necessary to dissolve the solids, the mixture was extracted with ethyl acetate (3×30 mL), the combined organic layers were washed with brine and dried over $Na_2SO_4$. The products were isolated by flash column chromatography (Teledyne Isco RediSep Rf 24 g; gradient 2% to 30% ethyl acetate - $CH_2Cl_2$) and evaporated to obtain viscous oil which solidifies overtime. Product yield 264 mg (89%) of orange solid material.
$^1$H NMR (300 MHz, cdcl$_3$) δ 7.73 (dd, J = 7.6, 1.0 Hz, 1H), 7.60 (t, J = 7.6 Hz, 1H), 7.14 (s, 2H), 7.05 (dd, J = 7.6, 1.0 Hz, 1H), 6.98 (d, J = 2.4 Hz, 2H), 6.51 (dd, J = 8.6, 2.4 Hz, 2H), 6.44 (d, J = 8.6 Hz, 2H), 1.68 (s, 9H).
$^{13}$C NMR (126 MHz, cdcl$_3$) δ 169.0, 165.9, 156.7, 156.2, 147.3, 134.7, 132.7, 129.2, 129.1, 126.1, 123.2, 122.4, 114.8,

112.9, 87.0, 84.1, 38.1, 34.8, 32.3, 28.1.
ESI-MS, positive mode: m/z = 459.2 $[M+H]^+$.
HRMS (ESI) calcd for $C_{28}H_{27}O_6$ $[M+H]^+$ 459.1802, found 459.1805.

**A.7 Tert-butyl 3,7-dihydroxy-5,5-dimethyl-3'-oxo-3'H,5H-spiro[dibenzo[b,e]siline-10,1'-isobenzofuran]-4'-carboxylate(10):**

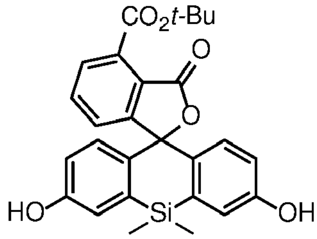

To a cooled (ice-water bath) solution of corresponding compound **7** (420 mg, 0.575 mmol, 1 eq.) in THF (15 mL) tetrabutylammonium fluoride trihydrate (725 mg, 2.3 mmol, 4 eq.) solution in THF (5 mL) was added. The resulting intensively coloured solution was stirred at 0 °C for 1 h. Sat. aq. $NH_4Cl$ (20 mL) was added followed by minimal amount of water necessary to dissolve the solids, the mixture was extracted with ethyl acetate (3×30 mL), the combined organic layers were washed with brine and dried over $Na_2SO_4$. The products were isolated by flash column chromatography (Teledyne Isco RediSep Rf 24 g; gradient 2% to 30% ethyl acetate - $CH_2Cl_2$) and evaporated to obtain viscous oil which solidifies overtime. Product yield 254 mg (93%) of orange solid material.
$^1H$ NMR (400 MHz, $d_6$-dmso) $\delta$ 9.74 (s, 2H), 7.83 (t, J = 7.6 Hz, 1H), 7.72 (dd, J = 7.6, 0.9 Hz, 1H), 7.46 (dd, J = 7.6, 0.9 Hz, 1H), 7.16 - 7.08 (m, 2H), 6.74 - 6.60 (m, 4H), 1.55 (s, 9H), 0.56 (s, 3H), 0.48 (s, 3H).
$^{13}C$ NMR (101 MHz, $d_6$-dmso) $\delta$ 167.4, 165.4, 157.3, 154.6, 137.5, 135.2, 134.5, 133.5, 129.0, 128.6, 127.1, 122.3, 120.7, 117.3, 90.0, 83.0, 28.0, 0.4, -1.4.
ESI-MS, positive mode: m/z = 475.2 $[M+H]^+$.
HRMS (ESI) calcd for $C_{27}H_{27}O_6Si$ $[M+H]^+$ 475.1571, found 475.1573.

**A.8 Tert-butyl 3-oxo-3',6'-bis(((trifluoromethyl)sulfonyl)oxy)-3H-spiro[isobenzofuran-1,9'-xanthene]-4-carboxylate(11):**

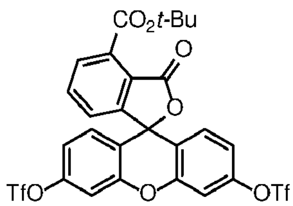

Trifluoromethanesulfonic anhydride 1M solution in DCM (1.85 mL, 1.85 mmol, 4 eq.) was slowly added dropwise to a solution of corresponding compound **8** (200 mg, 0.463 mmol, 1 eq.) and pyridine (0.3 mL, 3.7 mmol; 8 eq.) in dry DCM (10 mL), cooled in ice-water bath. The flask was then removed from the cooling bath, and the mixture was stirred at rt for 1 h. Afterwards, the mixture was diluted with water (30 mL), extracted with $CH_2Cl_2$ (3×20 mL), the combined extracts were washed with water, brine and dried over $Na_2SO_4$. The products were isolated by flash column chromatography (Teledyne Isco RediSep Rf 24 g; gradient 5% to 40% ethyl acetate - hexane).
Product yield 255 mg (79%) of white solid material.
$^1H$ NMR (400 MHz, $cdcl_3$) $\delta$ 7.87 (d, J = 7.4 Hz, 1H), 7.73 (t, J = 7.4 Hz, 1H), 7.30 (d, J = 2.2 Hz, 2H), 7.22 (d, J = 7.4 Hz, 1H), 7.08 - 6.99 (m, 4H), 1.70 (s, 9H).
$^{13}C$ NMR (101 MHz, $cdcl_3$) $\delta$ 165.4, 164.6, 153.3, 151.2, 150.2, 135.4, 133.7, 130.7, 130.0, 125.7, 122.4, 119.1, 118.6 (q, $^1J_{C-F}$ = 319 Hz, $-CF_3$), 117.7, 110.7, 83.9, 78.8, 28.0.
ESI-MS, positive mode: m/z = 697.0 $[M+H]^+$.
HRMS (ESI) calcd for $C_{27}H_{19}F_6O_{11}S_2$ $[M+H]^+$ 697.0267, found 697.0249.

**A.9 Tert-butyl 10,10-dimethyl-3'-oxo-3,6-bis(((trifluoromethyl)sulfonyl)oxy)-3'H,10H-spiro[anthracene-9,1'-isobenzofuran]-4'-carboxylate(12):**

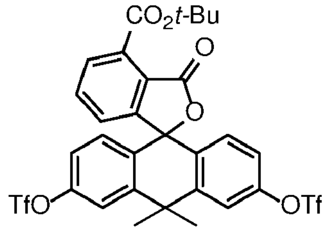

Trifluoromethanesulfonic anhydride 1M solution in DCM (2.14 mL, 2.14 mmol, 4 eq.) was slowly added dropwise to a solution of corresponding compound **9** (245 mg, 0.535 mmol, 1 eq.) and pyridine (0.35 mL, 4.3 mmol; 8 eq.) in dry DCM (10 mL), cooled in ice-water bath. The flask was then removed from the cooling bath, and the mixture was stirred at rt for 1 h. Afterwards, the mixture was diluted with water (30 mL), extracted with $CH_2Cl_2$ (3×20 mL), the combined extracts were washed with water, brine and dried over $Na_2SO_4$. The products were isolated by flash column chromatography (Teledyne Isco RediSep Rf 24 g; gradient 5% to 40% ethyl acetate - hexane). Product yield 271 mg (70%) of white solid material.
$^1$H NMR (400 MHz, $cdcl_3$) $\delta$ 7.83 (dd, J = 7.6, 0.9 Hz, 1H), 7.68 (t, J = 7.6 Hz, 1H), 7.54 (d, J = 2.5 Hz, 2H), 7.11 (dd, J = 8.8, 2.5 Hz, 2H), 7.07 (dd, J = 7.6, 0.9 Hz, 1H), 6.94 (d, J = 8.8 Hz, 2H), 1.89 (s, 3H), 1.79 (s, 3H), 1.70 (s, 9H).
$^{13}$C NMR (101 MHz, $cdcl_3$) $\delta$ 166.4, 164.8, 154.8, 150.1, 146.9, 135.1, 133.8, 131.3, 130.3, 130.2, 125.6, 122.6, 120.3, 119.6, 118.9 (q, 1JC-F = 319 Hz,-CF3), 83.8, 82.6, 38.8, 34.7, 33.0, 28.0. ESI-MS, positive mode: m/z = 745.1 $[M+Na]^+$.
HRMS (ESI) calcd for $C_{30}H_{25}O_{10}S_2F_6$ $[M+H]^+$ 723.0788, found 723.0800.

**A.10 Tert-butyl 5,5-dimethyl-3'-oxo-3,7-bis(((trifluoromethyl)sulfonyl)oxy)-3'H,5H-spiro[dibenzo[b,e]siline-10,1'-isobenzofuran]-4'-carboxylate (13):**

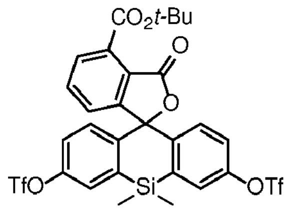

Trifluoromethanesulfonic anhydride 1M solution in DCM (1.94 mL, 1.94 mmol, 4 eq.) was slowly added dropwise to a solution of corresponding compound **10** (230 mg, 0.484 mmol, 1 eq.) and pyridine (0.31 mL, 3.87 mmol; 8 eq.) in dry DCM (10 mL), cooled in ice-water bath. The flask was then removed from the cooling bath, and the mixture was stirred at rt for 1 h. Afterwards, the mixture was diluted with water (30 mL), extracted with $CH_2Cl_2$ (3×20 mL), the combined extracts were washed with water, brine and dried over $Na_2SO_4$. The products were isolated by flash column chromatography (Teledyne Isco RediSep Rf 24 g; gradient 5% to 40% ethyl acetate - hexane). Product yield 271 mg (70%) of white solid material. Product yield 258 mg (72%) of white solid material.
$^1$H NMR (400 MHz, $cdcl_3$) $\delta$ 7.81 (dd, J = 7.6, 1.1 Hz, 1H), 7.75 (t, J = 7.6 Hz, 1H), 7.56 (dd, J = 2.3, 0.8 Hz, 2H), 7.39 (dd, J = 7.6, 1.1 Hz, 1H), 7.24 - 7.17 (m, 4H), 1.67 (s, 9H), 0.74 (s, 3H), 0.70 (s, 3H). $^{13}$C NMR (101 MHz, $cdcl_3$) $\delta$ 166.2, 164.8, 152.8, 149.3, 144.0, 138.7, 134.8, 134.3, 130.0, 129.2, 126.3, 126.1, 122.8, 122.6, 118.7 (q, 1JC-F = 319 Hz,-CF3), 87.3, 83.8, 28.0,-0.2, -1.7.
ESI-MS, positive mode: m/z = 761.0 $[M+Na]^+$.
HRMS (ESI) calcd for $C_{29}H_{25}O_{10}S_2SiF_6$ [M+H]+ 739.0557, found 739.0562.

**A.11 Tert-butyl 3',6'-bis(dimethylamino)-3-oxo-3H-spiro[isobenzofuran-1,9'-xanthene]-4-carboxylate (14):**

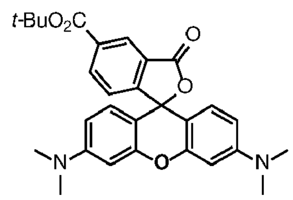

A mixture of $Pd_2(dba)_3$ (25.6 mg, 0.026 mmol, 0.1 eq.), Xantphos (44.8 mg, 0.077 mmol, 0.3 eq.), $Cs_2CO_3$ (251 mg, 0.77, 3 eq.) and corresponding triflate **11** (180 mg, 0.258 mmol, 1 eq) in dry 1,4-dioxane (2.5 mL) was degassed on a Schlenk line. Then 2M solution of dimethylamine in THF (323 $\mu$L, 0.645 mmol, 2.5 eq.) were introduced. Reaction mixture was stirred in a septa sealed tube at 100°C under argon for 18h. Upon cooling, the resulting brown mixture was diluted with water (30 mL), pH adjusted to 5-6 with AcOH and extracted with ethyl acetate (3×30 mL), the combined organic layers were washed with brine and dried over $Na_2SO_4$. The filtrate was evaporated and the product was isolated by flash chromatography Büchi Reveleris HP silica 24 g; gradient 2% to 30% MeOH - DCM. The fractions containing the product were evaporated, the residue was redissolved in acetonitrile - water (1:1), microfiltered through a 0.45 $\mu$m PTFE membrane filter and lyophilized to obtain 67 mg (53%) of pink solid.
$^1$H NMR (400 MHz, $cd_3od$) $\delta$ 8.00 (dd, J = 7.8, 1.3 Hz, 1H), 7.57 (t, J = 7.8 Hz, 1H), 7.39 (dd, J = 7.8, 1.3 Hz, 1H), 7.31 (d, J = 9.5 Hz, 2H), 7.00 (dd, J = 9.5, 2.5 Hz, 2H), 6.85 (d, J = 2.5 Hz, 2H), 3.26 (s, 12H), 1.61 (s, 9H).
$^{13}$C NMR (101 MHz, $cd_3od$) $\delta$ 173.7, 167.8, 159.1, 158.9, 158.8, 144.0, 133.6, 133.3, 131.9, 131.8, 131.6, 128.1, 115.3, 114.9, 97.3, 83.3, 40.9, 28.3.
ESI-MS, positive mode: m/z = 487.2 $[M+H]^+$.
HRMS (ESI) calcd for $C_{29}H_{31}N_2O_5$ $[M+H]^+$ 487.2227, found 487.2232

**A.12 Tert-butyl 10,10-dimethyl-3,6-bis(methylamino)-3'-oxo-3'H,10H-spiro[anthracene-9,1'-isobenzofuran]-4'-carboxylate (15):**

A mixture of $Pd_2(dba)_3$ (30.2mg, 0.033 mmol, 0.1 eq.), Xantphos (57.3 mg, 0.099 mmol, 0.3 eq.),

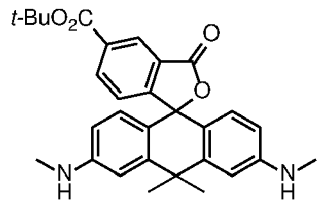

$Cs_2CO_3$ (322.5 mg, 0.99 mmol, 3 eq.) and corresponding triflate **12** (240 mg, 0.33 mmol, 1 eq) in dry 1,4-dioxane (2.5 mL) was degassed on a Schlenk line. Then solution of tert-butyl N-methylcarbamate (108 mg, 0.825 mmol, 2.5 eq.) in 1,4-dioxane (0.5 mL) were introduced. Reaction mixture was stirred in a septa sealed tube at 100°C under argon for 18h. Upon cooling, the resulting brown mixture was diluted with water (30 mL), pH adjusted to 5-6 with AcOH extracted with ethyl acetate (3×30 mL), the combined organic layers were washed with brine and dried over $Na_2SO_4$. Product was isolated by flash column chromatography Büchi Reveleris HP silica 24 g; gradient 5% to 50% EtOAc - Hexane. The fractions containing the product were evaporated, the residue was redissolved in acetonitrile - water (1:1), microfiltered through a 0.45 $\mu$m PTFE membrane filter and lyophilized to obtain 173 mg (76%) of violet powder.
$^1$H NMR (400 MHz, $cdcl_3$) $\delta$ 7.75 (dd, J = 7.6, 0.9 Hz, 1H), 7.59 (t, J = 7.6 Hz, 1H), 7.52 (d, J = 2.2 Hz, 2H), 7.06 (dd, J = 7.6, 0.9 Hz, 1H), 7.01 (dd, J = 8.6, 2.2 Hz, 2H), 6.76 (d, J = 8.6 Hz, 2H), 3.27 (s, 6H), 1.85 (s, 3H), 1.76 (s, 3H), 1.70 (s, 9H), 1.46 (s, 18H).
$^{13}$C NMR (101 MHz, $cdcl_3$) $\delta$ 167.4, 165.5, 156.2, 154.6, 145.3, 144.7, 134.5, 133.4, 129.5, 128.4, 127.9, 126.0, 123.6, 123.6, 123.2, 84.4, 83.6, 80.8, 38.3, 37.3, 35.0, 33.3, 28.5, 28.2.
ESI-MS, positive mode: m/z = 685.4 $[M+H]^+$.
HRMS (ESI) calcd for $C_{40}H_{49}N_2O_8$ $[M+H]^+$ 685.3483, found 685.3474.

**A.13 Tert-butyl 3,6-bis(dimethy)amino)-10,10-dimethy)-3'-oxo-3'H,10H-spiro[anthracene-9,l'-isobenzofuran]-4'-carboxylate (16):**

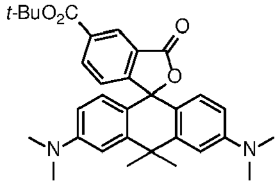

A mixture of $Pd_2(dba)_3$ (33 mg, 0.036 mmol, 0.1 eq.), Xantphos (62.5 mg, 0.108 mmol, 0.3 eq.), $Cs_2CO_3$ (352 mg, 1.08 mmol, 3 eq.) and corresponding triflate **12** (250 mg, 0.36 mmol, 1 eq) in dry 1,4-dioxane (2.5 mL) was degassed on a Schlenk line. Then 2M solution of dimethylamine in THF (450 μL, 0.9 mmol, 2.5 eq.) were introduced. Reaction mixture was stirred in a septa sealed tube at 100°C under argon for 18h. Upon cooling, the resulting brown mixture was diluted with water (30 mL), pH adjusted to 5-6 with AcOH and extracted with ethyl acetate (3×30 mL), the combined organic layers were washed with brine and dried over $Na_2SO_4$. Product was isolated by flash column chromatography Büchi Reveleris HP silica 24 g; gradient 20% to 80% EtOAc- Hexane. The fractions containing the product were evaporated, the residue was redissolved in 1,4-dioxane - water (1:1), microfiltered through a 0.45 μm PTFE membrane filter and lyophilized to obtain 131 mg (71%) of violet powder.
$^1$H NMR (400 MHz, cdcl$_3$) δ 7.69 (dd, J = 7.6, 1.0 Hz, 1H), 7.55 (t, J = 7.6 Hz, 1H), 7.09 (dd, J = 7.6, 1.0 Hz, 1H), 6.88 (d, J = 2.6 Hz, 2H), 6.65 (d, J = 8.8 Hz, 2H), 6.53 (dd, J = 8.8, 2.6 Hz, 2H), 2.99 (s, 12H), 1.87 (s, 3H), 1.78 (s, 3H), 1.70 (s, 9H).
$^{13}$C NMR (101 MHz, cdcl$_3$) δ 167.9, 166.0, 157.0, 150.8, 146.7, 134.0, 133.0, 129.1, 128.8, 126.1, 124.0, 119.5, 111.8, 109.2, 86.5, 83.3, 40.6, 38.6, 35.6, 33.1, 28.2.
ESI-MS, positive mode: m/z = 513.6 $[M+H]^+$.
HRMS (ESI) calcd for $C_{32}H_{37}N_2O_4$ $[M+H]^+$ 513.2748, found 513.2748.

**A.14 Tert-butyl 3,7-bis(dimethy)amino)-5,5-dimethy)-3'-oxo-3'H,5H-spiro[dibenzo[b,e]siline-10,1'-isobenzofuran]-4'-carboxylate (17):**

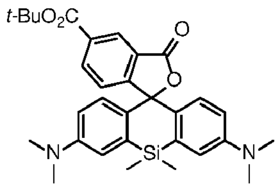

A mixture of $Pd_2(dba)_3$ (26 mg, 0.0284 mmol, 0.1 eq.), Xantphos (49.3 mg, 0.0852 mmol, 0.3 eq.), $Cs_2CO_3$ (278 mg, 0.85 mmol, 3 eq.) and corresponding triflate **13** (210 mg, 0.284 mmol, 1 eq) in dry 1,4-dioxane (2.5 mL) was degassed on a Schlenk line. Then 2M solution of dimethylamine in THF (355 μL, 0.71 mmol, 2.5 eq.) were introduced. Reaction mixture was stirred in a septa sealed tube at 100°C under argon for 18h. Upon cooling, the resulting brown mixture was diluted with water (30 mL), pH adjusted to 5-6 with AcOH and extracted with ethyl acetate (3×30 mL), the combined organic layers were washed with brine and dried over $Na_2SO_4$.

Product was isolated by flash column chromatography Büchi Reveleris HP silica 24 g; gradient 5% to 50% EtOAc - Hexane. The fractions containing the product were evaporated, the residue was redissolved in 1,4-dioxane - water (1:1), microfiltered through a 0.45 μm PTFE membrane filter and lyophilized to obtain 73 mg (48%) of light-blue powder.
$^1$H NMR (400 MHz, cdcl$_3$) δ 7.69 (dd, J = 7.6, 1.0 Hz, 1H), 7.61 (t, J = 7.6 Hz, 1H), 7.32 (dd, J = 7.6, 1.0 Hz, 1H), 6.96 (d, J = 2.9 Hz, 2H), 6.82 (d, J = 8.9 Hz, 2H), 6.57 (dd, J = 8.9, 2.9 Hz, 2H), 2.96 (s, 12H), 1.67 (s, 9H), 0.64 (s, 3H), 0.60 (s, 3H).
$^{13}$C NMR (101 MHz, cdcl$_3$) δ 167.8, 166.0, 155.8, 149.4, 136.7, 133.8, 133.3, 131.8, 128.7, 128.3, 126.5, 123.8, 116.6, 113.6, 90.3, 83.3, 40.4, 28.1, 0.4, -1.1.
ESI-MS, positive mode: m/z = 529.3 $[M+H]^+$.
HRMS (ESI) calcd for $C_{31}H_{37}N_2O_4Si$ $[M+H]^+$ 529.2517, found 529.2521.

**A.15 4-TMR-COOH (18):**

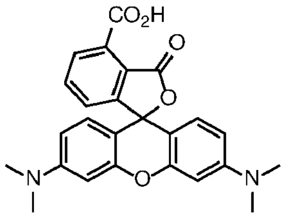

Trifluoroacetic acid (2 mL) was added dropwise to a solution of compound **14** (67 mg, 0.138 mmol) in DCM (8 mL). The resulting coloured solution was stirred at room temperature for 2h. The reaction mixture was then evaporated to dryness, the residue was reevaporated three times with toluene to remove excess of trifluoroacetic acid. The residue was lyophilized from 1,4-dioxane - water (1:1). Obtained 74 mg (98%) of pink powder as trifluoroacetic acid salt.
$^1$H NMR (400 MHz, $cd_3od$) δ 8.25 (dd, J = 7.8, 1.2 Hz, 1H), 7.82 (t, J = 7.8 Hz, 1H), 7.60 (dd, J = 7.8, 1.2 Hz, 1H), 7.21 (d, J = 9.5 Hz, 2H), 7.08 (dd, J = 9.5, 2.4 Hz, 2H), 6.87 (d, J = 2.4 Hz, 2H), 3.29 (s, 12H).
$^{13}$C NMR (101 MHz, $cd_3od$) δ 170.5, 168.6, 159.0, 159.0, 157.0, 136.8, 134.1, 132.8, 132.5, 132.2, 132.0, 131.0, 115.5, 115.2, 97.4, 41.0.
ESI-MS, positive mode: m/z = 431.2 $[M+H]^+$.
HRMS (ESI) calcd for $C_{25}H_{23}N_2O_5$ $[M+H]^+$ 431.1601, found 431.1606.

**A.16 4-580CP-COOH (19):**

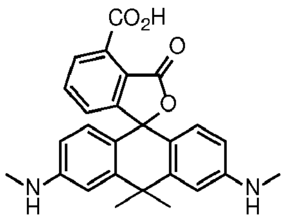

Trifluoroacetic acid (2 mL) was added dropwise to a solution of compound **15** (220 mg, 0.321mmol) in DCM (8 mL). The resulting coloured solution was stirred at room temperature for 3h. The reaction mixture was then evaporated to dryness, the residue was reevaporated three times with toluene to remove excess of trifluoroacetic acid. The residue was lyophilized from 1,4-dioxane - water (1:1). Obtained 170 mg (98%) of violet powder as trifluoroacetic acid salt.
$^1$H NMR (400 MHz, $cd_3od$) δ 8.17 (dd, J = 7.7, 1.1 Hz, 1H), 7.76 (t, J = 7.7 Hz, 1H), 7.52 (dd, J = 7.7, 1.1 Hz, 1H), 7.11 (d, J = 2.2 Hz, 2H), 7.03 (d, J = 9.2 Hz, 2H), 6.64 (dd, J = 9.2, 2.2 Hz, 2H), 3.06 (s, 6H), 1.81 (s, 3H), 1.68 (s, 3H).
$^{13}$C NMR (101 MHz, $cd_3od$) δ 170.6, 168.9, 162.5, 159.4, 139.6, 139.5, 136.6, 136.0, 134.2, 131.72, 131.68, 130.57, 122.1, 112.7 (visible in HSQC spectra), 42.7, 35.8, 31.9, 30.1.
ESI-MS, positive mode: m/z = 429.2 $[M+H]^+$.
HRMS (ESI) calcd for $C_{26}H_{25}N_2O_4$ $[M+H]^+$ 429.1809, found 429.1807.

**A.17 4-610CP-COOH (20):**

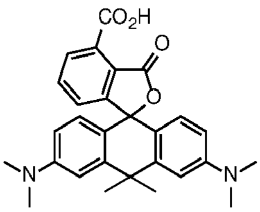

Trifluoroacetic acid (2 mL) was added dropwise to a solution of compound **16** (230 mg, 0.448 mmol) in DCM (8 mL). The resulting coloured solution was stirred at room temperature for 1h. The reaction mixture was then evaporated to dryness, the residue was re-evaporated three times with toluene to remove excess of trifluoroacetic acid. The residue was lyophilized from 1,4-dioxane - water (1:1). Obtained 251 mg (98%) of dark-violet powder as trifluoroacetic acid salt.

$^{1}H$ NMR (400 MHz, $cd_3od$) δ 8.17 (dd, J = 7.8, 1.2 Hz, 1H), 7.77 (t, J = 7.8 Hz, 1H), 7.53 (dd, J = 7.8, 1.2 Hz, 1H), 7.23 (d, J = 2.5 Hz, 2H), 7.09 (d, J = 9.4 Hz, 2H), 6.84 (dd, J = 9.4, 2.5 Hz, 2H), 3.34 (s, 12H), 1.86 (s, 3H), 1.72 (s, 3H).
$^{13}C$ NMR (101 MHz, $cd_3od$) δ 170.6, 168.9, 158.3, 157.9, 138.9, 136.6, 135.9, 134.2, 131.8, 131.7, 130.6, 122.0, 114.0, 112.1, 43.2, 41.0, 36.2, 32.3.
ESI-MS, positive mode: m/z = 457.2 $[M+H]^+$.
HRMS (ESI) calcd for $C_{28}H_{29}N_2O_4$ $[M+H]^+$ 457.2122, found 457.2119.

**A.18 4-SiR-COOH (21):**

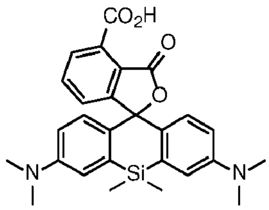

Trifluoroacetic acid (2 mL) was added dropwise to a solution of compound **17** (50 mg, 0.095 mmol) in DCM (8 mL). The resulting coloured solution was stirred at room temperature for 1h. The reaction mixture was then evaporated to dryness, the residue was re-evaporated three times with toluene to remove excess of trifluoroacetic acid. The residue was lyophilized from 1,4-dioxane - water (1:1). Obtained 54 mg (98%) of blue powder as trifluoroacetic acid salt.
$^{1}H$ NMR (400 MHz,$d_5$-pyridine) δ 8.17 (dd, J = 7.7, 0.9 Hz, 1H), 7.77 (t, J = 7.7 Hz, 1H), 7.54 (dd, J = 7.7, 0.9 Hz, 1H), 7.18 (d, J = 2.9 Hz, 2H), 7.04 (d, J = 9.0 Hz, 2H), 6.54 (dd, J = 9.0,2.9 Hz, 2H), 2.85 (s, 12H), 0.73 (s, 3H), 0.65 (s, 3H).
$^{13}C$ NMR (101 MHz, ds-pyridine) δ 170.1, 169.4, 150.4 (overlapped with pyridine, visible in HMBC spectra), 157.1, 137.4, 135.6, 134.9, 132.3, 129.7, 129.2, 127.0, 124.2 (overlapped with pyridine, visible in HMBC spectra), 117.4, 114.6, 92.3, 40.4, 0.8, -0.8.
ESI-MS, positive mode: m/z = 473.2 $[M+H]^+$.
HRMS (ESI) calcd for $C_{27}H_{29}N_2O_4Si$ $[M+H]^+$ 473.1891, found 473.1890.

SYNTHESIS EXAMPLE B

*Synthesis of rhodamine 4'-isomer fluorescent probes*

**B.1 DTX-C8-NHBOC (22)**

A solution of docetaxel (194 mg, 0.24 mmol) in 95% formic acid (2 mL) was stirred at room

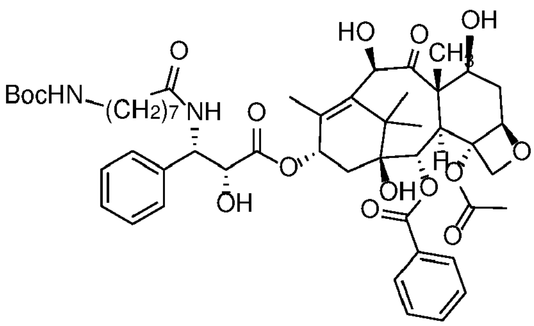

temperature for 4h. Reaction progress was monitored by HPLC analysis. Once reaction was complete, formic acid was evaporated on rotary evaporator and residue was dissolved in water and lyophilised to obtain white powder.

Into a solution of Boc-8-aminooctanoic acid (1.4 eq., 0.336 mmol, 91.7 mg) in MeCN (2 mL) was added HBTU (1.2 eq., 0.288 mmol, 109 mg), followed by DIPEA (4.3 eq., 1 mmol, 100 μL). Mixture was stirred at rt for 5 min and previously obtained des-Boc-docetaxel (0.24 mmol) was added. Mixture was stirred for 1 hour then solvent was removed by rotary evaporator. The residue was re-dissolved in 70% MeCN-H2O mixture, microfiltered through a 0.45 μm PTFE membrane filter and purified by the preperative HPLC (preparative column: Knauer 100 C18, 5 μm, 250 × 30 mm;

solvent A: acetonitrile, solvent B: H2O + 0.2% v/v HCOOH; temperature 25 °C, gradient A:B - 5 min 50:50 isocratic, 5-30 min 50:50 to 100:0 gradient). Fractions containing the product were collected, solvent was removed and obtained residue was lyophilised from 50:50 MeCN : H2O mixture to obtain product as white solid. Obtained 52% 118mg of white fluffy solid. $^1H$ NMR (400 MHz,$d_6$-dmso) δ 8.36 (d, J = 9.0 Hz, 1H), 7.99 (dt, J = 7.0, 1.4 Hz, 2H), 7.69 (tt, J = 7.4, 1.4 Hz, 1H), 7.60 (t, J = 7.5 Hz, 2H), 7.39 - 7.29 (m, 4H), 7.21 (tt, J = 7.2, 1.5 Hz, 1H), 6.73 (t, J = 5.7 Hz, 1H), 5.95 - 5.88 (m, 2H), 5.42 (d, J = 7.2 Hz, 1H), 5.27 (dd, J = 9.0, 5.9 Hz, 1H), 5.10 (s, 1H), 5.01 (d, J = 7.2 Hz, 1H), 4.97 - 4.88 (m, 2H), 4.58 (s, 1H), 4.41 (t, J = 6.3 Hz, 1H), 4.09 - 3.99 (m, 3H), 3.69 (d, J = 7.2 Hz, 1H), 2.87 (q, J = 6.7 Hz, 2H), 2.28 (s, 1H), 2.24 (s, 3H), 2.15 (t, J = 7.3 Hz, 2H), 1.98 (dd, J = 15.3, 9.2 Hz, 1H), 1.82 (dd, J = 15.4, 8.9 Hz, 1H), 1.75 (s, 3H), 1.73 - 1.59 (m, 1H), 1.53 (s, 3H), 1.49 - 1.43 (m, 2H), 1.36 (s, 9H), 1.34 - 1.26 (m, 2H), 1.18 (q, J = 9.0, 7.1 Hz, 6H), 1.03 (s, 3H), 0.99 (s, 3H).

$^{13}C$ NMR (101 MHz, $d_6$-dmso) δ 209.3, 172.6, 171.9, 169.7, 165.3, 155.5, 139.7, 136.8, 135.9, 133.3, 130.0, 129.6, 128.7, 128.1, 127.14, 127.10, 83.7, 80.3, 77.3, 76.9, 75.5, 74.8, 73.8, 73.6, 70.8, 70.0, 57.0, 55.0, 46.0, 42.9, 39.8, 36.5, 35.4, 35.1, 29.5, 28.6, 28.5, 28.3, 26.5, 26.2, 25.4, 22.4, 20.8, 13.7, 9.8.

ESI-MS, positive mode: m/z = 949.5 $[M+H]^+$.

HRMS (ESI) calcd for $C_{51}H_{69}N_2O_{15}$ $[M+H]^+$ 949.4692, found 949.4683.

**B.2 CTX-C8-NHBOC (23)**

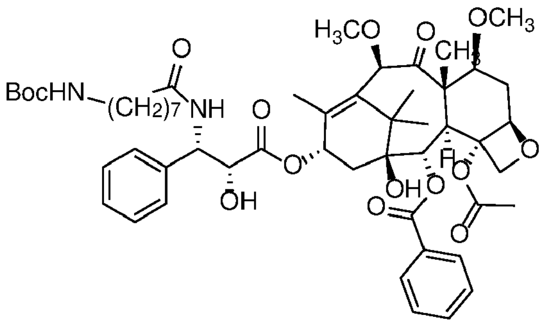

A solution of cabazitaxel (200 mg, 0.24 mmol) in 95% formic acid (2 mL) was stirred at room temperature for 2h. Reaction progress was monitored by HPLC analysis. Once reaction was complete, formic acid was evaporated on rotary evaporator and residue was dissolved in water and lyophilised to obtain white powder.

Into a solution of Boc-8-aminooctanoic acid (91.7 mg, 0.336 mmol,1.4 eq.,) in MeCN (2 mL) was added HBTU (109 mg, 0.288 mmol, 1.2 eq.), followed by DIPEA (100 μL, 1 mmol, 4.3 eq.). Mixture was stirred at rt for 5 min and previously obtained des-Boc-docetaxel (0.24 mmol) was added. Mixture was stirred for 1 hour then solvent was removed by rotary evaporator. The residue was re-dissolved in 70% MeCN-H2O mixture, microfiltered through a 0.45 μm PTFE membrane filter and purified by the preperative HPLC (preparative column: Knauer 100 C18, 5 μm, 250 × 30 mm; solvent A: acetonitrile, solvent B: H2O + 0.2% v/v HCOOH; temperature 25 °C, gradient A:B - 5 min 50:50 isocratic, 5-30 min 50:50 to 100:0 gradient). Fractions containing the product were collected, solvent was removed and obtained residue was lyophilised from 50:50 MeCN : H2O mixture to obtain product as white solid. Obtained 69% (162 mg) of white fluffy solid. $^1H$ NMR (400 MHz, $d_6$-dmso) δ 8.37 (d, J = 9.1 Hz, 1H), 8.00 (dt, J = 7.1, 1.4 Hz, 2H), 7.70 (tt, J = 7.4, 2.2 Hz, 1H), 7.61 (t, J = 7.4 Hz, 2H), 7.40 - 7.30 (m, 4H), 7.23 (tt, J = 7.4, 1.4 Hz, 1H), 6.74 (t, J = 5.7 Hz, 1H), 5.99 - 5.91 (m, 2H), 5.40 (d, J = 7.1 Hz, 1H), 5.30 (dd, J = 9.2, 5.8 Hz, 1H), 4.97 (dd, J = 9.7, 2.0 Hz, 1H), 4.72 (s, 1H), 4.66 (s, 1H), 4.44 (dd, J = 6.8, 5.8 Hz, 1H), 4.04 (s, 2H), 3.77 (dd, J = 10.6, 6.5 Hz, 1H), 3.65 (d, J = 7.1 Hz, 1H), 3.32 (s, 3H), 3.23 (s, 3H), 2.87 (q, J = 6.6 Hz, 2H), 2.72 - 2.63 (m, 1H), 2.26 (s, 3H), 2.17 (t, J = 7.2 Hz, 2H), 1.99 (dd, J = 15.4, 9.1 Hz, 1H), 1.92 - 1.86 (m, 1H), 1.85 (s, 3H), 1.53 (s, 3H), 1.52 - 1.43 (m, 3H), 1.37 (s, 9H), 1.35 - 1.30 (m, 2H), 1.24 - 1.16 (m, 6H), 1.04 (s, 3H), 0.99 (s, 3H).

$^{13}C$ NMR (101 MHz, $d_6$-dmso) δ 204.7, 172.6, 171.9, 169.9, 165.2, 155.5, 139.6, 138.4, 134.9, 133.3, 129.9, 129.6, 128.7, 128.1, 127.1, 83.2, 82.1, 80.3, 80.2, 77.3, 76.9, 75.3, 74.4, 73.6, 70.0, 56.6, 56.6, 56.0, 54.9, 46.4, 43.0, 39.8, 35.4, 34.9, 31.7, 29.5, 28.6, 28.5, 28.3, 26.7, 26.2, 25.4, 22.4, 21.2, 14.0, 10.2.

ESI-MS, positive mode: m/z = 977.5 $[M+H]^+$.

HRMS (ESI) calcd for $C_{53}H_{73}N_2O_{15}$ $[M+H]^+$ 977.5005, found 977.4995.

**B.3 LTX-C8-NHBOC (24)**

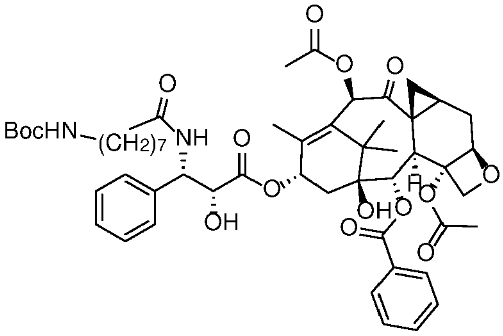

A solution of larotaxel (200 mg, 0.24 mmol) in 95% formic acid (2 mL) was stirred at room temperature for 1h. Reaction progress was monitored by HPLC analysis. Once reaction was complete, formic acid was evaporated on rotary evaporator and residue was dissolved in water and lyophilised to obtain white powder.

Into a solution of Boc-8-aminooctanoic acid (91.7 mg, 0.336 mmol,1.4 eq.,) in MeCN (2 mL) was added HBTU (109 mg, 0.288 mmol, 1.2 eq.), followed by DIPEA (100 μL, 1 mmol, 4.3 eq.). Mixture was stirred at rt for 5 min and previously obtained des-Boc-docetaxel (0.24 mmol) was added. Mixture was stirred for 1 hour then solvent was removed by rotary evaporator. The residue was re-dissolved in 70% MeCN-H2O mixture, microfiltered through a 0.45 μm PTFE membrane filter and purified by the preperative HPLC (preparative column: Knauer 100 C18, 5 μm, 250 × 30 mm; solvent A: acetonitrile, solvent B: H2O + 0.2% v/v HCOOH; temperature 25 °C, gradient A:B - 5 min 50:50 isocratic, 5-30 min 50:50 to 100:0 gradient). Fractions containing the product were collected, solvent was removed and obtained residue was lyophilised from 50:50 MeCN : H2O mixture to obtain product as white solid.

Obtained 57% (133 mg) of white fluffy solid.

$^1H$ NMR (400 MHz, $d_6$-dmso) δ 8.34 (d, J = 9.2 Hz, 1H), 8.08 - 8.00 (m, 2H), 7.69 (tt, J = 7.4, 1.4 Hz, 1H), 7.61 (t, J = 7.5 Hz, 2H), 7.41 - 7.29 (m, 4H), 7.19 (tt, J = 7.4, 1.7 Hz, 1H), 6.73 (t, J = 5.7 Hz, 1H), 6.12 (s, 1H), 6.02 - 5.91 (m, 1H), 5.87 (d, J = 6.8 Hz, 1H), 5.44 (d, J = 7.7 Hz, 1H), 5.34 (dd, J = 9.2, 5.2 Hz, 1H), 4.77 (s, 1H), 4.72 (d, J = 3.0 Hz, 1H), 4.49 (dd, J = 6.8, 5.2 Hz, 1H), 4.10 - 3.94 (m, 2H), 3.91 (d, J = 7.6 Hz, 1H), 2.86 (q, J = 6.5 Hz, 2H), 2.36 - 2.23 (m, 4H), 2.17 - 1.87 (m, 9H), 1.74 (d, J = 1.4 Hz, 3H), 1.54 (dd, J = 7.3, 4.8 Hz, 1H), 1.50 - 1.41 (m, 2H), 1.36 (s, 9H), 1.35 - 1.28 (m, 2H), 1.19 - 1.14 (m, 6H), 1.12 (s, 3H), 1.08 (s, 3H).

$^{13}C$ NMR (101 MHz, $d_6$-dmso) δ 202.0, 172.8, 172.4, 170.2, 169.5, 165.8, 156.0, 140.5, 140.3, 134.0, 133.7, 130.4, 130.1, 129.2, 128.6, 127.5, 127.5, 84.2, 80.0, 79.0, 77.8, 77.7, 75.7, 74.9, 74.0, 70.0, 55.1, 43.0, 40.3, 38.0, 36.1, 35.8, 34.9, 31.7, 29.9, 29.1, 28.9, 28.7, 26.7, 26.4, 26.0, 25.8, 22.4, 21.8, 21.0, 15.2, 14.4.

ESI-MS, positive mode: m/z = 973.6 $[M+H]^+$.

HRMS (ESI) calcd for $C_{53}H_{69}N_2O_{15}$ $[M+H]^+$ 973.4692, found 973.4687.

**B.4 4-TMR-DTX (25)**

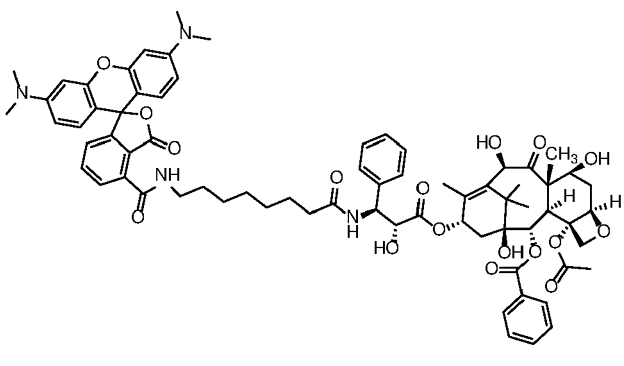

A solution of **DTX-C8-NHBoc (22)** (14.2 mg, 0.015 mmol) in 95% formic acid (1 mL) was stirred at room

temperature for 1 h. Reaction progress was monitored by HPLC analysis. Once reaction was complete, formic acid was evaporated on rotary evaporator and residue was dissolved in water and lyophilised to obtain white powder, which was used further without any additional purifications.

The **4-TMR-COOH (18)** dye TFA salt (5.5mg, 0.01 mmol, 1eq), DIPEA (52 μL, 0.3 mmol, 30 eq.) and HBTU (4.5 mg, 0.012 mmol, 1.2 eq.) were dissolved in 400 μL of dry MeCN and stirred at room temperature for 5 min. A solution of previously obtained deprotected DTX-C8-NH2 derivative (0.015 mmol, 1.5 eq) in MeCN and 10 μL of DIPEA were added to the reaction mixture and stirring continued for 1 hour. Reaction was monitored by HPLC analysis. Purified by preparative HPLC (preparative column: Eurospher 100 C18, 5 μm, 250 × 20 mm; solvent A: acetonitrile, solvent B: H2O + 0.2% v/v HCOOH; temperature 25 °C, gradient A:B - 5 min 20:80 isocratic, 5-30 min 20:80 to 70:30 gradient). Additionally purified by flash column chromatography (Interchim Puriflash 12g, 15μm normal phase column, gradient 3% to 30% DCM - MeOH), microfiltered through a 0.45 μm PTFE membrane filter and lyophilized from acetonitrile: water mixture. Yield 26% (3.3 mg) of light purple fluffy solid.

$^1$H NMR (400 MHz, $d_6$-dmso) δ 9.06 (t, J = 5.5 Hz, 1H), 8.38 (d, J = 9.0 Hz, 1H), 8.02 - 7.95 (m, 2H), 7.84 (dd, J = 7.6, 1.1 Hz, 1H), 7.76 (t, J = 7.6 Hz, 1H), 7.67 (tt, J = 7.2, 1.3 Hz, 1H), 7.64 - 7.55 (m, 2H), 7.41 - 7.28 (m, 4H), 7.25 - 7.17 (m, 2H), 6.64 - 6.57 (m, 2H), 6.53 - 6.43 (m, 4H), 5.99 - 5.86 (m, 2H), 5.41 (d, J = 7.2 Hz, 1H), 5.27 (dd, J = 9.0,5.9 Hz, 1H), 5.09 (d, J = 2.6 Hz, 1H), 5.01 (d, J = 7.3 Hz, 1H), 4.94 (d, J = 2.5 Hz, 1H), 4.90 (dd, J = 9.6, 2.2 Hz, 1H), 4.58 (s, 1H), 4.41 (t, J = 6.4 Hz, 1H), 4.12 - 3.96 (m, 3H), 3.68 (d, J = 7.1 Hz, 1H), 3.31 - 3.27 (m, 2H, overlapped with HDO), 2.94 (s, 13H), 2.33 - 2.26 (m, 1H), 2.23 (s, 3H), 2.17 (t, J = 7.3 Hz, 2H), 2.00 - 1.94 (m, 1H), 1.86 - 1.78 (m, 1H), 1.75 (s, 3H), 1.69 - 1.60 (m, 1H), 1.57 - 1.46 (m, 7H), 1.35 - 1.25 (m, 6H), 1.03 (s, 3H), 0.98 (s, 3H).

$^1$H-$^{13}$C NMR ((400, 101) MHz, $d_6$-dmso) δ (7.95 129.99), (7.81130.12), (7.73 135.55), (7.64133.72), (7.56 129.09), (7.33 128.55), (7.28 127.56), (7.20 125.80), (7.16 127.51), (6.57 129.08), (6.46 98.37), (6.46 109.29), (5.88 70.41), (5.38 75.24), (5.24 55.43), (5.06 74.19), (4.87 84.14), (4.38 74.03), (4.01 71.22), (3.98 75.85), (3.65 46.37), (3.29 39.79), (2.9140.22), (2.23, 1.63 36.87), (2.20 22.82), (2.14 35.86), (1.97, 1,78 35.53), (1.7114.09), (1.53 29.31), (1.49 10.24), (1.47 25.85), (1.31 26.89), (1.21 29.04), (1.21 29.22), (0.99 26.94), (0.95 21.26).

ESI-MS, positive mode: m/z = 1261.6 $[M+H]^+$.

HRMS (ESI) calcd for $C_{71}H_{81}N_4O_{17}$ $[M+H]^+$ 1261.5591, found 1261.5594.

**B.5 4-TMR-CTX (26)**

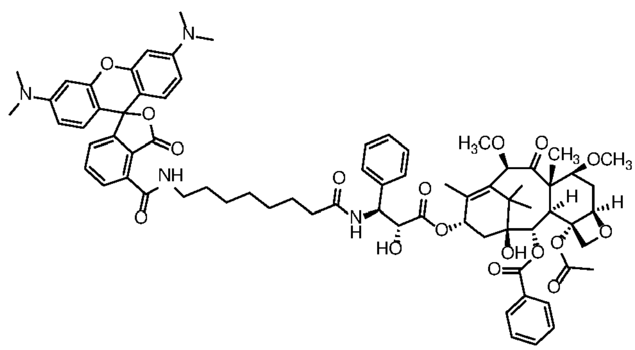

A solution of **CTX-C8-NHBoc (23)** (14.6 mg, 0.015 mmol) in 95% formic acid (1 mL) was stirred at room temperature for 1 h. Reaction progress was monitored by HPLC analysis. Once reaction was complete, formic acid was evaporated on rotary evaporator and residue was dissolved in water and lyophilised to obtain white powder, which was used further without any additional purifications.

The **4-TMR-COOH (18)** dye TFA salt (5.5 mg, 0.01 mmol, 1eq), DIPEA (52 μL, 0.3 mmol, 30 eq.) and HBTU (4.5 mg, 0.012 mmol, 1.2 eq.) were dissolved in 400 μL of dry MeCN and stirred at room temperature for 5 min. A solution of previously obtained deprotected CTX-C8-NH2 derivative (0.015 mmol, 1.5 eq) in MeCN and 10 μL of DIPEA were added to the reaction mixture and stirring continued for 1 hour. Reaction was monitored by HPLC analysis. Purified by preparative HPLC (preparative column: Eurospher 100 C18, 5 μm, 250 × 20 mm; solvent A: acetonitrile, solvent B: H2O + 0.2% v/v HCOOH; temperature 25 °C, gradient A:B - 5 min 20:80 isocratic, 5-30 min 20:80 to 70:30 gradient). Additionally purified by flash column chromatography (Interchim Puriflash 12g, 15μm column, gradient 3% to 30% DCM - MeOH), microfiltered through a 0.45 μm PTFE membrane filter and lyophilised from acetonitrile: water mixture. Yield 26% (3.3 mg) of light purple fluffy solid.

$^1$H NMR (400 MHz, $d_6$-dmso) δ 9.03 (t, J = 5.5 Hz, 1H), 8.35 (d, J = 9.1 Hz, 1H), 7.95 (d, J = 7.0 Hz, 2H), 7.81 (dd, J =

7.5, 1.1 Hz, 1H), 7.73 (t, J = 7.6 Hz, 1H), 7.67 - 7.61 (m, 1H), 7.59 - 7.53 (m, 2H), 7.36 - 7.28 (m, 4H), 7.22 - 7.15 (m, 2H), 6.58 (dd, J = 9.4, 1.6 Hz, 2H), 6.50 - 6.42 (m, 4H), 5.97 - 5.87 (m, 2H), 5.36 (d, J = 7.0 Hz, 1H), 5.27 (dd, J = 6.9, 2.4 Hz, 1H), 4.92 (d, J = 9.1 Hz, 1H), 4.67 (s, 1H), 4.62 (s, 1H), 4.40 (dd, J = 6.9, 5.8 Hz, 1H), 3.99 (s, 2H), 3.72 (dd, J = 10.9, 6.4 Hz, 1H), 3.60 (d, J = 7.1 Hz, 1H), 3.28 - 3.25 (m, 5H), 3.18 (s, 3H), 2.91 (s, 12H), 2.66 - 2.58 (m, 1H), 2.22 (s, 3H), 2.15 (t, J = 7.8 Hz, 2H), 1.99 - 1.92 (m, 3H), 1.88 - 1.81 (m, 1H), 1.80 (s, 3H), 1.54 - 1.47 (m, 8H), 1.27 - 1.23 (m, 6H), 1.00 (s, 3H), 0.94 (s, 3H).
$^1H$-$^{13}C$ NMR ((400, 101) MHz, $d_6$-dmso) δ (7.95 130.02), (7.80 130.16), (7.74 135.58), (7.64 133.77), (7.56 129.10), (7.32 128.58), (7.30 127.56), (7.20 125.78), (7.18 127.53), (6.57 129.08), (6.46 109.29), (6.46 98.37), (5.91 70.40), (5.35 74.79), (5.26 55.39), (4.93 83.66), (4.67 82.52), (4.39 74.01), (3.99 75.71), (3.72 80.68), (3.60 46.84), (3.29 39.78), (3.27 57.02), (3.18 57.08), (2.91 40.22), (2.63, 1.46 32.13), (2.21 22.82), (2.15 35.84), (1.98, 1.83 35.48), (1.95 26.99), (1.80 14.44), (1.49 10.56), (1.47 25.85), (1.32 26.90), (1.21 29.21), (1.20 31.71), (1.00 27.12), (0.94 21.63).
ESI-MS, positive mode: m/z = 1289.6 $[M+H]^+$.
HRMS (ESI) calcd for $C_{73}H_{85}N_4O_{17}$ $[M+H]^+$ 1289.5904, found 1289.5919.

**B.6 4-TMR-LTX (27)**

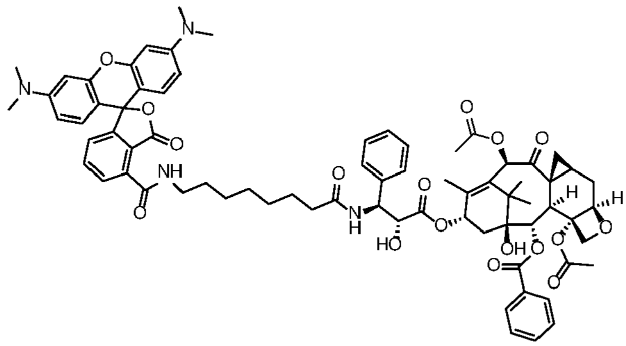

A solution of **LTX-C8-NHBoc (23)** (14.6 mg, 0.015 mmol) in 95% formic acid (1 mL) was stirred at room temperature for 1 h. Reaction progress was monitored by HPLC analysis. Once reaction was complete, formic acid was evaporated on rotary evaporator and residue was dissolved in water and lyophilised to obtain white powder, which was used further without any additional purifications.

The **4-TMR-COOH (18)** dye TFA salt (5.5 mg, 0.01 mmol, 1eq), DIPEA (52 μL, 0.3 mmol, 30 eq.) and HBTU (4.5 mg, 0.012 mmol, 1.2 eq.) were dissolved in 400 μL of dry MeCN and stirred at room temperature for 5 min. A solution of previously obtained deprotected LTX-C8-NH2 derivative (0.015 mmol, 1.5 eq) in MeCN and 10 μL of DIPEA were added to the reaction mixture and stirring continued for 1 hour. Reaction was monitored by HPLC analysis.

Purified by preparative HPLC (preparative column: Eurospher 100 C18, 5 μm, 250 × 20 mm; solvent A: acetonitrile, solvent B: H2O + 0.2% v/v HCOOH; temperature 25 °C, gradient A:B - 5 min 20:80 isocratic, 5-30 min 20:80 to 70:30 gradient). Additionally purified by flash column chromatography (Interchim Puriflash 12g, 15μm column, gradient 3% to 30% DCM - MeOH), microfiltered through a 0.45 μm PTFE membrane filter and lyophilised from acetonitrile: water mixture. Yield 44% (5.6 mg) of light purple fluffy solid.
$^1H$ NMR (400 MHz, $d_6$-dmso) δ 9.07 (t, J = 5.5 Hz, 1H), 8.50 (d, J = 9.1 Hz, 1H), 8.02 (d, J = 7.2 Hz, 2H), 7.84 (d, J = 7.5 Hz, 1H), 7.76 (t, J = 7.6 Hz, 1H), 7.68 (dd, J = 8.4, 6.1 Hz, 1H), 7.60 (t, J = 7.6 Hz, 2H), 7.37 - 7.30 (m, 4H), 7.23 (d, J = 7.5 Hz, 1H), 7.17 (tt, J = 7.3, 1.9 Hz, 1H), 6.61 (d, J = 9.5 Hz, 2H), 6.50 - 6.46 (m, 4H), 6.12 (s, 1H), 5.94 (t, J = 9.0 Hz, 1H), 5.50 - 5.33 (m, 2H), 5.32 (dd, J = 9.1, 5.4 Hz, 1H), 4.77 (s, 1H), 4.71 (d, J = 3.1 Hz, 1H), 4.49 (d, J = 5.5 Hz, 1H), 4.05 - 3.96 (m, 2H), 3.90 (d, J = 7.6 Hz, 1H), 3.30 - 3.27 (m, 2H), 2.94 (s, 12H), 2.32 - 2.25 (m, 4H), 2.17 - 1.86 (m, 9H), 1.74 (s, 3H), 1.59 - 1.44 (m, 5H), 1.35 - 1.20 (m, 7H), 1.11 (s, 3H), 1.08 (s, 3H).
$^1H$-$^{13}C$ NMR ((400, 101) MHz, $d_6$-dmso) δ (8.00 130.09), (7.81130.12), (7.74 135.60), (7.65 133.75), (7.57 129.19), (7.31 128.62), (7.30 127.50), (7.20 125.77), (7.15 127.38), (6.57 129.09), (6.47 109.30), (6.46 98.37), (6.09 75.67), (5.92 69.90), (5.40 79.98), (5.29 55.23), (4.69 84.16), (4.46 73.96), (3.96 74.90), (3.88 37.97), (3.29 39.77), (2.91 38.96), (2.91 40.22), (2.26 22.36), (2.25 26.03), (2.13 35.83), (2.09 20.99), (1.81 14.82), (1.71 14.39), (1.52 29.37), (1.45 25.88), (1.30 26.91), (1.27 29.01), (1.21 29.10), (1.15 31.63), (1.08 21.82), (1.05 26.34).
ESI-MS, positive mode: m/z = 1285.7 $[M+H]^+$.
HRMS (ESI) calcd for $C_{73}H_{81}N_4O_{17}$ [M+H]+ 1285.5591, found 1285.5591.

**B.7 4-580CP-LTX (28)**

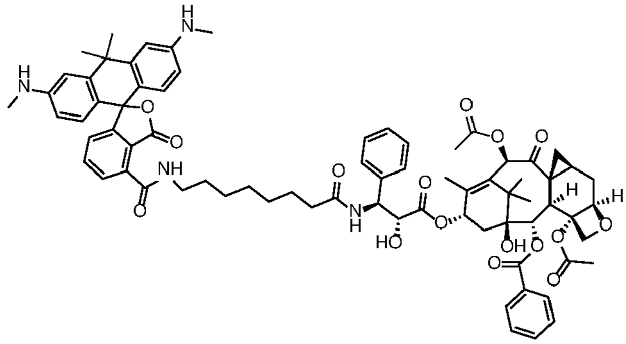

A solution of **LTX-C8-NHBoc (23)** (14.6 mg, 0.015 mmol) in 95% formic acid (1 mL) was stirred at room temperature for 1 h. Reaction progress was monitored by HPLC analysis. Once reaction was complete, formic acid was evaporated on rotary evaporator and residue was dissolved in water and lyophilised to obtain a white powder, which was used further without any additional purifications.

The **4-580CP-COOH (19)** dye TFA salt (5.4 mg, 0.01 mmol, 1eq), DIPEA (52 μL, 0.3 mmol, 30 eq.) and HBTU (4.5 mg, 0.012 mmol, 1.2 eq.) were dissolved in 400 μL of dry MeCN and stirred at room temperature for 5 min. A solution of previously obtained deprotected LTX-C8-NH2 derivative (0.015 mmol, 1.5 eq) in MeCN and 10 μL of DIPEA were added to the reaction mixture and stirring continued for 1 hour. Reaction was monitored by HPLC analysis.

Purified by preparative HPLC (preparative column: Eurospher 100 C18, 5 μm, 250 × 20 mm; solvent A: acetonitrile, solvent B: H2O + 0.2% v/v HCOOH; temperature 25 °C, gradient A:B - 5 min 30:70 isocratic, 5-30 min 30:70 to 70:30 gradient). Additionally purified by flash column chromatography (Interchim Puriflash 12g, 15μm column, gradient 2% to 15% DCM - MeOH) and lyophilised from acetonitrile: water mixture. Yield 38% (4.9 mg) of violet fluffy solid.

$^1$H NMR (500 MHz, $d_6$-dmso) δ 9.14 (t, J = 5.5 Hz, 1H), 8.37 (d, J = 9.2 Hz, 1H), 8.03 (d, J = 7.1 Hz, 2H), 7.78 (dd, J = 7.5, 1.0 Hz, 1H), 7.71 - 7.65 (m, 2H), 7.60 (t, J = 7.6 Hz, 2H), 7.38 - 7.31 (m, 4H), 7.17 (tt, J = 7.0, 1.6 Hz, 1H), 7.05 (dd, J = 7.7, 1.0 Hz, 1H), 6.76 (d, J = 2.4 Hz, 2H), 6.45 (d, J = 8.6 Hz, 2H), 6.41 - 6.36 (m, 2H), 6.12 (s, 1H), 5.96 (t, J = 8.9 Hz, 1H), 5.91 - 5.80 (m, 3H), 5.43 (d, J = 7.7 Hz, 1H), 5.34 (dd, J = 9.2, 5.2 Hz, 1H), 4.78 (s, 1H), 4.72 (d, J = 4.1 Hz, 1H), 4.49 (dd, J = 6.8, 5.3 Hz, 1H), 4.05 - 3.97 (m, 2H), 3.91 (d, J = 7.6 Hz, 1H), 3.30 - 3.16 (m, 2H), 2.70 (s, 3H), 2.69 (s, 3H), 2.31 - 2.25 (m, 4H), 2.18 - 1.88 (m, 9H), 1.75 (s, 3H), 1.74 (s, 3H), 1.65 (s, 3H), 1.57 - 1.45 (m, 5H), 1.37 - 1.23 (m, 7H), 1.11 (s, 3H), 1.08 (s, 3H).

$^1$H-$^{13}$C NMR ((500,126) MHz, $d_6$-dmso) δ (8.03 129.69), (7.77 129.15), (7.69 134.93), (7.68 133.36), (7.60 128.78), (7.35 128.15), (7.33 127.07), (7.17 127.04), (7.06 125.16), (6.75 107.95), (6.46 128.47), (6.38 111.40), (6.11 75.23), (5.95 69.56), (5.44 79.53), (5.34 54.71), (4.71 83.75), (4.49 73.54), (4.02, 3.99 74.38), (3.91 37.53), (3.32 39.34), (2.69 29.56), (2.31,1.95 25.56), (2.30 21.95), (2.16 35.41), (2.12 20.57), (2.09, 1.89 35.55), (2.03, 1.53 14.88), (1.75 33.25), (1.73 13.98), (1.65 34.56), (1.55 28.92), (1.47 25.46), (1.36 28.30), (1.33 26.48), (1.23 28.88), (1.23 28.57), (1.11 21.41), (1.07 25.91).

ESI-MS, positive mode: m/z = 1283.7 $[M+H]^+$.

HRMS (ESI) calcd for $C_{74}H_{82}N_4O_{16}$ $[M+H]^+$ 1283.5799, found 1283.5796

**B.8 4-610CP-DTX (29)**

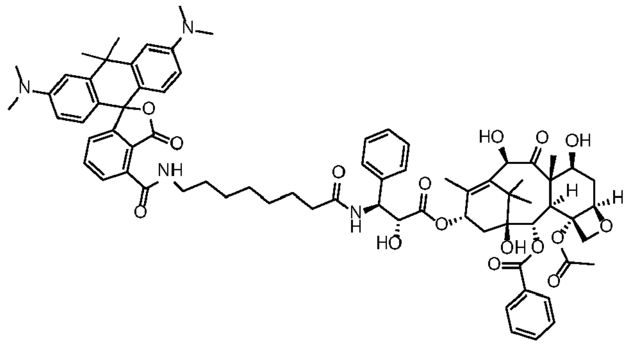

A solution of **DTX-C8-NHBoc (22)** (14.2 mg, 0.015 mmol) in 95% formic acid (1 mL) was stirred at room temperature for 1 h. Reaction progress was monitored by HPLC analysis. Once reaction was complete, formic acid was evaporated on rotary evaporator and residue was dissolved in water and lyophilised to obtain white powder, which was used further without any additional purifications.

The **4-610CP-COOH (20)** dye TFA salt (5.7 mg, 0.01 mmol, 1eq), DIPEA (52 μL, 0.3 mmol, 30 eq.) and HBTU (4.5 mg, 0.012 mmol, 1.2 eq.) were dissolved in 400 μL of dry MeCN and stirred at room temperature for 5 min. A solution of previously obtained deprotected DTX-C8-NH2 derivative (0.015 mmol, 1.5 eq) in MeCN and 10 μL of DIPEA were added to the reaction mixture and stirring continued for 1 hour. Reaction was monitored by HPLC analysis.

Purified by preparative HPLC (preparative column: Eurospher 100 C18, 5 μm, 250 × 20 mm; solvent A: acetonitrile, solvent B: H2O + 0.2% v/v HCOOH; temperature 25 °C, gradient A:B - 5 min 30:70 isocratic, 5-30 min 30:70 to 100:0 gradient). Lyophilised from acetonitrile: water mixture. Yield 42% (5.4 mg) of slightly blue fluffy solid.

$^1$H NMR (400 MHz, $d_6$-dmso) δ 9.11 (t, J = 5.5 Hz, 1H), 8.37 (d, J = 9.0 Hz, 1H), 8.03 - 7.95 (m, 2H), 7.78 (dd, J = 7.5, 1.0 Hz, 1H), 7.72 - 7.64 (m, 2H), 7.59 (t, J = 7.4 Hz, 2H), 7.39 - 7.30 (m, 4H), 7.20 (tt, J = 7.1, 1.4 Hz, 1H), 7.04 (d, J = 7.7 Hz, 1H), 6.91 (s, 2H), 6.63 - 6.52 (m, 4H), 6.03 - 5.82 (m, 2H), 5.41 (d, J = 7.3 Hz, 1H), 5.28 (dd, J = 9.1, 5.8 Hz, 1H), 5.10 (s, 1H), 5.01 (s, 1H), 4.90 (dd, J = 9.8, 1.6 Hz, 1H), 4.58 (s, 1H), 4.41 (t, J = 5.0 Hz, 1H), 4.10 - 3.96 (m, 3H), 3.68 (d, J = 7.2 Hz, 1H), 3.31 - 3.27 (m, 2H), 2.94 (s, 12H), 2.32 - 2.25 (m, 1H), 2.24 (s, 3H), 2.17 (t, J = 7.3 Hz, 2H), 1.97 (dd, J = 15.1, 9.6 Hz, 1H), 1.86 - 1.80 (m, 4H), 1.75 (s, 3H), 1.72 (s, 3H), 1.69 - 1.63 (m, 1H), 1.61 - 1.55 (m, 2H), 1.52 (s, 3H), 1.52 - 1.47 (m, 2H), 1.38 - 1.25 (m, 6H), 1.03 (s, 3H), 0.98 (s, 3H).

$^1$H-$^{13}$C NMR ((400, 101) MHz, $d_6$-dmso) δ (7.95 130.00), (7.74 129.59), (7.65 135.43), (7.64 133.73), (7.56 129.09), (7.33 128.57), (7.29 127.57), (7.17 127.53), (7.01 125.46), (6.88 109.57), (6.54 112.20), (6.53 128.80), (5.88 70.45), (5.38 75.26), (5.25 55.43), (5.06 74.19), (4.86 84.14), (4.38 74.02), (4.02 71.22), (3.99 75.89), (3.65 46.41), (3.30 39.78), (2.90 40.44), (2.24, 1.62 36.90), (2.21 22.82), (2.14 35.86), (1.94, 1.78 35.44), (1.79 34.01), (1.72 14.09), (1.68 34.96), (1.54 29.35), (1.49 10.24), (1.47 25.86), (1.32 26.91), (1.24 29.03), (1.20 29.22), (1.00 26.94), (0.95 21.26).

ESI-MS, positive mode: m/z = 1287.6 [M+H]$^+$.

HRMS (ESI) calcd for $C_{74}H_{87}N_4O_{16}$ [M+H]$^+$ 1287.6112, found 1287.6110.

### B.9 4-610CP-CTX (30)

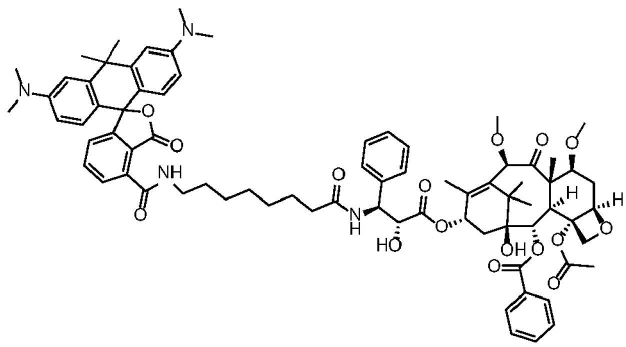

A solution of **CTX-C8-NHBoc (23)** (14.6 mg, 0.015 mmol) in 95% formic acid (1 mL) was stirred at room temperature for 1 h. Reaction progress was monitored by HPLC analysis. Once reaction was complete, formic acid was

evaporated on rotary evaporator and residue was dissolved in water and lyophilised to obtain white powder, which was used further without any additional purifications.

The **4-610CP-COOH (20)** dye TFA salt (5.7 mg, 0.01 mmol, 1eq), DIPEA (52 μL, 0.3 mmol, 30 eq.) and HBTU (4.5 mg, 0.012 mmol, 1.2 eq.) were dissolved in 400 μL of dry MeCN and stirred at room temperature for 5 min. A solution of previously obtained deprotected CTX-C8-NH2 derivative (0.015 mmol, 1.5 eq) in MeCN and 10 μL of DIPEA were added to the reaction mixture and stirring continued for 1 hour. Reaction was monitored by HPLC analysis.

Purified by preparative HPLC (preparative column: Eurospher 100 C18, 5 μm, 250 × 20 mm; solvent A: acetonitrile, solvent B: H2O + 0.2% v/v HCOOH; temperature 25 °C, gradient A:B - 5 min 30:70 isocratic, 5-30 min 30:70 to 100:0 gradient). Lyophilised from acetonitrile: water mixture. Yield 46% (6.0 mg) of light blue fluffy solid.

$^1$H NMR (400 MHz, $d_6$-dmso) δ 9.09 (t, J = 5.5 Hz, 1H), 8.37 (d, J = 9.0 Hz, 1H), 7.98 - 7.91 (m, 2H), 7.75 (dd, J = 7.5,1.0 Hz, 1H), 7.68 - 7.61 (m, 2H), 7.61 - 7.51 (m, 2H), 7.36 - 7.26 (m, 4H), 7.18 (tt, J = 7.1, 1.6 Hz, 1H), 7.01 (dd, J = 7.7, 1.0 Hz, 1H), 6.88 (d, J = 2.0 Hz, 2H), 6.60 - 6.49 (m, 4H), 6.00 - 5.87 (m, 2H), 5.36 (d, J = 7.1 Hz, 1H), 5.26 (dd, J = 9.1, 5.8 Hz, 1H), 4.92 (dd, J = 9.6, 2.1 Hz, 1H), 4.68 (s, 1H), 4.62 (s, 1H), 4.40 (t, J = 6.2 Hz, 1H), 3.99 (s, 2H), 3.72 (dd, J = 10.6, 6.6 Hz, 1H), 3.60 (d, J = 7.1 Hz, 1H), 3.34 - 3.31 (m, 2H), 3.27 (s, 3H), 3.18 (s, 3H), 2.91 (s, 12H), 2.69 - 2.57 (m, 1H), 2.21 (s, 3H), 2.15 (t, J = 7.3 Hz, 2H), 1.97 - 1.91 (m, 1H), 1.88 - 1.82 (m, 1H), 1.80 (s, 3H), 1.79 (s, 3H), 1.69 (s, 3H), 1.56 - 1.43 (m, 8H), 1.37 - 1.23 (m, 6H), 1.00 (s, 3H), 0.94 (s, 3H).

$^1$H-$^{13}$C NMR ((400, 101) MHz, $d_6$-dmso) δ (7.95 130.01), (7.75 129.60), (7.66 135.42), (7.64 133.80), (7.56 129.10), (7.33 128.58), (7.30 127.57), (7.18 127.54), (7.01 125.47), (6.88 109.55), (6.53 112.18), (6.53 128.79), (5.92 70.41), (5.36 74.79), (5.26 55.41), (4.91 83.63), (4.67 82.52), (4.40 74.01), (3.99 75.70), (3.72 80.67), (3.60 46.85), (3.30 39.78), (3.27 57.03), (3.18 57.07), (2.90 40.41), (2.62, 1.46 32.14), (2.21 22.81), (2.15 35.84), (1.94, 1.84 35.26), (1.80 14.44), (1.79 34.01), (1.68 34.94), (1.54 29.33), (1.49 10.58), (1.48 25.84), (1.32 26.90), (1.24 29.05), (1.22 29.13), (1.00 27.12), (0.94 21.63).

ESI-MS, positive mode: m/z = 1315.6 $[M+H]^+$.

HRMS (ESI) calcd for C76H91N4O16 $[M+H]^+$ 1315.6425, found 1315.6409.

**B.10 4-610CP-LTX (31)**

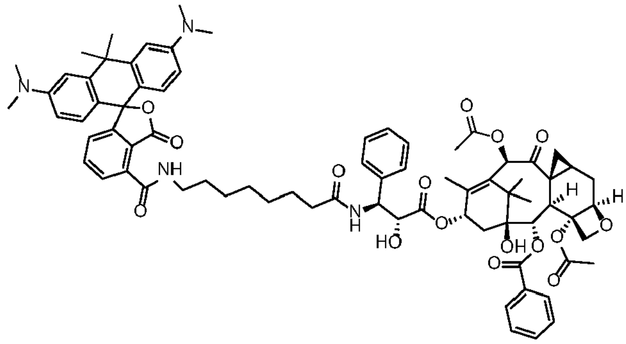

A solution of **LTX-C8-NHBoc (23)** (14.6 mg, 0.015 mmol) in 95% formic acid (1 mL) was stirred at room temperature for 1 h. Reaction progress was monitored by HPLC analysis. Once reaction was complete, formic acid was evaporated on rotary evaporator and residue was dissolved in water and lyophilised to obtain white powder, which was used further without any additional purifications.

The **4-610CP-COOH (20)** dye TFA salt (5.7 mg, 0.01 mmol, 1eq), DIPEA (52 μL, 0.3 mmol, 30 eq.) and HBTU (4.5 mg, 0.012 mmol, 1.2 eq.) were dissolved in 400 μL of dry MeCN and stirred at room temperature for 5 min. A solution of previously obtained deprotected LTX-C8-NH2 derivative (0.015 mmol, 1.5 eq) in MeCN and 10 μL of DIPEA were added to the reaction mixture and stirring continued for 1 hour. Reaction was monitored by HPLC analysis.

Purified by preparative HPLC (preparative column: Eurospher 100 C18, 5 μm, 250 × 20 mm; solvent A: acetonitrile, solvent B: H2O + 0.2% v/v HCOOH; temperature 25 °C, gradient A:B - 5 min 30:70 isocratic, 5-30 min 30:70 to 100:0 gradient). Lyophilised from acetonitrile: water mixture. Yield 35% (4.7 mg) of light blue fluffy solid.

$^1$H NMR (400 MHz, $d_6$-dmso) δ 9.09 (t, J = 5.5 Hz, 1H), 8.37 (d, J = 8.8 Hz, 1H), 8.04 - 7.96 (m, 2H), 7.75 (d, J = 7.4 Hz, 1H), 7.70 - 7.61 (m, 2H), 7.57 (t, J = 7.6 Hz, 2H), 7.37 - 7.25 (m, 4H), 7.14 (tt, J = 7.0, 1.9 Hz, 1H), 7.01 (d, J = 7.6 Hz, 1H), 6.88 (d, J = 2.1 Hz, 2H), 6.59 - 6.48 (m, 4H), 6.09 (s, 1H), 6.00 - 5.81 (m, 2H), 5.40 (d, J = 7.6 Hz, 1H), 5.31 (dd, J = 9.1, 5.3 Hz, 1H), 4.74 (s, 1H), 4.68 (d, J = 3.7 Hz, 1H), 4.46 (d, J = 3.8 Hz, 1H), 3.97 (dd, J = 20.5, 8.5 Hz, 2H), 3.88 (d, J = 7.6 Hz, 1H), 3.28 - 3.26 (m, 2H), 2.91 (s, 12H), 2.27 (s, 4H), 2.15 - 1.84 (m, 9H), 1.79 (s, 3H), 1.71 (s, 3H),

1.69 (s, 3H), 1.55 - 1.44 (m, 5H), 1.33 - 1.21 (m, 7H), 1.08 (s, 3H), 1.05 (s, 3H).
ESI-MS, positive mode: m/z = 1311.6 $[M+H]^+$.
HRMS (ESI) calcd for $C_{76}H_{87}N_4O_{16}$ [M+H]+ 1311.6112, found 1311.6085.

**B.11 4-SiR-DTX (32)**

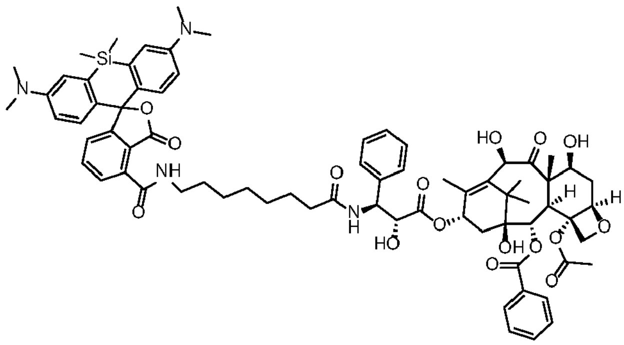

A solution of **DTX-C8-NHBoc (22)** (14.2 mg, 0.015 mmol) in 95% formic acid (1 mL) was stirred at room temperature for 1 h. Reaction progress was monitored by HPLC analysis. Once reaction was complete, formic acid was evaporated on rotary evaporator and residue was dissolved in water and lyophilised to obtain white powder, which was used further without any additional purifications.

The **4-SiR-COOH (20)** dye TFA salt (5.9 mg, 0.01 mmol, 1eq), DIPEA (52 μL, 0.3 mmol, 30 eq.) and HBTU (4.5 mg, 0.012 mmol, 1.2 eq.) were dissolved in 400 μL of dry MeCN and stirred at room temperature for 5 min. A solution of previously obtained deprotected DTX-C8-NH2 derivative (0.015 mmol, 1.5 eq) in MeCN and 10 μL of DIPEA were added to the reaction mixture and stirring continued for 1 hour. Reaction was monitored by HPLC analysis.

Purified by preparative HPLC (preparative column: Eurospher 100 C18, 5 μm, 250 × 20 mm; solvent A: acetonitrile, solvent B: H2O + 0.2% v/v HCOOH; temperature 25 °C, gradient A:B - 5 min 40:60 isocratic, 5-30 min 40:60 to 100:0 gradient) and lyophilised from acetonitrile and water mixture. Yield 29% (3.8 mg) of slightly blue fluffy solid.
$^1H$ NMR (400 MHz, $d_6$-dmso) δ 9.01 (t, J = 5.5 Hz, 1H), 8.38 (d, J = 9.0 Hz, 1H), 8.03 - 7.95 (m, 2H), 7.80 - 7.72 (m, 2H), 7.67 (tt, J = 7.3, 1.5 Hz, 1H), 7.59 (t, J = 7.7, 7.2 Hz, 2H), 7.40 - 7.29 (m, 4H), 7.27 - 7.22 (m, 1H), 7.22 - 7.16 (m, 1H), 7.00 (d, J = 2.8 Hz, 2H), 6.70 (dd, J = 8.9, 1.0 Hz, 2H), 6.67 - 6.60 (m, 2H), 5.96 - 5.87 (m, 2H), 5.41 (d, J = 7.2 Hz, 1H), 5.27 (dd, J = 9.0, 5.9 Hz, 1H), 5.09 (d, J = 2.5 Hz, 1H), 5.01 (d, J = 7.2 Hz, 1H), 4.94 (d, J = 2.5 Hz, 1H), 4.90 (dd, J = 9.7, 2.2 Hz, 1H), 4.58 (s, 1H), 4.41 (t, J = 6.4 Hz, 1H), 4.10 - 3.96 (m, 3H), 3.68 (d, J = 7.1 Hz, 1H), 3.30 - 3.25 (m, 2H), 2.92 (s, 12H), 2.34 - 2.25 (m, 1H), 2.24 (s, 3H), 2.17 (t, J = 7.4 Hz, 2H), 2.02 - 1.92 (m, 1H), 1.81 (dd, J = 15.3, 9.0 Hz, 1H), 1.75 (s, 3H), 1.66 (dd, J = 15.4, 10.1 Hz, 1H), 1.58 - 1.45 (m, 7H), 1.39 - 1.24 (m, 6H), 1.03 (s, 3H), 0.98 (s, 3H), 0.62 (s, 3H), 0.52 (s, 3H).
$^1H$-$^{13}C$ NMR ((400, 101) MHz, $d_6$-dmso) δ (7.98 130.02), (7.76 129.42), (7.76 134.93), (7.67 133.74), (7.60 129.08), (7.36 128.57), (7.32 127.56), (7.24 125.80), (7.20 127.58), (7.00 116.66), (6.69 128.36), (6.64 114.05), (5.91 70.43), (5.42 75.21), (5.28 55.43), (5.10 74.21), (4.90 84.11), (4.42 73.99), (4.06 71.20), (4.03 75.88), (3.68 46.40), (3.30 39.76), (2.92 40.22), (2.27, 1.66 36.90), (2.24 22.83), (2.17 35.85), (1.97, 1.81 35.53), (1.75 14.10), (1.54 29.30), (1.53 10.24), (1.50 25.85), (1.34 26.88), (1.26 29.03), (1.25 29.06), (1.03 26.94), (0.98 21.26), (0.62 -0.75), (0.52 0.41).
ESI-MS, positive mode: m/z = 1303.6 $[M+H]^+$.
HRMS (ESI) calcd for $C_{73}H_{87}N_4O_{16}Si$ $[M+H]^+$ 1303.5881, found 1303.5882.

**B.12 4-SiR-CTX (33)**

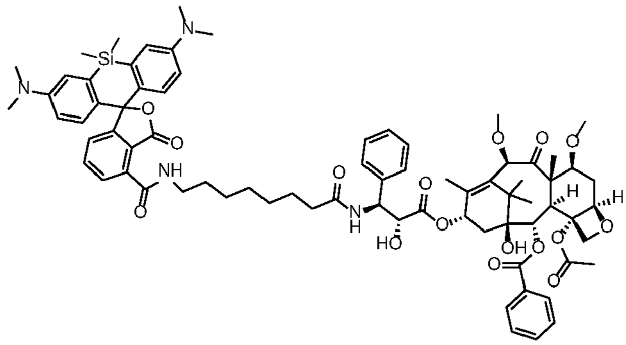

A solution of **CTX-C8-NHBoc (23)** (14.6 mg, 0.015 mmol) in 95% formic acid (1 mL) was stirred at room temperature for 1 h. Reaction progress was monitored by HPLC analysis. Once reaction was complete, formic acid was evaporated on rotary evaporator and residue was dissolved in water and lyophilised to obtain white powder, which was used further without any additional purifications.

The **4-SiR-COOH (20)** dye TFA salt (5.9 mg, 0.01 mmol, 1eq), DIPEA (52 μL, 0.3 mmol, 30 eq.) and HBTU (4.5 mg, 0.012 mmol, 1.2 eq.) were dissolved in 400 μL of dry MeCN and stirred at room temperature for 5 min. A solution of previously obtained deprotected CTX-C8-NH2 derivative (0.015 mmol, 1.5 eq) in MeCN and 10 μL of DIPEA were added to the reaction mixture and stirring continued for 1 hour. Reaction was monitored by HPLC analysis.

Purified by preparative HPLC (preparative column: Eurospher 100 C18, 5 μm, 250 × 20 mm; solvent A: acetonitrile, solvent B: H2O + 0.2% v/v HCOOH; temperature 25 °C, gradient A:B - 5 min 40:60 isocratic, 5-30 min 40:60 to 100:0 gradient) and lyophilised from acetonitrile and water mixture. Yield 51% (6.8 mg) of slightly blue fluffy solid.
$^1$H NMR (400 MHz, $d_6$-dmso) δ 8.99 (t, J = 5.5 Hz, 1H), 8.34 (d, J = 9.0 Hz, 1H), 7.95 (d, J = 6.9 Hz, 2H), 7.74 - 7.71 (m, 2H), 7.66 - 7.61 (m, 1H), 7.56 (t, J = 7.5 Hz, 2H), 7.32 (ddd, J = 15.2, 8.2, 6.8 Hz, 4H), 7.21 (h, J = 2.9, 1.9 Hz, 2H), 6.97 (d, J = 2.8 Hz, 2H), 6.67 (dd, J = 9.0, 1.2 Hz, 2H), 6.63 - 6.59 (m, 2H), 5.92 (dd, J = 8.5, 6.4 Hz, 2H), 5.36 (d, J = 7.1 Hz, 1H), 5.26 (dd, J = 9.1, 5.8 Hz, 1H), 4.92 (d, J = 9.5 Hz, 1H), 4.67 (s, 1H), 4.62 (s, 1H), 4.40 (dd, J = 6.9, 5.8 Hz, 1H), 3.99 (s, 2H), 3.72 (dd, J = 10.6, 6.5 Hz, 1H), 3.60 (d, J = 7.1 Hz, 1H), 3.27 (s, 3H), 3.27 - 3.24 (m, 2H), 3.18 (s, 3H), 2.89 (s, 12H), 2.69 - 2.56 (m, 1H), 2.22 (s, 3H), 2.15 (t, J = 7.4 Hz, 2H), 1.94 (dd, J = 15.3, 8.9 Hz, 1H), 1.87 - 1.81 (m, 1H), 1.80 (s, 3H), 1.54 - 1.43 (m, 8H), 1.34 - 1.17 (m, 6H), 1.00 (s, 3H), 0.94 (s, 3H), 0.59 (s, 3H), 0.49 (s, 3H).
$^1$H-$^{13}$C NMR ((400, 101) MHz, $d_6$-dmso) δ (7.95 129.99), (7.73 129.43), (7.73 134.94), (7.64 133.81), (7.56 129.12), (7.33 128.58), (7.30 127.56), (7.21 125.83), (7.18 127.50), (6.97 116.66), (6.66 128.36), (6.61 114.05), (5.92 70.41), (5.36 74.79), (5.26 55.36), (4.92 83.65), (4.67 82.53), (4.39 74.02), (3.99 75.71), (3.73 80.65), (3.60 46.85), (3.27 57.03), (3.27 39.77), (3.18 57.09), (2.88 40.22), (2.62, 1.47 32.13), (2.22 22.83), (2.14 35.85), (1.94, 1.85 35.32), (1.80 14.45), (1.51 29.28), (1.49 10.58), (1.47 25.86), (1.30 26.87), (1.22 29.10), (1.21 29.15), (1.00 27.13), (0.94 21.64), (0.59 -0.75), (0.49 0.41).
ESI-MS, positive mode: m/z = 1331.6 $[M+H]^+$.
HRMS (ESI) calcd for $C_{75}H_{91}N_4O_{16}Si$ $[M+H]^+$ 1331.6194, found 1331.6184.

**B.13 4-SiR-LTX (34)**

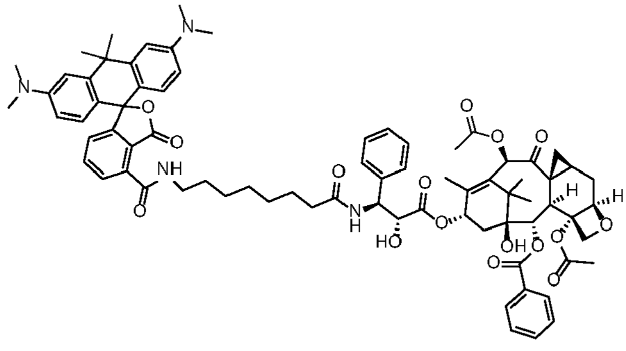

A solution of **LTX-C8-NHBoc (23)** (14.6 mg, 0.015 mmol) in 95% formic acid (1 mL) was stirred at room

temperature for 1 h. Reaction progress was monitored by HPLC analysis. Once reaction was complete, formic acid was evaporated on rotary evaporator and residue was dissolved in water and lyophilised to obtain white powder, which was used further without any additional purifications.

The **4-SiR-COOH (20)** dye TFA salt (5.7 mg, 0.01 mmol, 1eq), DIPEA (52 μL, 0.3 mmol, 30 eq.) and HBTU (4.5 mg, 0.012 mmol, 1.2 eq.) were dissolved in 400 μL of dry MeCN and stirred at room temperature for 5 min. A solution of previously obtained deprotected LTX-C8-NH2 derivative (0.015 mmol, 1.5 eq) in MeCN and 10 μL of DIPEA were added to the reaction mixture and stirring continued for 1 hour. Reaction was monitored by HPLC analysis.

Purified by preparative HPLC (preparative column: Eurospher 100 C18, 5 μm, 250 × 20 mm; solvent A: acetonitrile, solvent B: H2O + 0.2% v/v HCOOH; temperature 25 °C, gradient A:B - 5 min 40:60 isocratic, 5-30 min 40:60 to 100:0 gradient). Lyophilised from acetonitrile: water mixture. Yield 41% (5.4 mg) of light blue fluffy solid.

$^1$H NMR (400 MHz, $d_6$-dmso) δ 8.99 (t, J = 5.5 Hz, 1H), 8.35 (d, J = 9.2 Hz, 1H), 8.00 (dd, J = 7.0, 1.4 Hz, 2H), 7.78 - 7.68 (m, 2H), 7.65 (tt, J = 7.3, 1.3 Hz, 1H), 7.57 (t, J = 7.4 Hz, 2H), 7.37 - 7.25 (m, 4H), 7.22 (dd, J = 5.7, 3.0 Hz, 1H), 7.14 (tt, J = 7.2, 1.9 Hz, 1H), 6.97 (d, J = 2.8 Hz, 2H), 6.67 (dd, J = 8.8, 0.8 Hz, 2H), 6.60 (ddd, J = 9.0, 2.8, 1.4 Hz, 2H), 6.09 (s, 1H), 5.99 - 5.84 (m, 2H), 5.41 (d, J = 7.7 Hz, 1H), 5.31 (dd, J = 9.2, 5.2 Hz, 1H), 4.74 (s, 1H), 4.68 (d, J = 4.1 Hz, 1H), 4.46 (t, J = 5.3 Hz, 1H), 3.98 (dd, J = 20.6, 8.6 Hz, 2H), 3.88 (d, J = 7.6 Hz, 1H), 3.25 (d, J = 6.7 Hz, 2H), 2.89 (s, 12H), 2.27 (s, 4H), 2.15 - 1.83 (m, 9H), 1.71 (s, 3H), 1.55 - 1.40 (m, 5H), 1.31 - 1.16 (m, 7H), 1.08 (s, 3H), 1.05 (s, 3H), 0.59 (s, 3H), 0.49 (s, 3H).

$^1$H-$^{13}$C NMR ((400, 101) MHz, $d_6$-dmso) δ (7.99 130.06), (7.74 129.45), (7.73 134.93), (7.64 133.75), (7.57 129.16), (7.30 127.71), (7.22 125.83), (7.14 127.47), (6.97 116.67), (6.66 128.37), (6.65 114.02), (6.61 114.03), (6.09 75.65), (5.92 69.96), (5.40 79.98), (5.31 55.15), (4.68 84.19), (4.46 73.97), (3.97 74.81), (3.88 37.96), (3.26 39.74), (2.88 40.21), (2.27 22.36), (2.24, 1.96 26.00), (2.12 35.84), (2.09 20.98), (2.07, 1.87 36.02), (1.99, 1.51 15.14), (1.71 14.39), (1.50 29.29), (1.44 25.87), (1.28 26.86), (1.22 26.99), (1.20 29.11), (1.14 31.67), (1.08 21.82), (1.05 26.34), (0.59 -0.76), (0.49 0.41).

ESI-MS, positive mode: m/z = 1327.6 $[M+H]^+$.

HRMS (ESI) calcd for $C_{75}H_{87}N_4O_{16}Si$ $[M+H]^+$ 1327.5881, found 1327.5895.

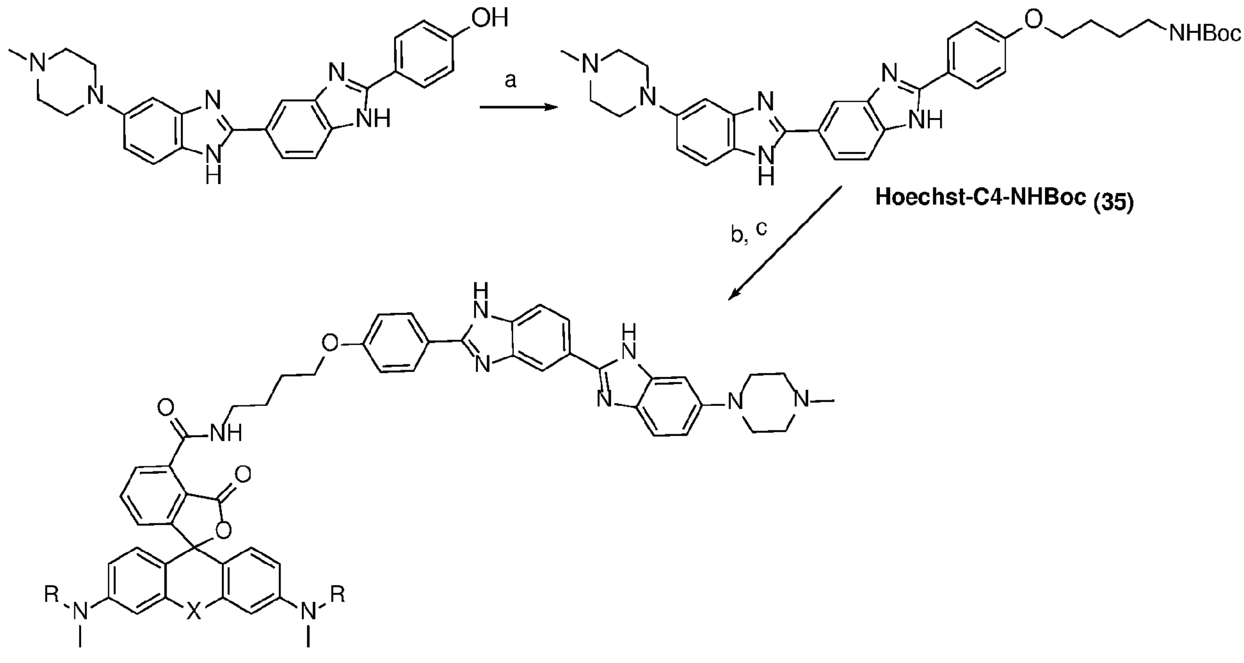


Hoechst-C4-NHBoc (35)

**4-TMR-Hoechst (36)** (R = $-CH_3$; X = O)
**4-580CP-Hoechst (37)** (R = -H; X = $C(CH_3)_2$)

(a) 4-(Bocamino)butyl bromide, $K_2CO_3$, DMF, 60°C, 14h. (b) 4:1 DCM:TFA, rt, 3h. (c) dye **18**, **19**, **20** or **21**, EDCI, DMAP, DMF, rt, 1h.

**B.14 Hoechst-C4-NHBoc (35)**

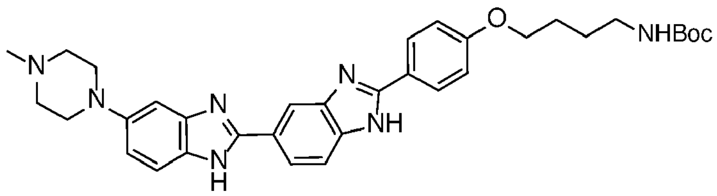

The free base of Hoechst 33258 was prepared by dissolving commercial Hoechst 33258 trihydrochloride (500 mg, 0.936 mmol) in $H_2O$ (30 mL) and adding a solution of $K_2CO_3$ (388 mg, 2.81 mmol, 3eq.) in $H_2O$ (10 mL). The precipitate thus formed was isolated by filtration, washed with $H_2O$ and lyophilised from water 1,4-dioxane mixture. The resulting Hoechst 33258 base (397 mg, 0.936 mmol, 1 eq.) was suspended in dry DMF (1 mL). $K_2CO_3$ (388 mg, 2.81 mmol, 3 eq.) was added followed by 4-(Boc-amino)butyl bromide (283 mg, 1.12 mmol, 1.2 eq.). The reaction was heated at 60 °C for 14 h. The reaction mixture was then cooled to room temperature, DMF was evaporated under reduced pressure. The residue was suspended in DCM and deposited on celite by evaporating the solvent. Product was isolated by flash column chromatography (Büchi Reveleris HP silica 40 g; gradient 20% to 90% $CH_2Cl_2$ - $CH_2Cl_2$:MeOH: $NH_{3(aq)}$ [9:1:0.2]) as yellow solid, yield 306 mg (55%).

$^1$H NMR (600 MHz, $CD_3OD$) δ 8.22 (d, *J* = 1.6 Hz, 1H), 8.01 (d, *J* = 8.8 Hz, 2H), 7.92 (dd, *J* = 8.4, 1.6 Hz, 1H), 7.66 (d, *J* = 8.4 Hz, 1H), 7.50 (d, *J* = 8.7 Hz, 1H), 7.13 (d, *J* = 2.3 Hz, 1H), 7.01 - 7.07 (m, 3H), 4.03 (t, *J* = 6.4 Hz, 2H), 3.23 - 3.33 (m, 4H), 3.11 (t, *J* = 7.0 Hz, 2H), 2.79 - 2.84 (m, 4H), 2.48 (s, 3H), 1.80 (p, *J* = 7.2 Hz, 2H), 1.65 (p, *J* = 7.2 Hz, 2H), δ 1.43 (s, 9H).

$^{13}$C NMR (126 MHz, $CD_3OD$) δ 169.6, 162.4, 158.4, 155.2, 153.7, 149.2, 148.3, 140.4, 136.0, 129.5, 125.5, 122.8, 122.4, 116.5, 116.3, 116.0, 113.8, 102.5, 79.9, 68.9, 56.0, 51.4, 45.6, 41.1, 28.8, 27.7. HRMS (ESI) calcd for $C_{34}H_{42}N_7O_3$ $[M+H]^+$ 596.3344, found 596.3340.

**B.15 4-TMR-Hoechst (36)**

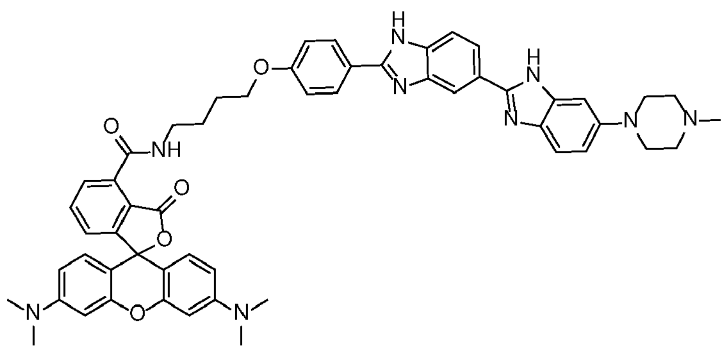

Trifluoroacetic acid (0.2 mL) was added dropwise to a solution of **Hoechst-C4-NHBoc (35)** (16.5 mg, 0.0276 mmol) in $CH_2Cl_2$ (0.8 mL). The resulting intense yellow solution was stirred at room temperature for 3h. The reaction mixture was then evaporated to dryness, the residue was re-evaporated three times with MeOH to remove excess trifluoroacetic acid. The residue was lyophilized from aqueous dioxane. Deprotected product obtained quantatively as trifluoroacetic acid salt ($[Hoechst\text{-}(CH_2)_4NH_3]^{4+}[CF_3COO^-]_4$) as yellow solid. Compound used in further step without additional purification.

**4-TMR-COOH-TFA** salt (10 mg, 0.0184 mmol, 1 eq.), EDCI-HCl (8.4 mg, 0.0276 mmol, 1.5 eq.) DMAP (0.11 mmol, 13.4 mg, 6 eq.) and previously obtained $[Hoechst\text{-}(CH_2)_4NH_3]^{4+}[CF_3COO^-]_4$ (26 mg, 0.0276 mmol, 1.5 eq.) were dissolved in 1 mL of dry DMF and stirred for 1 hour. Then DMF was removed at rt under reduced pressure. The product was purified by preparative HPLC (preparative column: Eurospher 100 C18, 5 μm, 250 × 20 mm; solvent A: acetonitrile, solvent B: H2O + 0.2% v/v HCOOH; temperature 25 °C, gradient A:B - 5 min 20:80 isocratic, 5-30 min 20:80 to 70:30 gradient). Water acetonitrile mixture was removed by rotary evaporator and product was purified one more time by flash chromatography (silica-gel cartridge: Interchim Puriflash 12g, 15 μm column, gradient 20% to 80% $CH_2Cl_2$ - $CH_2Cl_2$:MeOH: $NH_{3(aq)}$ [85:15:2]). Solvents were removed and a product was lyophilised from water acetonitrile mixture to obtain 9 mg (54%) of purple solid.

$^1$H NMR (400 MHz, $CD_3OD+CF_3COOD$) δ 8.58 (dd, J = 1.7, 0.7 Hz, 1H), 8.25 (dd, J = 8.7, 1.7 Hz, 1H), 8.18 (d, J = 9.0

Hz, 2H), 8.07 (dd, J = 8.7, 0.7 Hz, 1H), 7.83 - 7.79 (m, 2H), 7.76 (d, J = 9.1 Hz, 1H), 7.51 (dd, J = 6.2, 2.7 Hz, 1H), 7.44 (dd, J = 9.2, 2.2 Hz, 1H), 7.36 (d, J = 2.2 Hz, 1H), 7.29 (d, J = 9.0 Hz, 2H), 7.23 (d, J = 9.5 Hz, 2H), 7.07 (dd, J = 9.5, 2.5 Hz, 2H), 6.97 (d, J = 2.4 Hz, 2H), 4.22 (t, J = 6.2 Hz, 2H), 3.97 (d, J = 13.2 Hz, 2H), 3.68 (d, J = 12.1 Hz, 2H), 3.49 (t, J = 6.9 Hz, 2H), 3.36 (d, J = 11.3 Hz, 2H), 3.30 (s, 12H), 3.19 (d, J = 11.9 Hz, 2H), 3.00 (s, 3H), 2.03 - 1.94 (m, 2H), 1.87 (dd, J = 8.7, 6.0 Hz, 2H).

$^{13}$C NMR (101 MHz, $CD_3OD+CF_3COOD$) δ 171.1, 169.2, 165.6, 159.1, 158.8, 154.3, 151.1, 149.5, 139.2, 134.6, 133.9, 133.7, 132.5, 131.9, 131.5, 130.2, 128.0, 126.3, 121.7, 120.9, 120.0, 118.1, 117.5, 117.2, 116.4, 115.7, 115.5, 115.2, 115.0, 114.6, 112.4, 101.0, 97.5, 69.5, 54.6, 48.4 (visible in HSQC), 43.6, 41.0, 40.7, 27.5, 26.9.

ESI-MS, positive mode: m/z = 908.5 $[M+H]^+$.

HRMS (ESI) calcd for $C_{54}H_{54}N_9O_5$ $[M+H]^+$ 908.4242, found 908.4230.

**B.16 4-580CP-Hoechst (37)**

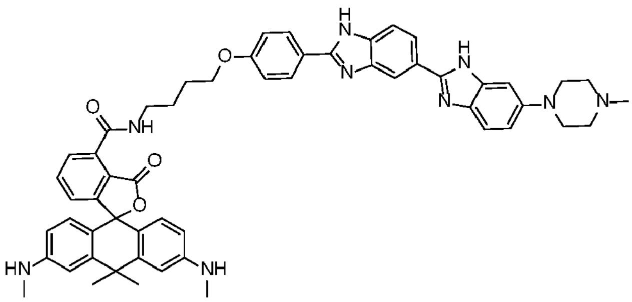

Trifluoroacetic acid (0.2 mL) was added dropwise to a solution of **Hoechst-C4-NHBoc (35)** (16.5 mg, 0.0276 mmol) in $CH_2Cl_2$ (0.8 mL). The resulting intense yellow solution was stirred at room temperature for 3h. The reaction mixture was then evaporated to dryness, the residue was re-evaporated three times with MeOH to remove excess trifluoroacetic acid. The residue was lyophilized from aqueous dioxane.

Deprotected product obtained quantatively as trifluoroacetic acid salt ($[Hoechst\text{-}(CH_2)_4NH_3]^{4+}[CF_3COO^-]_4$) as yellow solid. Compound used in further step without additional purification.

**4-580CP-COOH-**TFA salt (0.0184 mmol, 10 mg, 1 eq.), EDCl-HCl (0.0276 mmol, 8.4mg, 1.5 eq.) DMAP (0.11 mmol, 13.4 mg, 6 eq.) and $[Hoechst\text{-}(CH_2)_4NH_3]^{4+}[CF_3COO^-]_4$ (0.0276, 26 mg, 1.5 eq.) were dissolved in 1 mL of dry DMF and stirred for 1 hour. Then DMF was removed at rt under reduced pressure. The product was purified by preparative HPLC (preparative column: Eurospher 100 C18, 5 μm, 250 × 20 mm; solvent A: acetonitrile, solvent B: $H_2O$ + 0.2% v/v HCOOH; temperature 25 °C, gradient A:B - 5 min 20:80 isocratic, 5-30 min 20:80 to 70:30 gradient). Water acetonitrile mixture was removed by rotary evaporator and product was purified one more time by flash chromatography (silica-gel cartridge: Interchim Puriflash 12g, 15μm column, gradient 20% to 80% $CH_2Cl_2$- $CH_2Cl_2$:MeOH: $NH_{3(aq)}$ [85:15:2]). Solvents were removed and a product was lyophilised from water acetonitrile mixture to obtain 6 mg (36%) of dark-violet solid.

$^1$H NMR (400 MHz, $CD_3OD+CF_3COOD$) δ 8.39 (s, 1H), 8.17 - 8.04 (m, 3H), 7.95 (d, J = 8.6 Hz, 1H), 7.86 - 7.78 (m, 2H), 7.76 (d, J = 8.9 Hz, 1H), 7.48 - 7.39 (m, 2H), 7.36 (s, 1H), 7.21 - 7.12 (m, 4H), 7.09 (d, J = 9.1 Hz, 2H), 6.68 (dd, J = 9.1, 2.3 Hz, 2H), 4.13 (t, J = 5.7 Hz, 2H), 3.98 (d, J = 13.1 Hz, 2H), 3.77 - 3.71 (m, 2H), 3.52 (t, J = 6.0 Hz, 2H), 3.35 (d, J = 10.6 Hz, 2H), 3.27 (d, J = 13.3 Hz, 2H), 3.07 (s, 6H), 3.03 (s, 3H), 1.99 - 1.85 (m, 4H), 1.83 (s, 3H), 1.70 (s, 3H).

$^{13}$C NMR (101 MHz, $CD_3OD+CF_3COOD$) δ 169.7, 168.6, 162.7, 161.7, 161.4, 157.3, 156.7, 153.7, 149.2, 148.7, 137.4, 136.8, 136.7, 133.2, 132.0, 131.1, 130.3, 129.2, 128.1, 126.9, 124.7, 123.2, 120.7, 118.6, 118.1, 115.2, 115.2, 114.3, 113.7, 112.7, 111.0, 99.7, 67.8, 53.2, 47.1, 42.3, 41.1, 39.4, 34.2, 30.8, 28.9, 26.2, 25.5.

ESI-MS, positive mode: m/z = 906.4 $[M+H]^+$.

HRMS (ESI) calcd for $C_{55}H_{56}N_9O_4$ $[M+H]^+$ 906.4450, found 906.4455.

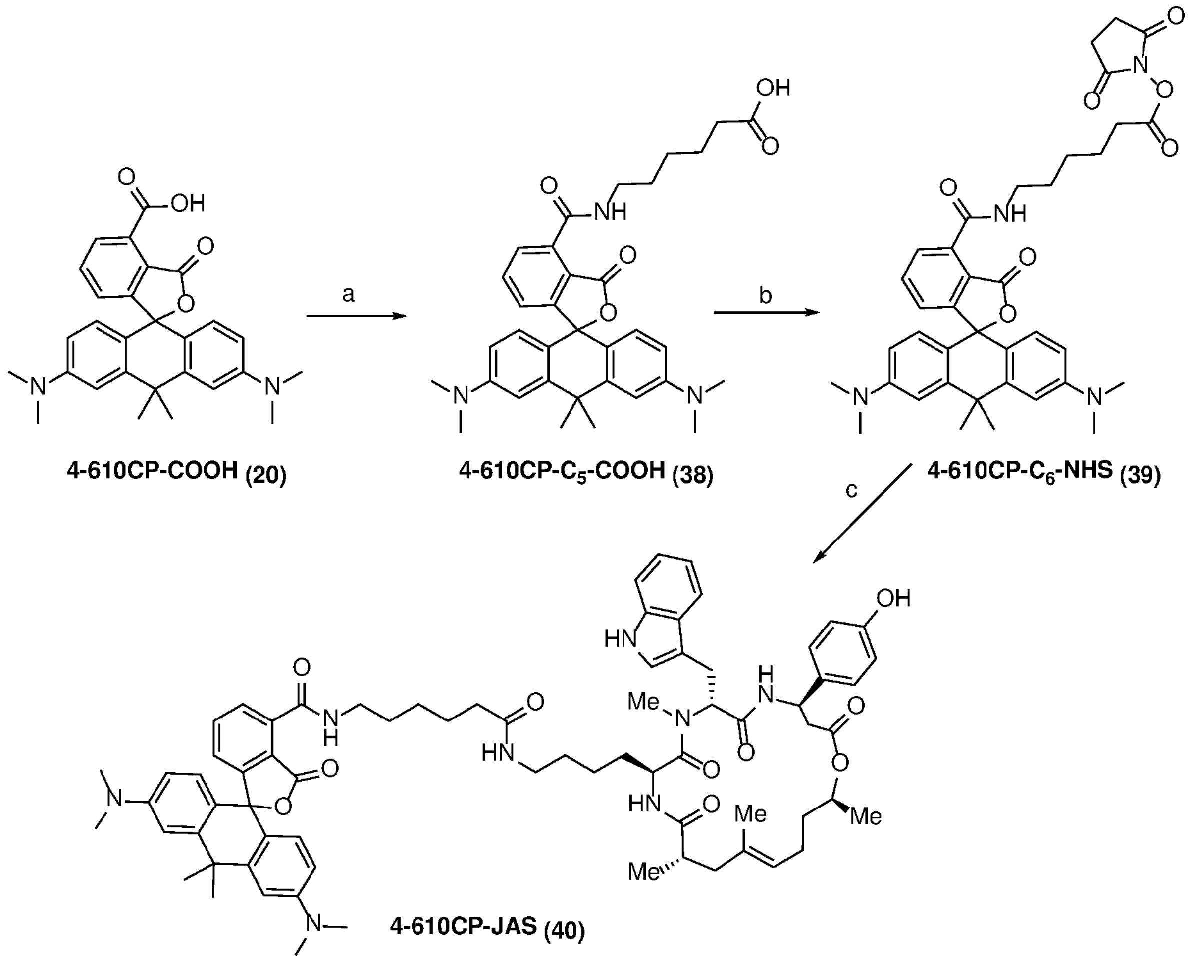


4-610CP-COOH (20) 4-610CP-$C_5$-COOH (38) 4-610CP-$C_6$-NHS (39)

4-610CP-JAS (40)

(a) 1) HBTU, DIPEA, DMSO, rt. 15 min, 2) 6-aminocaproic acid, DMSO:$H_2O$ 1:1 (b) TSTU, DIPEA, MeCN, 2h rt. (c) des-bromo-des-methyl-Lys-jasplakinolide, DIPEA, MeCN, rt, 1h.

**B.17 4-610CP-$C_5$-COOH (38)**

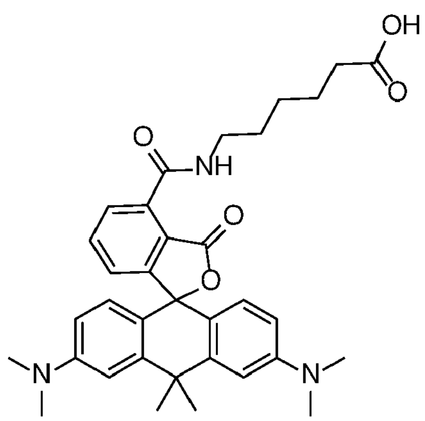

**4-610CP-COOH (20)** TFA salt (0.0175 mmol, 10 mg, 1 eq.), DIPEA (15 μL, 0.0875 mmol, 5 eq.) and HBTU (0.021 mmol, 8.0 mg, 1.2 eq.) were dissolved in 400 μL of dry DMSO and stirred at room temperature for 15 min. A solution of 6-aminocaproic acid (0.0175 mmol, 2.3 mg, 1 eq.) in 200 μL of DMSO:H2O mixture (1:1) was added to the

reaction mixture and stirring continued for 1 hour. Reaction mixture was neutralised with formic acid and product was purified by preparative HPLC (preparative column: Eurospher 100 C18, 5 μm, 250 × 20 mm; solvent A: acetonitrile, solvent B: H2O + 0.2% v/v HCOOH; temperature 25 °C, gradient A:B - 5 min 40:60 isocratic, 5-30 min 40:60 to 80:20 gradient). Product lyophilised from acetonitrile: water mixture to obtain 7 mg (70%) of blue powder.

$^1$H NMR (400 MHz, $CD_3CN$) δ 9.74 (t, J = 5.1 Hz, 1H), 8.28 (dd, J = 7.7, 1.0 Hz, 1H), 7.72 (t, J = 7.7 Hz, 1H), 7.08 (dd, J = 7.7, 1.0 Hz, 1H), 6.96 (d, J = 2.3 Hz, 2H), 6.64 - 6.53 (m, 4H), 3.49 (q, J = 7.0 Hz, 2H), 2.97 (s, 13H), 2.31 (t, J = 7.4 Hz, 2H), 2.20 (s, 1H), 1.86 (s, 3H), 1.75 (s, 3H), 1.73 - 1.61 (m, 4H), 1.56 - 1.44 (m, 2H).

$^{13}$C NMR (101 MHz, $CD_3CN$) δ 175.08, 172.55, 164.64, 158.41, 152.15, 147.76, 136.10, 134.66, 132.21, 129.55, 127.07, 123.70, 119.05, 112.78, 110.31, 40.67, 40.59, 39.27, 35.31, 34.14, 33.58, 29.72, 27.25, 25.32.

ESI-MS, positive mode: m/z = 570.3 $[M+H]^+$.

HRMS (ESI) calcd for $C_{34}H_{40}N_3O_5$ $[M+H]^+$ 570.2962, found 570.2965.

**B.18 4-610CP-$C_6$-NHS (39)**

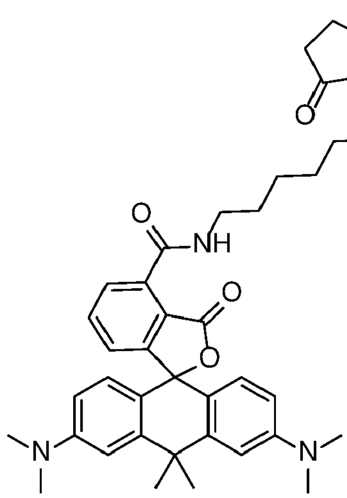

**4-610CP-$C_5$-COOH (37)** (0.021 mmol, 12.0 mg), TSTU (0.0274 mmol, 8.1 mg) and DIPEA (36 μL, 0.2064 mmol) were dissolved in 500 μL of MeCN and stirred for 1 hour. Then MeCN was removed by rotary evaporator and obtained product was purified by flash chromatography (silica-gel cartridge: Interchim Puriflash 12g, 15μm column, gradient 20% to 100% DCM - EtOAc). The solvents were removed and the obtained NHS ester was redissolved in 1,4-dioxane, microfiltered through a 0.45 μm PTFE membrane filter and lyophilized to obtain 10 mg (71%) of blue powder.

$^1$H NMR (400 MHz, Acetonitrile-$d_3$) δ 9.71 (s, 1H), 8.28 (dd, *J* = 7.7, 1.0 Hz, 1H), 7.74 (t, *J* = 7.7 Hz, 1H), 7.09 (dd, *J* = 7.7, 1.0 Hz, 1H), 6.97 (d, *J* = 2.3 Hz, 2H), 6.71 - 6.54 (m, 4H), 3.51 (td, *J* = 6.9, 5.4 Hz, 2H), 2.99 (s, 12H), 2.75 (s, 4H), 2.67 (t, *J* = 7.4 Hz, 2H), 1.87 (s, 3H), 1.85 - 1.77 (m, 2H), 1.76 (s, 3H), 1.76 - 1.68 (m, 2H), 1.63 - 1.54 (m, 2H).

ESI-MS, positive mode: m/z = 667.2 $[M+H]^+$.

**B.19 4-610CP-JAS (40)**

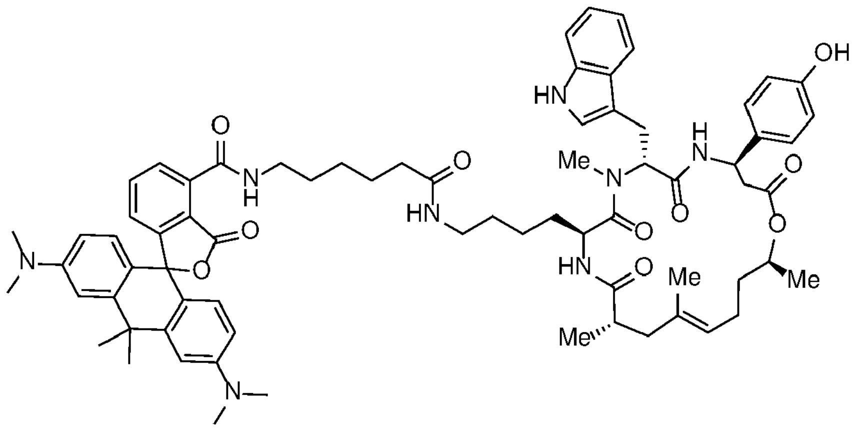

The **4-610CP-$C_6$-NHS (39)** (2.7 mg, 0.0041 mmol) ester was dissolved in 400 μL of MeCN followed by the addition of deprotected des-bromo-des-methyl-Lys-jasplakinolide (0.00342 mmol, 2.3 mg) (Tannert, R., Milroy, L. G., Ellinger, B., Hu, T. S., Arndt, H. D., Waldmann, H., J Am Chem Soc, 2010, 132 (9), 3063-3077) and DIPEA (12 μL, 0.0688 mmol). The reaction mixture was stirred for 1 hour and purified by preparative HPLC (preparative column: Eurospher 100 C18, 5 μm, 250 × 20 mm; solvent A: acetonitrile, solvent B: H2O + 0.2% v/v HCOOH; temperature 25 °C, gradient A:B - 5 min 30:70 isocratic, 5-30 min 30:70 to 100:0 gradient). Product lyophilised from acetonitrile: water mixture to obtain 1.8 mg (48%) of light blue powder.

$^1H$ NMR (400 MHz,$d_6$-dmso) δ 10.79 (d, *J* = 1.8 Hz, 1H), 9.27 (s, 1H), 9.11 (t, *J* = 5.5 Hz, 1H), 8.61 (d, *J* = 8.8 Hz, 1H), 7.75 (d, *J* = 7.6 Hz, 1H), 7.70 - 7.60 (m, 4H), 7.26 (d, *J* = 8.1 Hz, 1H), 7.10 (d, *J* = 8.6 Hz, 2H), 7.03 (d, *J* = 2.3 Hz, 1H), 7.02 - 6.97 (m, 2H), 6.92 (t, *J* = 7.2 Hz, 1H), 6.88 (d, *J* = 2.1 Hz, 2H), 6.67 (d, *J* = 8.5 Hz, 2H), 6.58 - 6.50 (m, 4H), 5.49 (dd, *J* = 11.3, 5.1 Hz, 1H), 5.22 - 5.11 (m, 1H), 4.89 (t, *J* = 7.1 Hz, 1H), 4.64 (h, *J* = 6.4 Hz, 1H), 4.58 - 4.44 (m, 1H), 3.35 - 3.31 (m, 2H), 3.08 - 2.96 (m, 4H), 2.91 (s, 12H), 2.89 - 2.71 (m, 3H), 2.65 (dd, *J* = 14.7, 11.3 Hz, 1H), 2.55 (dd, *J* = 14.7, 3.2 Hz, 1H), 2.53 - 2.48 (m, 2H), 2.14 (dd, *J* = 14.6, 11.5 Hz, 1H), 2.05 (t, *J* = 7.5 Hz, 2H), 1.86 - 1.75 (m, 5H), 1.74 - 1.65 (m, 4H), 1.61 - 1.49 (m, 4H), 1.45 (s, 3H), 1.38 - 1.33 (m, 2H), 1.22 - 1.20 (m, 2H), 1.13 (d, *J* = 6.3 Hz, 3H), 1.10 - 1.00 (m, 2H), 0.90 (d, *J* = 6.8 Hz, 3H), 0.85 - 0.71 (m, 4H).

ESI-MS, positive mode: m/z = 1247.6 $[M+Na]^+$.

HRMS (ESI) calcd for $C_{72}H_{89}N_8O_{10}$ $[M+H]^+$ 1225.6696, found 1225.6689.

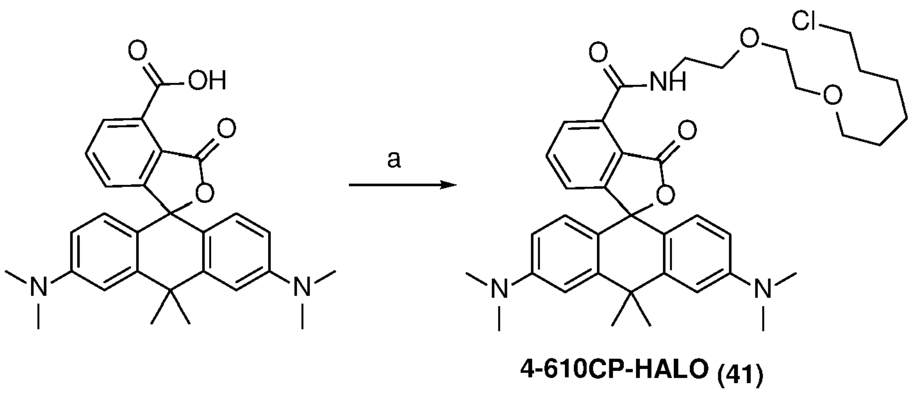


**4-610CP-HALO (41)**

(a) HBTU, DIPEA, $NH_2-(CH_2)_2-O(CH_2)_2-O-(CH_2)_6-Cl$, MeCN, rt, 2h

**B.20 4-610CP-HALO (40)**

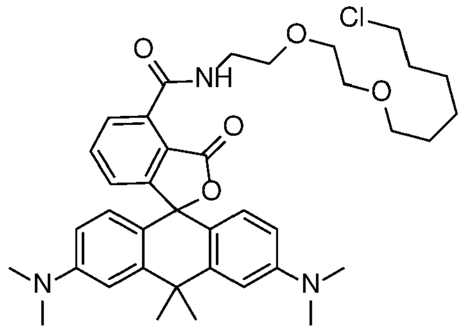

**4-610CP-COOH (20)**-TFA salt (10 mg, 0.0175 mmol, 1 eq.), DIPEA (15 μL, 0.0875 mmol, 5 eq.) and HBTU (8.0 mg, 0.021 mmol, 1.2 eq.) were dissolved in 400 μL of dry MeCN and stirred at room temperature for 5 min.

A solution of $NH_2$-$(CH_2)_2$-O-$(CH_2)_2$-O-$(CH_2)_6$-Cl (6 mg, 0.0267 mmol, 1.53 eq.) in 100 μL of dry MeCN was added to the reaction mixture and stirring continued for 1 hour. Reaction mixture was neutralised with formic acid and product was purified by preparative HPLC (preparative column: Eurospher 100 C18, 5 μm, 250 × 20 mm; solvent A: acetonitrile, solvent B: H2O + 0.2% v/v HCOOH; temperature 25 °C, gradient A:B - 5 min 40:60 isocratic, 5-30 min 30:70 to 100:0 gradient). Product was purified one more time by flash chromatography (silica-gel cartridge: Interchim Puriflash 12g, 15μm column, gradient 20% to 80% $CH_2Cl_2$- $CH_2Cl_2$:MeOH [90:10]). Solvents were removed and a product was lyophilised from acetonitrile: water mixture to obtain 7 mg (60%) of blue-violet powder.

$^1$H NMR (400 MHz, $d_6$-DMSO) δ 9.34 (t, *J* = 5.5 Hz, 1H), 7.83 (dd, *J* = 7.5, 1.0 Hz, 1H), 7.70 (t, *J* = 7.6 Hz, 1H), 7.06 (dd, *J* = 7.7, 1.0 Hz, 1H), 6.91 (d, *J* = 2.2 Hz, 2H), 6.63 - 6.52 (m, 4H), 3.63 (t, *J* = 5.9 Hz, 2H), 3.60 - 3.49 (m, 8H), 3.38 (t, *J* = 6.5 Hz, 2H), 2.94 (s, 12H), 1.82 (s, 3H), 1.72 (s, 3H), 1.71 - 1.61 (m, 2H), 1.47 (q, *J* = 6.9 Hz, 2H), 1.37 - 1.26 (m, 4H).

$^{13}$C NMR (126 MHz, $d_6$-DMSO) δ 169.4, 164.7, 156.5, 150.6, 146.0, 135.0, 134.5, 129.3, 128.4, 125.2, 121.9, 118.1, 111.7, 109.1, 86.6, 70.2, 69.7, 69.5, 68.9, 45.4, 39.9, 39.8, 37.9, 34.6, 33.5, 32.0, 29.1, 26.1, 24.9.

ESI-MS, positive mode: m/z = 662.3 $[M+H]^+$.

EXAMPLE 1

*Characterization of rhodamine 4'-isomer dyes and 4'-isomer based fluorescent probes*

The neighboring group effect (NGE) as described above could be detected by NMR or HPLC methods (Fig. 1): Rhodamine 4'-isomers containing NGE show significant NMR chemical shift of the interacting atoms which are not bound via covalent chemical bond and increased HPLC retention times. The NGE is most profound after attachment of the linker or a ligand by conversion of the carboxylic group to carboxamide.

Fig. 1 shows the occurrence of neighboring group effect in the 4'-isomers of rhodamines. **a,** Neighboring group effect shifts spirolactone-zwitterion equilibrium of rhodamines. X = O, S, NRs, $CR_5R_6$, $SO_2$, P(O)OH, P(O)$OR_5$, $SiR_5R_6$, $GeR_5R_6$, $R_n$ = alkyl. **b**, Chemical shift differences of amide proton of TMR-LTX regioisomeric probes. **c**, Comparison of retention times of **TMR-LTX** regioisomeric probes in HPLC analysis with SB-C18 column and isocratic elution conditions (75:25 MeOH : $H_2O$ 25mM $HCOONH_4$ pH = 3.6).

The ability of the fluorophores to switch between spirolactone and zwitterion states can be characterized by measuring the absorbance in water-1,4-dioxane mixtures with known dielectric constant (Åkerlöf G, Short AO.. J Am Chem Soc 1936, 58(7): 1241-1243). $D_{50}$ is commonly used as numeric value for the evaluation of the equilibrium changes and represents a dielectric constant at which absorbance is halved.

Fig. 2 a) shows graphs representing the absorbance of **TMR-LTX** positional isomers at $\lambda_{max}$ versus dielectric constant (D) of 1,4-dioxane-water mixtures and Fig.2 b) shows ${}^{Dye}O_{50}$values of positional isomers of **TMR-COOH** and ${}^{probe}D_{50}$ of **TMR-LTX.**

As evident from Fig. 2, for 5'- and 6'-isomers, the ${}^{probe}D_{50}$ values of conjugates are only marginally higher than ${}^{Dye}D_{50}$ of corresponding fluorophores. In contrast to this, ${}^{Probe}D_{50}$ values of 4'-isomers conjugates are increased compared to the corresponding ${}^{Dye}D_{50}$ values of the fluorophore, and significantly higher than conjugates ${}^{probe}D_{50}$ values of 5'- and 6'-isomers. In addition, NMR shift value of -CONH- proton of the probe is significantly increased (> 0.3 ppm) in 4'-isomers compared to the corresponding values of 5'- and 6'-isomers. This indicates that the conversion of carboxyl group to amide modulates NGE further and induces higher spirolactone content at equilibrium and thus implies higher hydrophobicity of 4'-isomer conjugates. Most importantly, it demonstrates the major difference between 5'/6'-isomers and 4'-isomer of rhodamine class dyes.

The following Table 1 shows comparative $D_{50}$ values for different positional isomers of selected rhodamine probes.

| | 1,4-Dioxane-Water with constant 0.3%SDS additive | | | |
|---|---|---|---|---|
| Probe | **TMR-LTX** | **580CP-LTX** | **610CP-CTX** | **SiR-CTX** |
| | $D_{50}$ | $D_{50}$ | $D_{50}$ | $D_{50}$ |
| 4'-isomer | 26.5 ± 0.5 | 62 ± 1 | 65 ± 2 | >80 |
| 5'-isomer | 13.0 ± 0.4 | 40.0 ± 0.6 | 39.7 ± 0.2 | 66.8 ± 0.3 |
| 6'-isomer | 11.2 ± 0.5 | 36.0 ± 0.7 | 36.4 ± 0.4 | 66.5 ± 0.4 |

The following Table 2 shows comparative chemical shift of amide NH proton in DMSO-*d6* of isomeric dye-C8-taxane conjugates

| Probe | Conjugate isomer | | |
|---|---|---|---|
| | 4'-isomer | 5'-isomer | 6'-isomer |
| TMR-LTX | 9.07 | 8.76 | 8.62 |
| 580CP-LTX | 9.14 | 8.72 | 8.69 |
| 610CP-CTX | 9.09 | 8.73 | 8.66 |
| SiR-CTX | 8.99 | 8.75 | 8.69 |
| Average | 9.07 ± 0.06 | 8.74 ± 0.01 | 8.67 ± 0.03 |

As demonstrated in further experiments and Fig. 3, this neighboring group effect results in dramatically increased cell membrane permeability while keeping all photophysical properties almost unchanged. As an example, the inventors prepared the larotaxel derivative (**4-TMR-LTX**) for labelling of tubulin in living cells (Fig. 3a). **4-TMR-LTX** demonstrates excellent membrane permeability compared to widely used 5'- and 6'-isomers and permits the highly specific tubulin labelling in mammalian cells by simply incubating cells with **4-TMR-LTX** (Fig. 3b,c). To prove the increased membrane permeability, the inventors relied on the property of the targeting moiety, larotaxel, to perturb cell cycle. **4-TMR-LTX** induce accumulation of subG1 phase cells almost as efficiently as parent compound. In contrast, the 5'- and 6'-isomers showed much higher $EC_{50}$ (Figure 6d). This demonstrates the superior permeability, labeling specificity and biocompatibility of the 4'-isomer. Importantly, the use of the observed NGE enhanced cell membrane permeability could be extended to other rhodamine like fluorophores - carbopyronines (580CP and 610CP) 4'-isomers derivatives (Figure 3b-d).

As mentioned above, Fig. 3. illustrates the tubulin labelling of mammalian cells: a, structures of the tubulin probes. b, Wide-field fluorescence microscopy of living primary fibroblasts stained 100 nM TMR-LTX isomers for 1h at 37°C. Cells were washed once with HBSS and imaged in growth DMEM media. Inserts shows zoomed-in images. Scale bars: large field of view - 100 μm, insert - 10 μm. Hoechst staining is shown in cyan and all tubulin probes were shown in magenta. c, Quantification of fluorescence signal in the cytoplasm of living cells stained with tubulin probes. Data were presented as mean ± s.e.m., N = 3 independent experiments, each time n > 100 cells were quantified. d, Cytotoxicity of tubulin fluorescent probes presented as half maximal effective concentration (EC50) after 24h incubation at 37°C in growth media. Cytotoxicity was determined as fraction of cells population containing less then single set of genetic material (sub G1 DNA content). Data were presented as mean ± s.e.m., N = 3 independent experiments, each time n > 100 cells were quantified.

Furthermore, said increased cell permeability and favourable optical properties are also observed with multiple targeting moieties, as demonstrated with ligands targeting actin, DNA and Halo-tag (Figure 4 and 5). This demonstrates that rhodamine 4'-isomers represent a unique platform for the development of biocompatible fluorescent probes, in which the rhodamine core structure is derivatized with: (i) ligands that specifically bind to other biomolecules in vitro or in vivo, (ii) reactive groups such as activated esters or (iii) molecules that can control the fluorescence properties of the fluorescent dyes, like quenchers.

Fig. 4 shows the performance of DNA and actin fluorescent probes based on rhodamines' positional isomers: a, Structure of DNA probes showing attachment point positional isomerism. **b**, Wide-field microscopy images of living primary fibroblasts were stained with 100 nM 4/5/6-580CP-Hoechst (magenta) for 1h at 37°C overlaid with of the light transmission (grey). Cells washed once with HBSS and imaged in growth DMEM media. Inserts show zoomed-in images. Scale bars: large field of view 100 μm, inserts - 10 μm. **c**, Quantification of DNA probes' fluorescence signal in the nuclei. Data presented as mean ± s.d., N = 3 independent experiments, each n > 100 cells. **d**, Structure of actin probes

attachment point positional isomerism. **e,** Wide-field microscopy overlay images of the light transmission (grey) and fluorescence channels: Hoechst - blue, 610CP - yellow. Living primary fibroblasts stained with 100 nM 4/5/6-610CP-JAS for 1h at 37°C. Cells were washed once with HBSS and imaged in growth DMEM media. Inserts show zoomed-in images. Scale bars: large field of view 100 μm, inserts - 10 μm. **f**, Quantification of 4/5/6-610CP-JAS fluorescence signal in the cytoplasm of living cells. Data were presented as mean ± s.d., N = 3 independent experiments, each n > 100 cells.

Fig. 5 shows the SDS-PAGE in-gel fluorescence of 100 nM Halo-tag purified protein labeled with 100 nM 4-610CP-Halo substrate for the indicated periods of time at 37°C.

EXAMPLE 2

*STED optical microscopy of living cells*

The novel fluorescent probes were applied for STED nanoscopy imaging in living cells. For these experiments, STED optical microscopy was performed as indicated above.

The inventors obtained an unprecedented quality of images at resolution below diffraction limit. A combination of TMR and 610CP was successfully applied for two-color imaging of living cells.

Fig. 6 shows STED nanoscopy imaging of living cells stained with rhodamine 4'-isomer probes: a, Confocal and STED microscopy images of microtubules in living human fibroblasts stained with 100 nM **4-610CP-CTX** for 1h at 37°C. Scale bar 1 μm. **b,** Confocal and STED microscopy images of microtubules in living human fibroblasts stained with 100 nM **4-SiR-CTX** for 1h at 37°C. Scale bar 1 μm. **c**, Deconvolved STED image of human female primary fibroblast nucleus stained with 100 nM **4-580CP-Hoechst** showing the inactivated X chromosome (Xi). Insets - confocal and STED microscopy zoomed-in images of Xi region. Scale bars: 500 nm - insets, 1 μm - main image. **d,** Two-colour STED no-wash image of primary human fibroblasts stained with 100 nM **4-610CP-JAS** and 10 nM **4-TMR-LTX** for 1h at 37°C. Scale bar 10 nm.

**Claims**

**1.** A fluorescent dye which is a rhodamine 4'-isomer having the following general structural formula **A:**

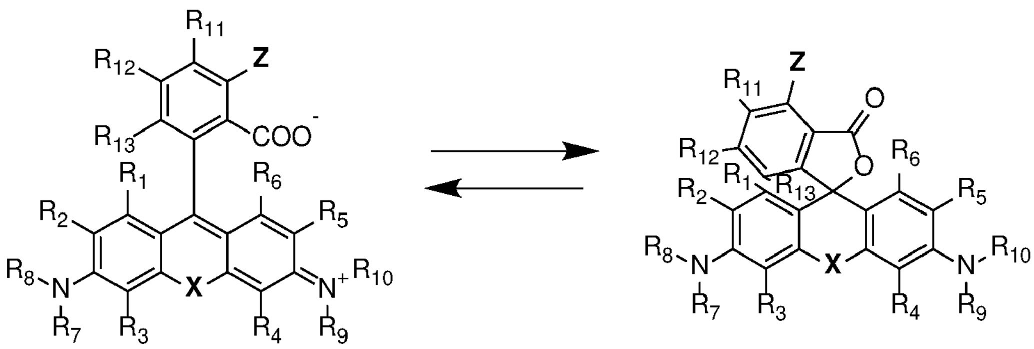

Zwitterion form　　　　Spirolactone form

**Aa**　　　　**Ab**

wherein

$R_1$, $R_2$, $R_3$, $R_4$ $R_5$ $R_6$ $R_7$ $R_8$ $R_9$ $R_{10}$ $R_{11}$ $R_{12}$ $R_{13}$ are independently selected from H, halogen, D, CN, OH, O(alkyl), O(aryl), SH, S(alkyl), S(aryl), S(O)(alkyl), S(O)(aryl), $S(O)_2$(alkyl), $S(O)_2$(aryl), $N_3$, $NH_2$, NH(alkyl), $N(alkyl)_2$, NH(aryl), $NH(aryl)_2$, $NO_2$, CHO, C(O)(alkyl), C(O)(aryl), COOH, COO(alkyl) COO(aryl), C(O)NH(alkyl), C(O)NH(aryl), $C(O)N(alkyl)_2$, $C(O)N(aryl)_2$, P(O)OH(alkyl), P(O)OH(aryl), $P(O)(\text{-O-alkyl})_2$, $P(O)(\text{-O-aryl})_2$, $PO_3H_2$, $SO_3H$, alkyl, bulky alkyl, substituted alkyl, alkenyl, substituted alkenyl, substituted bulky alkenyl;
$R_2$ and $R_8$ or $R_7$ and $R_8$ taken together may form a cyclic structure;
$R_5$ and $R_9$ or $R_9$ and $R_{10}$ taken together may form a cyclic structure;
$R_3$ and $R_7$ or $R_4$ and $R_9$ taken together may form a cyclic structure;
$R_1$ and $R_2$ or $R_5$ and $R_6$ taken together may form a cyclic structure;
X is selected from $CR_{14}R_{15}$, a heteroatom, in particular O, S, $NR_{14}$, $SO_2$, P(O)OH, $P(O)OR_{14}$, $SiR_{14}R_{15}$,

$GeR_{14}R_{15}$, where $R_{14}$ and $R_{15}$ are alkyl or aryl;

Z is selected from O(alkyl), O(aryl), S(alkyl), S(aryl), S(O)(alkyl), S(O)(aryl), $S(O)_2$(alkyl), $S(O)_2$(aryl), $S(O)_2$(-O-alkyl), $S(O)_2$(-O-aryl), $S(O)_2$NH(alkyl), $S(O)_2$NH(aryl), $S(O)_2N(alkyl)_2$, $S(O)_2N(aryl)_2$, $S(O)_2$N(alkyl)(aryl), NH(alkyl), $N(alkyl)_2$, N(alkyl)(aryl), NH(aryl), $N(aryl)_2$, C(O)O(alkyl), C(O)O(aryl), C(O)(alkyl), C(O)(aryl), P(O)OH(-NH-alkyl), P(O)OH(-O-alkyl), P(O)OH(-NH-aryl), P(O)OH(-O-aryl), $P(O)(-O-alkyl)_2$, $P(O)(-NH-alkyl)_2$, $P(O)OH(-N(alkyl)_2)$, $P(O)OH(-N(aryl)_2)$, $P(O)(-N(aryl)_2)_2$, $P(O)(-N(alkyl)_2)(-N(alkyl)_2)$, $P(O)(-O-aryl)_2$, $P(O)(-NH-aryl)_2$, P(O)(-O-alkyl)(-O-aryl), P(O)(-NH-alkyl)(-O-aryl), P(O)(-O-alkyl)(-NH-aryl), P(O)(-NH-alkyl)(-NH-aryl), in particularly -C(O)OH, -C(O)NH(alkyl), C(O)NH(aryl), $CON(alkyl)_2$, or any group which induces a neighboring group effect via steric, ionic or bonding interactions with the adjacent carboxyl group involved in spirolactone formation resulting in a shift of the equilibrium between zwitterionic form and spirolactone form towards the spirolactone form as indicated by an increased $D_{50}$ value of the 4'-isomer compared to the $D_{50}$ value of a reference isomer, in particular a 5'- or 6'-isomer, and preferably an absolute $D_{50}$ value of the 4'-isomer which is a numeric value of at least 13, wherein said $D_{50}$ value represents the dielectric constant at which the absorbance of a dye sample in a mixture of 1,4-dioxane and water is halved.

2. The fluorescent dye according to claim 1, having one of the following structural formulae **B** - **E**

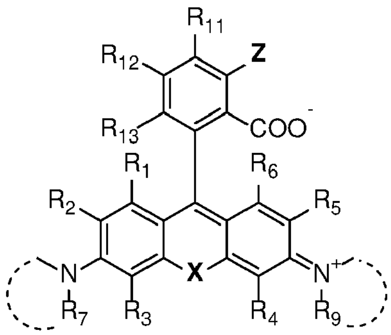

**B**

wherein the cyclic structure formed by $R_7$ and $R_8$ and/or by $R_9$ and $R_{10}$ taken together represents a non-aromatic heterocycle, in particular selected from azetidine, pyrrolidine, piperidine, morpholine, azepane or azecane;

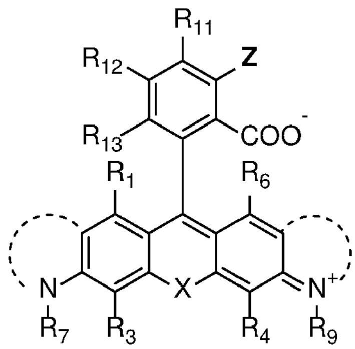

**C**

wherein the cyclic structure formed by $R_2$ and $R_8$ and/or by $R_5$ and $R_{10}$ taken together represents an aromatic or non-aromatic 5 or 6 atom membered heterocyclic structure, in particular selected from azetidine, pyrrolidine, piperidine, azepane or azecane;

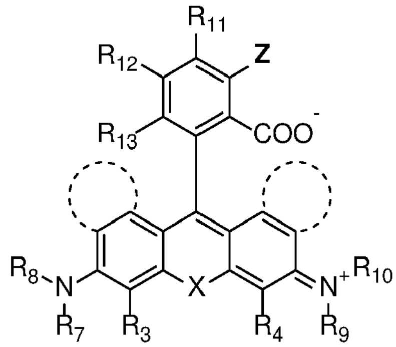

**D**

wherein the cyclic structure formed by $R_1$ and $R_2$ and/or by $R_5$ and $R_6$ taken together represents an aromatic or non-aromatic 5 or 6 atom membered heterocyclic structure, in particular selected from azetidine, pyrrolidine, piperidine, azepane or azecane;

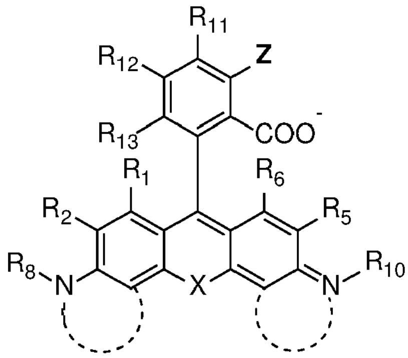

**E**

wherein the cyclic structure formed by $R_3$ and $R_7$ and/or by $R_4$ and $R_9$ taken together represents an aromatic or non-aromatic 5 or 6 atom membered heterocyclic structure, in particular selected from azetidine, pyrrolidine, piperidine, azepane or azecane.

**3.** The fluorescent dye according to claim 1, having one of the following structural formulae

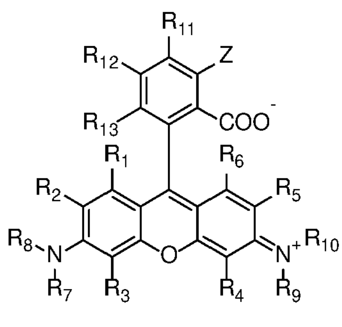

**F**

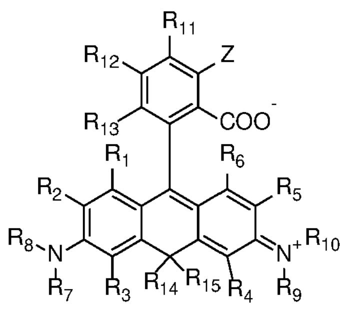

**G**

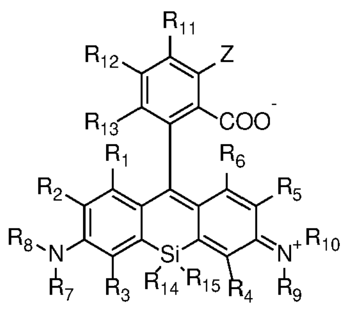

**H**

wherein

$R_1$, $R_2$, $R_3$, $R_4$ $R_5$ $R_6$ $R_7$ $R_8$ $R_9$ $R_{10}$ $R_{11}$ $R_{12}$ $R_{13}$ are independently selected from H, halogen, D, CN, OH, O(alkyl), O(aryl), SH, S(alkyl), S(aryl), S(O)(alkyl), S(O)(aryl), $S(O)_2$(alkyl), $S(O)_2$(aryl), $N_3$, $NH_2$, NH(alkyl), $N(alkyl)_2$, NH(aryl), $NH(aryl)_2$, $NO_2$, CHO, C(O)(alkyl), C(O)(aryl), COOH, COO(alkyl) COO(aryl), C(O)NH(alkyl), C(O)NH(aryl), $C(O)N(alkyl)_2$, $C(O)N(aryl)_2$, P(O)OH(alkyl), P(O)OH(aryl), $P(O)(-O-alkyl)_2$, $P(O)(-O-aryl)_2$,

$PO_3H_2$, $SO_3H$, alkyl, bulky alkyl, substituted alkyl, alkenyl, substituted alkenyl, substituted bulky alkenyl;
$R_2$ and $R_8$ or $R_7$ and $R_8$ taken together may form a cyclic structure;
$R_5$ and $R_{10}$ or $R_9$ and $R_{10}$ taken together may form a cyclic structure;
$R_3$ and $R_7$ or $R_4$ and $R_9$ taken together may form a cyclic structure;
$R_1$ and $R_2$ or $R_5$ and $R_6$ taken together may form a cyclic structure;
Z is selected from O(alkyl), O(aryl), S(alkyl), S(aryl), S(O)(alkyl), S(O)(aryl), $S(O)_2$(alkyl), $S(O)_2$(aryl), $S(O)_2$(-O-alkyl), $S(O)_2$(-O-aryl), $S(O)_2$NH(alkyl), $S(O)_2$NH(aryl), $S(O)_2N(alkyl)_2$, $S(O)_2N(aryl)_2$, $S(O)_2$N(alkyl)(aryl), NH(alkyl), $N(alkyl)_2$, N(alkyl)(aryl), NH(aryl), $N(aryl)_2$, C(O)O(alkyl), C(O)O(aryl), C(O)(alkyl), C(O)(aryl), P(O)OH(-NH-alkyl), P(O)OH(-O-alkyl), P(O)OH(-NH-aryl), P(O)OH(-O-aryl), $P(O)(-O\text{-}alkyl)_2$, $P(O)(-NH\text{-}alkyl)_2$, $P(O)OH(-N(alkyl)_2)$, $P(O)OH(-N(aryl)_2)$, $P(O)(-N(aryl)_2)_2$, $P(O)(-N(alkyl)_2)(-N(alkyl)_2)$, $P(O)(-O\text{-}aryl)_2$, $P(O)(-NH\text{-}aryl)_2$, P(O)(-O-alkyl)(-O-aryl), P(O)(-NH-alkyl)(-O-aryl), P(O)(-O-alkyl)(-NH-aryl), P(O)(-NH-alkyl)(-NH-aryl), in particularly -C(O)OH, -C(O)NH(alkyl), C(O)NH(aryl), $CON(alkyl)_2$, or any group which induces a neighboring group effect as defined in claim 1.

**4.** The fluorescent dye according to any one of claims 1 to 3, having one of the following structural formulae:

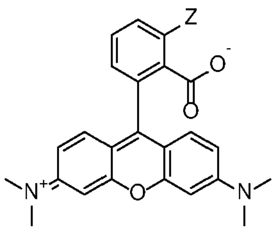
**4-TMR**

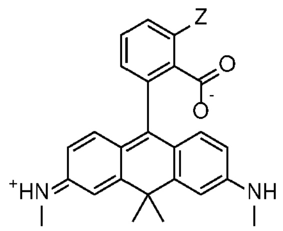
**4-580CP**

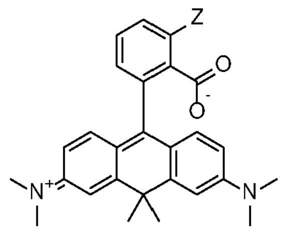
**4-610CP**

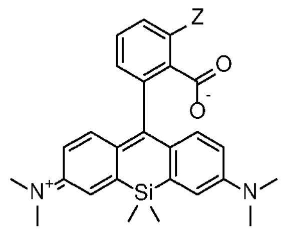
**4-SiR**

wherein
Z is selected from O(alkyl), O(aryl), S(alkyl), S(aryl), S(O)(alkyl), S(O)(aryl), $S(O)_2$(alkyl), $S(O)_2$(aryl), $S(O)_2$(-O-alkyl), $S(O)_2$(-O-aryl), $S(O)_2$NH(alkyl), $S(O)_2$NH(aryl), $S(O)_2N(alkyl)_2$, $S(O)_2N(aryl)_2$, $S(O)_2$N(alkyl)(aryl), NH(alkyl), $N(alkyl)_2$, N(alkyl)(aryl), NH(aryl), $N(aryl)_2$, C(O)O(alkyl), C(O)O(aryl), C(O)(alkyl), C(O)(aryl), P(O)OH(-NH-alkyl), P(O)OH(-O-alkyl), P(O)OH(-NH-aryl), P(O)OH(-O-aryl), $P(O)(-O\text{-}alkyl)_2$, $P(O)(-NH\text{-}alkyl)_2$, $P(O)OH(-N(alkyl)_2)$, $P(O)OH(-N(aryl)_2)$, $P(O)(-N(aryl)_2)_2$, $P(O)(-N(alkyl)_2)(-N(alkyl)_2)$, $P(O)(-O\text{-}aryl)_2$, $P(O)(-NH\text{-}aryl)_2$, P(O)(-O-alkyl)(-O-aryl), P(O)(-NH-alkyl)(-O-aryl), P(O)(-O-alkyl)(-NH-aryl), P(O)(-NH-alkyl)(-NH-aryl), in particularly -C(O)OH, -C(O)NH(alkyl), C(O)NH(aryl), $CON(alkyl)_2$, or any group which induces a neighboring group effect as defined in claim 1.

**5.** A rhodamine 4'-isomer derivative comprising a rhodamine 4'-isomer having one of the general structural formulae A-H described above, wherein Z is selected from O(alkyl), O(aryl), S(alkyl), S(aryl), S(O)(alkyl), S(O)(aryl), $S(O)_2$(alkyl), $S(O)_2$(aryl), $S(O)_2$(-O-alkyl), $S(O)_2$(-O-aryl), $S(O)_2$NH(alkyl), $S(O)_2$NH(aryl), $S(O)_2N(alkyl)_2$, $S(O)_2N(aryl)_2$, $S(O)_2$N(alkyl)(aryl), NH(alkyl), $N(alkyl)_2$, N(alkyl)(aryl), NH(aryl), $N(aryl)_2$, C(O)O(alkyl), C(O)O(aryl), C(O)(alkyl), C(O)(aryl), P(O)OH(-NH-alkyl), P(O)OH(-O-alkyl), P(O)OH(-NH-aryl), P(O)OH(-O-aryl), $P(O)(-O\text{-}alkyl)_2$, $P(O)(-NH\text{-}alkyl)_2$, $P(O)OH(-N(alkyl)_2)$, $P(O)OH(-N(aryl)_2)$, $P(O)(-N(aryl)_2)_2$, $P(O)(-N(alkyl)_2)(-N(alkyl)_2)$, $P(O)(-O\text{-}aryl)_2$, $P(O)(-NH\text{-}aryl)_2$, P(O)(-O-alkyl)(-O-aryl), P(O)(-NH-alkyl)(-O-aryl), P(O)(-O-alkyl)(-NH-aryl), P(O)(-NH-alkyl)(-NH-aryl), in particularly -C(O)OH, -C(O)NH(alkyl), C(O)NH(aryl), $CON(alkyl)_2$, or any group which induces a neighboring group effect via steric, ionic or bonding interactions as indicated by an increased $D_{50}$ value of the 4'-isomer derivative compared to the $D_{50}$ value of a reference isomer derivative, in particular a 5'- or 6'-isomer derivative, and preferably an absolute $D_{50}$ value of the 4'-isomer derivative which is a numeric value of at least 15 and which is higher than that of the corresponding 5'- or 6'-isomer derivative by a difference of least 10, wherein said $D_{50}$ value represents the dielectric constant at which the absorbance of a dye sample in a mixture of 1,4-dioxane and water is halved, coupled to at least one reactive group or ligand which is capable to interact with or bind to other molecules, wherein said reactive group or ligand may be coupled to the rhodamine 4'-isomer fluorophore either directly or via a linker.

**6.** The rhodamine 4'-isomer derivative according to claim 5, wherein the linker comprises or consists of a straight or branched alkyl chain with 1- 21 C atoms which may be substituted by one or more functional groups and may include heteroatoms and/or aromatic groups, and preferably represents a moiety selected from the following group:

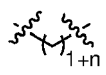 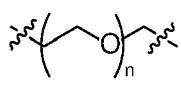 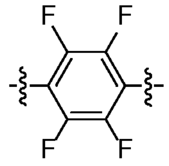 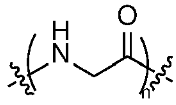 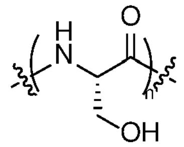

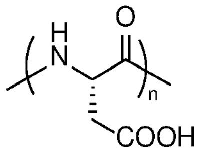 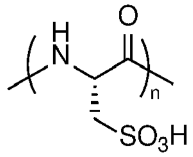 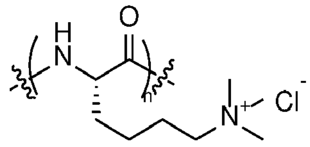 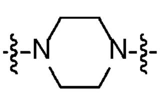

with n = an integer of 1-20.

**7.** The rhodamine 4'-isomer derivative according to claim 5 or 6, wherein the reactive group is selected from the group comprising an activated ester, an amine, a thiol, an azide, an ethyne, a maleimide, a tetrazine, N- hydroxysuccinimide or an alcohol group, or wherein the ligand is a ligand which binds specifically to a protein, peptide, nucleotide or nucleic acid, carbohydrate, iodoacetamide or which is capable to effect or participate in chelation of $NH_4^+$ or metal ions, in particular $Li^+$, $Na^+$, $K^+$, $Cs^+$, $Rb^+$, $Cu^+$, $Tl^+$, $Hg^+$, $Ag^+$, $Au^+$, $Ca^{2+}$, $Ba^{2+}$, $Sr^{2+}$, $Be^{2+}$, $Zn^{2+}$, $Ni^{2+}$, $Cu^{2+}$, $Mg^{2+}$, $Co^{2+}$, $Fe^{2+}$, $Mn^{2+}$, $Pt^{2+}$, $Cd^{2+}$, $Hg^{2+}$, $Sn^{2+}$, $Pb^{2+}$, $Au^{3+}Cr^{3+}$, $Co^{3+}$, $Fe^{3+}$, $Al^{3+}$, $Ga^{3+}$, $In^{3+}$,$SC^{3+}$, $Ti^{3+}$, $Tl^{3+}$,$V^{3+}$,$Y^{3+}$, $La^{3+}$, $Pt^{4+}$, $Pb^{4+}$, $Ce^{4+}$, $Ge^{4+}$, $Th^{4+}$,$Zr^{4+}$,$U^{4+}$.

**8.** The rhodamine 4'-isomer derivative according to any one of claims 5-7, wherein the reactive group or ligand is coupled directly or via a linker to group Z.

**9.** The rhodamine 4'-isomer derivative according claim 8, wherein the reactive group or ligand is coupled to group Z by an amide bond formed between a carboxyl functional group or amine functional group provided by Z and an amine functional group or carboxyl functional group provided by the linker or ligand, or wherein the ligand is selected from the group consisting of benzylguanine, benzylcytosine, a primary alkyl chloride and trimethoprim.

**10.** A conjugate comprising a rhodamine 4'-isomer according to any one of claims 1 to 4 or rhodamine 4'-isomer derivative according to any one of claims 5 or 9 coupled to or associated with a molecule which is selected from the group comprising a peptide, protein, in particular an enzyme or antibody, tubulin or actin, a nucleotide, nucleic acid sequence, including DNA and RNA, a lipid, carbohydrate, an organic or inorganic polyphosphate, a pharmaceutical drug, a metabolite, a reactive oxygen species (ROS), $NH_4^+$, a metal ion, in particular $Li^+$, $Na^+$, $K^+$, $Cs^+$, $Rb^+$, $Cu^+$, $Tl^+$, $Hg^+$, $Ag^+$, $Au^+$, $Ca^{2+}$, $Ba^{2+}$, $Sr^{2+}$, $Be^{2+}$, $Zn^{2+}$, $Ni^{2+}$, $Cu^{2+}$, $Mg^{2+}$, $Co^{2+}$, $Fe^{2+}$, $Mn^{2+}$, $Pt^{2+}$, $Cd^{2+}$, $Hg^{2+}$, $Sn^{2+}$, $Pb^{2+}$, $Au^{3+}$ $Cr^{3+}$, $Co^{3+}$, $Fe^{3+}$, $Al^{3+}$, $Ga^{3+}$, $In^{3+}$,$Sc^{3+}$, $Ti^{3+}$, $Tl^{3+}$,$V^{3+}$,$Y^{3+}$, $La^{3+}$, $Pt^{4+}$, $Pb^{4+}$, $Ce^{4+}$, $Ge^{4+}$, $Th^{4+}$,$Zr^{4+}$,$U^{4+}$, or complex thereof.

**11.** Use of the compounds according to any one of claims 1-9 for conjugation with an analyte or molecule of interest, in particular selected from the group comprising a peptide, protein, in particular an enzyme or antibody, tubulin or actin, a nucleotide, nucleic acid sequence, including DNA and RNA, a lipid, carbohydrate, an organic or inorganic polyphosphate, a pharmaceutical drug, a toxin, a metabolite, a reactive oxygen species (ROS), $NH_4^+$, a metal ion, in particular, $Li^+$, $Na^+$, $K^+$, $Cs^+$, $Rb^+$, $Cu^+$, $Tl^+$, $Hg^+$, $Ag^+$, $Au^+$, $Ca^{2+}$, $Ba^{2+}$, $Sr^{2+}$, $Be^{2+}$,$Zn^{2+}$, $Ni^{2+}$, $Cu^{2+}$, $Mg^{2+}$, $Co^{2+}$, $Fe^{2+}$, $Mn^{2+}$, $Pt^{2+}$, $Cd^{2+}$, $Hg^{2+}$, $Sn^{2+}$, $Pb^{2+}$,$Au^{3+}$, $Cr^{3+}$, $Co^{3+}$, $Fe^{3+}$, $Al^{3+}$, $Ga^{3+}$, $In^{3+}$,$Sc^{3+}$, $Ti^{3+}$, $Tl^{3+}$,$V^{3+}$,$Y^{3+}$, $La^{3+}$, $Pt^{4+}$, $Pb^{4+}$, $Ce^{4+}$, $Ge^{4+}$, $Th^{4+}$,$Zr^{4+}$,$U^{4+}$, or complex thereof.

**12.** The use according to claim 11, wherein the conjugation comprises formation of at least one covalent chemical bond or at least one molecular complex with a chemical entity or substance, such as amine, carboxylic acid, aldehyde, alcohol, aromatic compound, heterocycle, dye, amino acid, amino acid residue coupled to any chemical entity, peptide, protein, carbohydrate, nucleic acid, toxin and lipid.

**13.** The use according to claim 11, wherein the ligand of the rhodamine 4'-isomer derivative which is capable to interact with or bind to other molecules is benzylguanine and the protein is SNAP-tag, or the ligand is benzylcytosine and the proteine is CLIP-tag, or the ligand is a primary alkyl chloride and the protein is Halo-tag, or the ligand is trimethoprim and the protein is dihydrofolate reductase.

**14.** The use of the compounds according to any one of claims 1-9 or of their conjugates according to claim 10 as cell permeable substances penetrating through membranes of living and fixed cells in vivo or in vitro.

**15.** Use of the compounds according to any one of claims 1-9 or of their conjugates according to claim 10 as such or after photoactivation for tracking and monitoring dynamic processes in a sample or in an object.

**16.** The use according to claim 15, wherein a rhodamine 4'-isomer derivative coupled to tubulin, DNA, RNA, lipid, or actin is used for cell cycle monitoring in living cells or living tissues in vivo or in vitro.

**17.** Use of rhodamine 4'-isomers derivatives according to claims 5-9 that have high singlet oxygen ($^1O_2$) quantum yield, preferably at least 5 %, for chromophore-assisted light inactivation (CALI) and photodynamic therapy (PDT) in living cells and in living organisms including humans, mammals, birds, fish, hemichordates, molluscs, tunicates, cnidarians, cephalochordates, flatworms, nematodes, annelids, tardigrades, reptiles, arthropods, echinoderms, chaetognathas, rotifers, frogs, plants, sponges or fungi.

**18.** Use of the compounds according to any one of claims 1-9 or of their conjugates according to claim 10 as labels in microscopic, spectroscopic and other imaging techniques, in particular fluorescence microscopy and spectroscopy, in microfluidic devices, capillary electrophoresis, fluorescence activated cell sorting, DNA sequencing, sequence-specific genome labeling, analyte tracking techniques in vitro or in vivo.

**19.** The use according to claim 18, wherein the analyte tracking techniques comprise at least one of the following sequences of steps:

- interacting with or binding of the rhodamine 4'-isomer derivative to the analyte, which is a metal ion, resulting in metal ion chelation and in a corresponding change in fluorescence properties of the rhodamine 4'-isomer derivative, and using this change in fluorescence properties for metal ion imaging in vitro or in vivo, in particular in living cells and in living organisms including humans, mammals, birds, fish, hemichordates, molluscs, tunicates, cnidarians, cephalochordates, flatworms, nematodes, annelids, tardigrades, reptiles, arthropods, echinoderms, chaetognathas, rotifers, frogs, plants, sponges or fungi;
- reacting of the rhodamine 4'-isomer derivative with reactive oxygen species (ROS) resulting in a corresponding change in fluorescence properties of the rhodamine 4'-isomer derivative, and using this change in fluorescence properties for ROS imaging in vitro or in vivo, in particular in living cells and in living organisms such as humans, mammals, birds, fish, hemichordates, molluscs, tunicates, cnidarians, cephalochordates, flatworms, nematodes, annelids, tardigrades, reptiles, arthropods, echinoderms, chaetognathas, rotifers, frogs, plants, sponges or fungi;
- selectively interacting or reacting of the rhodamine 4'-isomer derivative with an enzyme, resulting in a corresponding change in fluorescence properties of the rhodamine 4'-isomer derivative, and using this change in fluorescence properties for detection, quantification and imaging of enzymatic activity in vitro and in vivo;
- selectively interacting or reacting of the rhodamine 4'-isomer derivative with a lipid, organic and inorganic polyphosphate, protein, carbohydrate, metabolites, DNA or RNA, resulting in a corresponding change in fluorescence properties, and using this change in fluorescence properties for lipid, polyphosphate, protein, carbohydrate, metabolite, DNA or RNA imaging in vitro or in vivo;
- interacting or reacting of rhodamine 4'-isomers derivatives coupled to any drug or drug candidate with a target molecule or target site in a cell or tissue, resulting in a corresponding change in fluorescence properties, and using this change in fluorescence properties for drug-target interaction monitoring using fluorescence imaging or NMR in vitro or in vivo;
- interacting or reacting of a rhodamine 4'-isomer derivative with oxygen, fluoride or glucose through non-covalent complex or covalent bond formation, resulting in a corresponding change in fluorescence properties of the rhodamine 4'-isomer derivative, and using this change in fluorescence properties for oxygen, fluoride or glucose sensing in vitro or in vivo, in particular in living cells and in living organisms, including humans, mammals, birds, fish, hemichordates, molluscs, tunicates, cnidarians, cephalochordates, flatworms, nematodes, annelids, tardigrades, reptiles, arthropods, echinoderms, chaetognathas, rotifers, frogs, plants, sponges or fungi.

**20.** The use according to claim 18, wherein the imaging techniques comprise stimulated emission depletion microscopy [STED], single molecule spectroscopy, single molecule switching (SMS) "nanoscopy" (diffraction unlimited optical resolution by using switching of the fluorescence of the single molecules, such as single molecule localization microscopy [SMLM], structured illumination microscopy (SIM), light-sheet microscopy, photoactivation localization microscopy [PALM, PALMIRA, fPALM], stochastic optical reconstruction microscopy [STORM]), fluorescence correlation spectroscopy [FCS] or fluorescence anisotropy spectroscopy, fluorescence recovery after photobleaching

[FRAP], fluorescence lifetime imaging [FLIM], ground state depletion with individual molecular return [GSDIM], and fluorescence resonant energy transfer [FRET], correlative fluorescence - electron microscopy, correlative fluorescence - cryo-electron microscopy, microscale thermophoresis, fluorescence in situ hybridization (FISH), nuclear magnetic resonance spectroscopy.

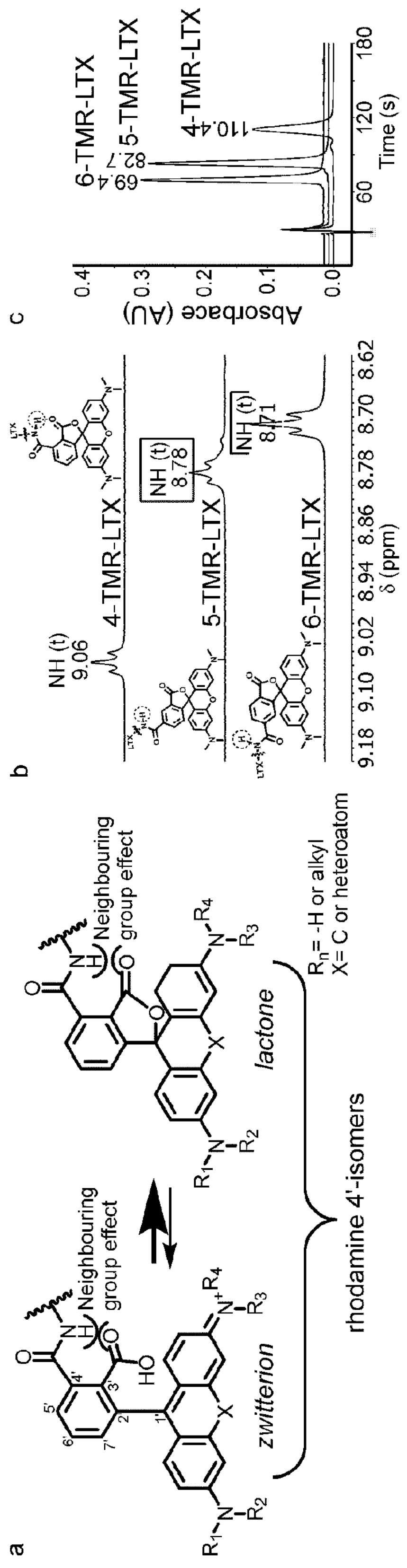
a
Neighbouring group effect
zwitterion
lactone
rhodamine 4'-isomers
b
NH (t) 9.06
4-TMR-LTX
NH (t) 8.78
5-TMR-LTX
NH (t) 8.71
6-TMR-LTX
9.18 9.10 9.02 8.94 8.86 8.78 8.70 8.62
δ (ppm)
c
6-TMR-LTX
5-TMR-LTX
4-TMR-LTX
69.4
82.7
110.4
Absorbace (AU)
0.0 0.1 0.2 0.3 0.4
60 120 180
Time (s)

FIG. 1

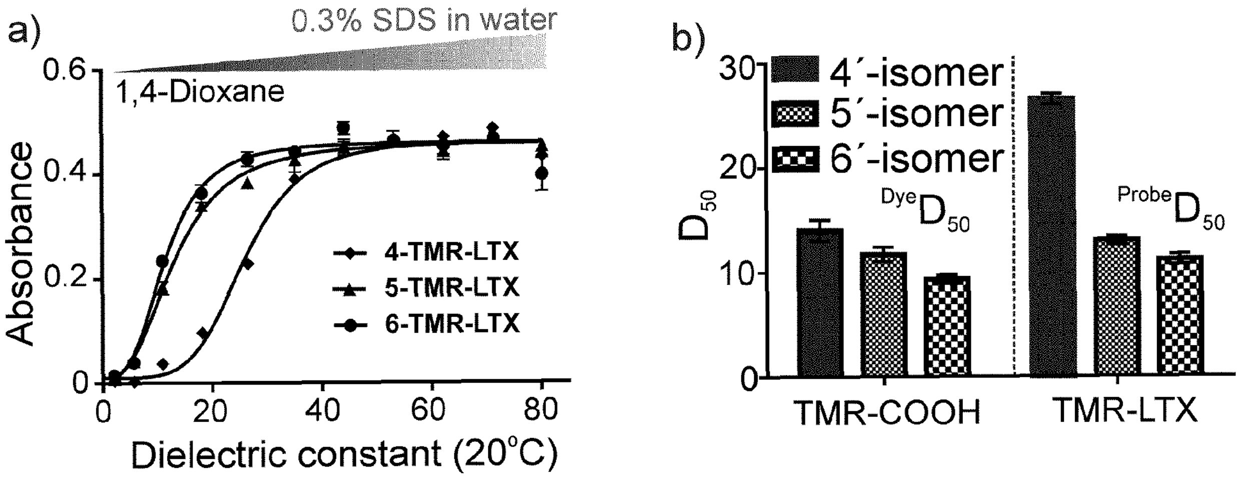
a)
0.3% SDS in water
1,4-Dioxane
0.6
0.4
0.2
0
Absorbance
4-TMR-LTX
5-TMR-LTX
6-TMR-LTX
0
20
40
60
80
Dielectric constant (20°C)
b)
30
20
10
0
$D_{50}$
4´-isomer
5´-isomer
6´-isomer
$^{Dye}D_{50}$
$^{Probe}D_{50}$
TMR-COOH
TMR-LTX

FIG. 2

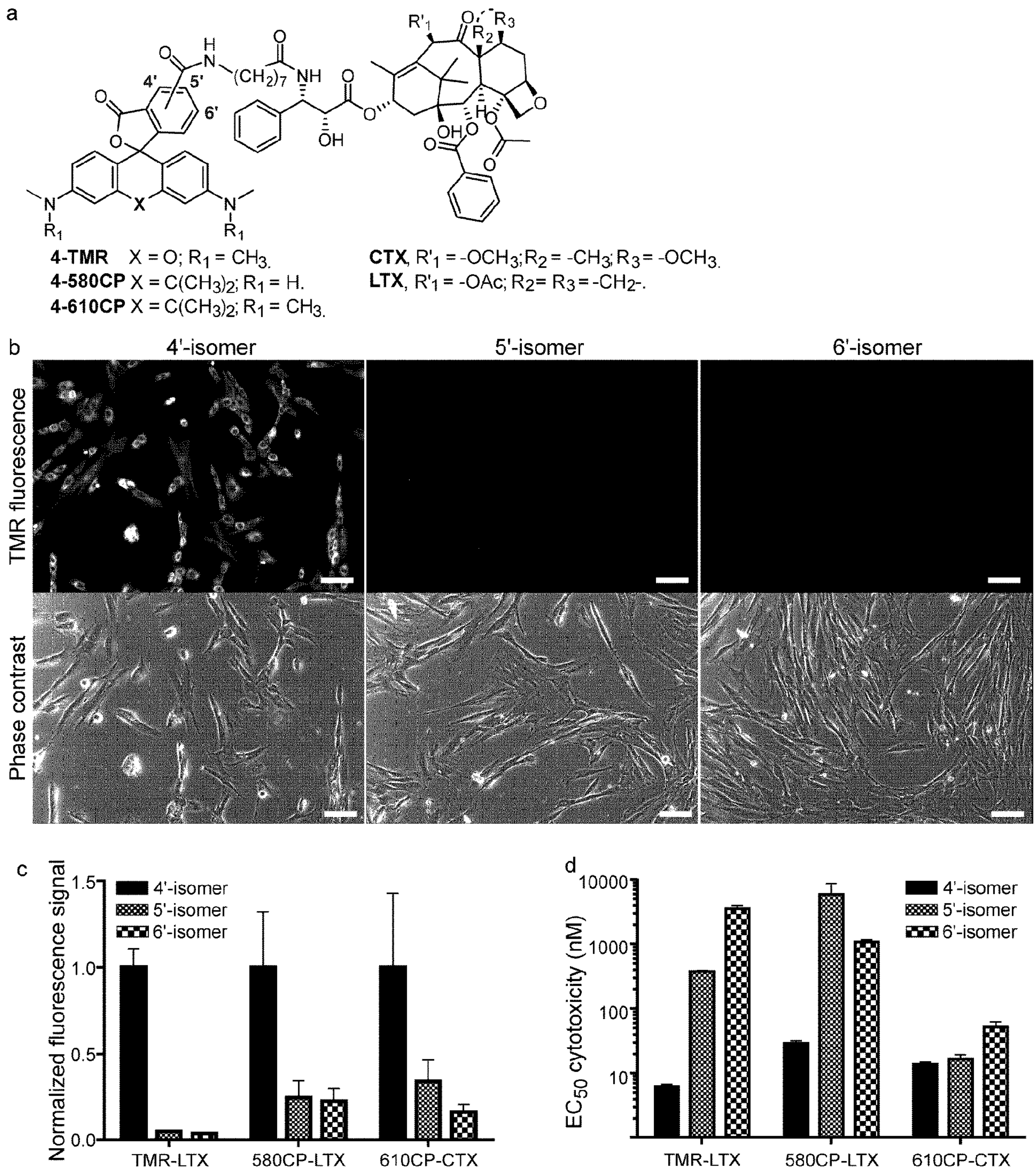
a
4-TMR X = O; $R_1$ = $CH_3$.
4-580CP X = $C(CH_3)_2$; $R_1$ = H.
4-610CP X = $C(CH_3)_2$; $R_1$ = $CH_3$.
CTX, $R'_1$ = $-OCH_3$; $R_2$ = $-CH_3$; $R_3$ = $-OCH_3$.
LTX, $R'_1$ = -OAc; $R_2$ = $R_3$ = $-CH_2-$.
b
4'-isomer
5'-isomer
6'-isomer
TMR fluorescence
Phase contrast
c
Normalized fluorescence signal
1.5
1.0
0.5
0.0
4'-isomer
5'-isomer
6'-isomer
TMR-LTX
580CP-LTX
610CP-CTX
d
$EC_{50}$ cytotoxicity (nM)
10000
1000
100
10
4'-isomer
5'-isomer
6'-isomer
TMR-LTX
580CP-LTX
610CP-CTX

FIG. 3

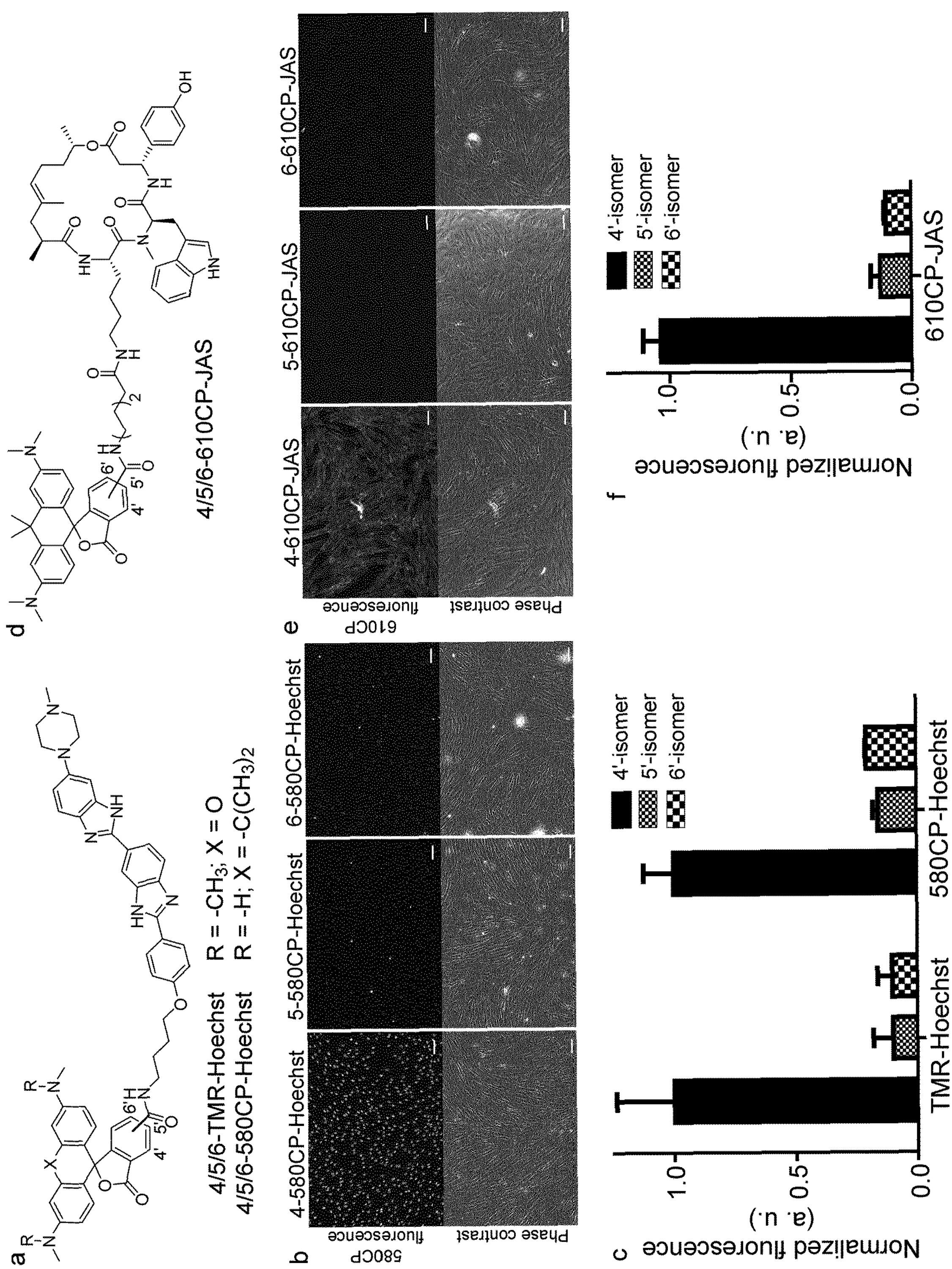
a
4/5/6-TMR-Hoechst R = -CH$_3$; X = O
4/5/6-580CP-Hoechst R = -H; X = -C(CH$_3$)$_2$
d
4/5/6-610CP-JAS
b
580CP fluorescence
Phase contrast
4-580CP-Hoechst
5-580CP-Hoechst
6-580CP-Hoechst
e
610CP fluorescence
Phase contrast
4-610CP-JAS
5-610CP-JAS
6-610CP-JAS
c
Normalized fluorescence (a. u.)
1.0
0.5
0.0
TMR-Hoechst
580CP-Hoechst
4'-isomer
5'-isomer
6'-isomer
f
Normalized fluorescence (a. u.)
1.0
0.5
0.0
610CP-JAS
4'-isomer
5'-isomer
6'-isomer

FIG. 4

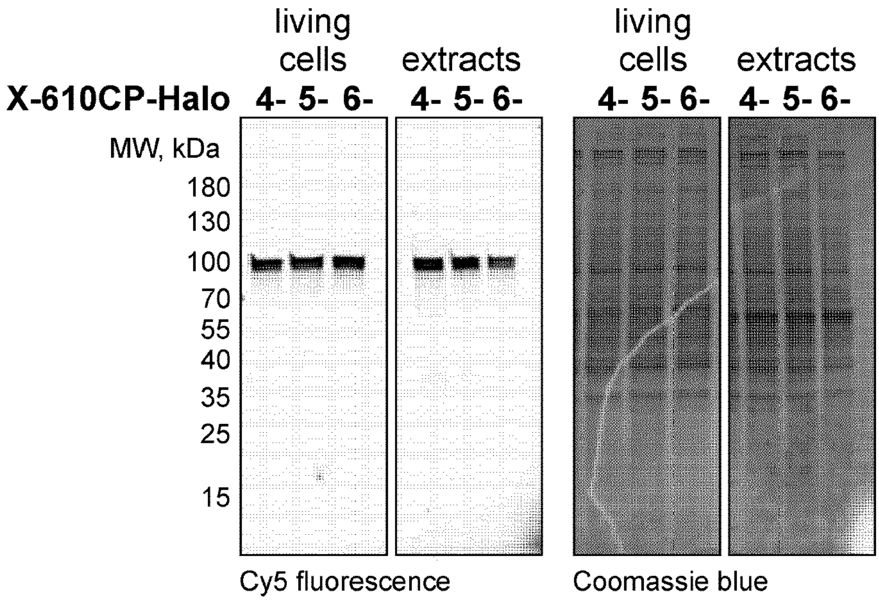
living cells
extracts
living cells
extracts
X-610CP-Halo
4- 5- 6-
4- 5- 6-
4- 5- 6-
4- 5- 6-
MW, kDa
180
130
100
70
55
40
35
25
15
Cy5 fluorescence
Coomassie blue

Fig. 5

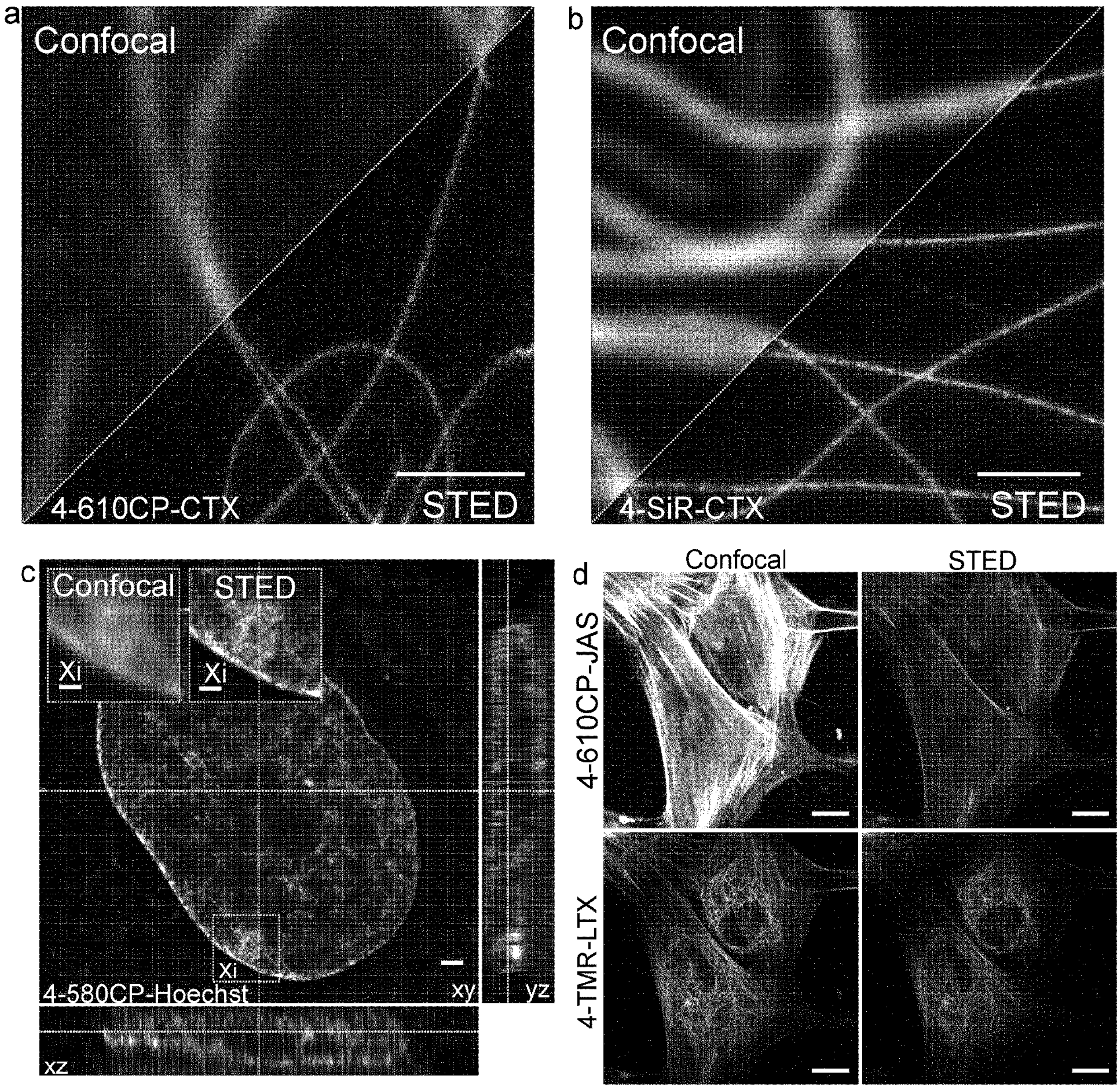
a
Confocal
4-610CP-CTX
STED
b
Confocal
4-SiR-CTX
STED
c
Confocal
STED
Xi
Xi
Xi
4-580CP-Hoechst
xy
yz
xz
d
Confocal
STED
4-610CP-JAS
4-TMR-LTX

FIG. 6

Europäisches Patentamt
European Patent Office
Office européen des brevets

# EUROPEAN SEARCH REPORT

Application Number
EP 20 15 6964

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>A | CN 110 272 432 A (DALIAN INST CHEM & PHYSICS CAS)<br>24 September 2019 (2019-09-24)<br>* page 10, paragraph 61 * | 1,3,4<br>2,5-20 | INV.<br>C09B11/28<br>C09B57/00<br>G01N33/53 |
| X<br>A | US 2017/067831 A1 (YAMADA KOHEI [JP])<br>9 March 2017 (2017-03-09)<br>* page 8, paragraph 107; compound (4) * | 1,3<br>2,4-20 | |
| X<br>A | KIRILL KOLMAKOV ET AL: "Far-Red Emitting Fluorescent Dyes for Optical Nanoscopy: Fluorinated Silicon-Rhodamines (SiRF Dyes) and Phosphorylated Oxazines",<br>CHEMISTRY - A EUROPEAN JOURNAL,<br>vol. 21, no. 38,<br>13 August 2015 (2015-08-13), pages 13344-13356, XP055723258,<br>DE<br>ISSN: 0947-6539, DOI: 10.1002/chem.201501394<br>* page 13348; compounds 15a, 15a-All * | 1,3,5-8, 10<br>2,4,9, 11-20 | |
| X | KOCISOVA E ET AL: "Monitoring of labeled antisense oligonucleotides within living cells by using a multifrequency phase/modulation approach for fluorescence lifetime measurements",<br>JOURNAL OF MOLECULAR STRUCTURE, ELSEVIER, AMSTERDAM, NL,<br>vol. 651-653, 1 June 2003 (2003-06-01), pages 115-122, XP027401500,<br>ISSN: 0022-2860<br>[retrieved on 2003-06-01]<br>* page 116 - page 117; compounds TMR-dT15 *<br>* page 120; figure 4 * | 1-20 | TECHNICAL FIELDS SEARCHED (IPC)<br>G01N<br>C09B |

-/--

The present search report has been drawn up for all claims

| Place of search | Date of completion of the search | Examiner |
|---|---|---|
| Munich | 27 August 2020 | Jeanjean, Fabien |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

4

EPO FORM 1503 03.82 (P04C01)

page 1 of 3

Europäisches Patentamt
European Patent Office
Office européen des brevets

# EUROPEAN SEARCH REPORT

Application Number
EP 20 15 6964

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | JÉRÔME CLERC ET AL: "Syringolin A Selectively Labels the 20?S Proteasome in Murine EL4 and Wild-Type and Bortezomib-Adapted Leukaemic Cell Lines", CHEMBIOCHEM, vol. 10, no. 16, 2 November 2009 (2009-11-02), pages 2638-2643, XP055723494, ISSN: 1439-4227, DOI: 10.1002/cbic.200900411 * page 2639; compounds Rh-SylA * * page 2640 - page 2641; figures 1-2 * ----- | 1-20 | |
| X | JOLIMAITRE P ET AL: "Synthesis and preliminary physical applications of a rhodamin-biotin phosphatidylethanolamine, an easy attainable lipid double probe", CHEMISTRY AND PHYSICS OF LIPIDS, LIMERICK, IR, vol. 133, no. 2, 1 February 2005 (2005-02-01), pages 215-223, XP027648655, ISSN: 0009-3084 [retrieved on 2005-02-01] | 1,3-12 | |
| A | * page 219; compounds 6-7 * ----- | 2,13-20 | |
| X | CN 109 504 015 A (CHANGZHOU YISHI PHOTOELECTRIC TECH CO LTD) 22 March 2019 (2019-03-22) | 1,3,5-8, 10 | |
| A | * page 18, paragraph 52; compounds VI-1 * * page 19, paragraph 58; compounds I-1 * ----- | 2,4,9, 11-20 | |

-/--

TECHNICAL FIELDS SEARCHED (IPC)

The present search report has been drawn up for all claims

4

| Place of search | Date of completion of the search | Examiner |
|---|---|---|
| Munich | 27 August 2020 | Jeanjean, Fabien |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 20 15 6964

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | KYLE R. GEE ET AL: "Caged Q-rhodamine dextran: a new photoactivated fluorescent tracer", BIOORGANIC & MEDICINAL CHEMISTRY LETTERS, vol. 11, no. 16, 1 August 2001 (2001-08-01), pages 2181-2183, XP055723602, AMSTERDAM, NL ISSN: 0960-894X, DOI: 10.1016/S0960-894X(01)00421-8 * page 2181; compound 5 * * page 2182; figure 1 * ----- | 1-20 | |
| X<br>A | CN 108 795 407 A (CHANGZHOU YISHI PHOTOELECTRIC TECH CO LTD) 13 November 2018 (2018-11-13) * page 12, paragraph 40 - paragraph 44; compounds II-1, I-1 * * page 13, paragraph 48; compounds V-1 * * page 14, paragraph 52; compounds I-2 * ----- | 1,3-8,10<br>2,9, 11-20 | |
| | | | TECHNICAL FIELDS SEARCHED (IPC) |

The present search report has been drawn up for all claims

| Place of search | Date of completion of the search | Examiner |
|---|---|---|
| Munich | 27 August 2020 | Jeanjean, Fabien |

4

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 20 15 6964

This annex lists the patent family members relating to the patent documents cited in the above-mentioned European search report. The members are as contained in the European Patent Office EDP file on The European Patent Office is in no way liable for these particulars which are merely given for the purpose of information.

27-08-2020

| Patent document cited in search report | Publication date | Patent family member(s) | Publication date |
|---|---|---|---|
| CN 110272432 A | 24-09-2019 | NONE | |
| US 2017067831 A1 | 09-03-2017 | JP 6613736 B2 | 04-12-2019 |
| | | JP 2017053638 A | 16-03-2017 |
| | | US 2017067831 A1 | 09-03-2017 |
| CN 109504015 A | 22-03-2019 | NONE | |
| CN 108795407 A | 13-11-2018 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **SAHL, S. J. ; HELL, S. W. ; JAKOBS, S.** *Nat. Rev. Mol. Cell. Biol.,* 2017, vol. 18 (11), 685-701
- **HELL, S. W.** *Angew Chem Int Ed Engl,* 2015, vol. 54 (28), 8054-66
- **WANG, L. ; FREI, M. S. ; SALIM, A. ; JOHNSSON, K.** *J Am Chem Soc,* 2019, vol. 141 (7), 2770-2781
- **BUTKEVICH A.N. ; MITRONOVA G.Y. ; SIDENSTEIN. S.C. ; KLOCKE J.L. ; KAMIN D. ; MEINEKE D.N.H. ; D'ESTE E. ; KRAEMER P.T. ; DANZL J.G. ; BELOV V.N.** *Angew. Chem. Int. Ed.,* 2016, vol. 55, 3290-3294
- **LUKINAVIČIUS G ; UMEZAWA K ; OLIVIER N ; HONIGMANN A ; YANG G ; PLASS T ; MUELLER V ; REYMOND L ; CORRÊA IR JR ; LUO ZG.** *Nat Chem,* 2013, vol. 5 (2), 132-139
- **UNO, S. N. ; TIWARI, D. K. ; KAMIYA, M. ; ARAI, Y. ; NAGAI, T. ; URANO, Y.** *Microscopy (Oxf),* 2015, vol. 64 (4), 263-77
- **LUKINAVIČIUS, G. ; REYMOND, L. ; UMEZAWA, K. ; SALLIN, O. ; D'ESTE, E. ; GOTTFERT, F. ; TA, H. ; HELL, S. W. ; URANO, Y. ; JOHNSSON, K.** *J. Am. Chem. Soc.,* 2016, vol. 138 (30), 9365-8
- **ZHENG, Q. ; AYALA, A. X. ; CHUNG, I. ; WEIGEL, A. V. ; RANJAN, A. ; FALCO, N. ; GRIMM, J. B. ; TKACHUK, A. N. ; WU, C. ; LIPPINCOTT-SCHWARTZ, J.** *ACS Cent Sci,* 2019, vol. 5 (9), 1602-1613
- **WANG, L. ; TRAN, M. ; D'ESTE, E. ; ROBERTI, J. ; KOCH, B. ; XUE, L. ; JOHNSSON, K.** *bioRxiv,* 2019, 690867
- **KEPPLER, A. ; GENDREIZIG, S. ; GRONEMEYER, T. ; PICK, H. ; VOGEL, H. ; JOHNSSON, K.** *Nat Biotechnol,* 2003, vol. 21 (1), 86-9
- **HINNER, M. J. ; JOHNSSON, K.** *Curr Opin Biotechnol,* 2010, vol. 21 (6), 766-76
- **LIU, C. C. ; SCHULTZ, P. G.** *Annu Rev Biochem,* 2010, vol. 79, 413-44
- **KEPPLER, A. ; ARRIVOLI, C. ; SIRONI, L. ; ELLENBERG, J.** *Biotechniques,* 2006, vol. 41 (2), 167-70
- *BIOTECHNIQUES,* vol. 172, 174-5
- **MAUREL, D. ; BANALA, S. ; LAROCHE, T. ; JOHNSSON, K.** *ACS Chem Biol,* 2010, vol. 5 (5), 507-16
- **JONES, S. A. ; SHIM, S. H. ; HE, J. ; ZHUANG, X.** *Nat Methods,* 2011, vol. 8 (6), 499-508
- **ÅKERLÖF G ; SHORT AO.** *J Am Chem Soc,* 1936, vol. 58 (7), 1241-1243
- **GOTTLIEB H.E. ; KOTLYAR V. ; NUDELMAN A.** *J Org Chem,* 1997, vol. 62, 7512-7515
- **G. ÅKERLÖƒ ; A.O. SHORT.** The Dielectric Constant of Dioxane-Water Mixtures between 0 and 80°. *J. Am. Chem. Soc.,* 1936, vol. 58 (7), 1241-1243
- **J. SCHINDELIN ; I. ARGANDA-CARRERAS ; E. FRISE ; V. KAYNIG ; M. LONGAIR ; T. PIETZSCH ; S. PREIBISCH ; C. RUEDEN ; S. SAALFELD ; B. SCHMID.** Fiji: an open-source platform for biological-image analysis. *Nat Methods,* 2012, vol. 9 (7), 676-82
- **A.E. CARPENTER ; T.R. JONES ; M.R. LAMPRECHT ; C. CLARKE ; I.H. KANG ; O. FRIMAN ; D.A. GUERTIN ; J.H. CHANG ; R.A. LINDQUIST ; J. MOFFAT.** CellProfiler: image analysis software for identifying and quantifying cell phenotypes. *Genome Biol,* 2006, vol. 7 (10), R100
- **MARTINEZ-PERAGON, A. ; MIGUEL, D. ; JURADO, R. ; JUSTICIA, J. ; ALVAREZ-PEZ, J. M. ; CUERVA, J. M. ; CROVETTO, L.** *Chemistry,* 2014, vol. 20 (2), 447-55
- **GRIMM, J. B. ; SUNG, A. J. ; LEGANT, W. R. ; HULAMM, P. ; MATLOSZ, S. M. ; BETZIG, E. ; LAVIS, L. D.** *ACS Chem Biol,* 2013, vol. 8 (6), 1303-1310
- **BUTKEVICH, A. N. ; BELOV, V. N. ; KOLMAKOV, K. ; SOKOLOV, V. V. ; SHOJAEI, H. ; SIDENSTEIN, S. C. ; KAMIN, D ; MATTHIAS, J. ; VLIJM, R. ; ENGELHARDT, J.** *Chemistry - A European Journal,* 2017, vol. 23 (50), 12114-12119
- **TANNERT, R. ; MILROY, L. G. ; ELLINGER, B. ; HU, T. S. ; ARNDT, H. D. ; WALDMANN, H.** *J Am Chem Soc,* 2010, vol. 132 (9), 3063-3077